(19) Europäisches Patentamt
European Patent Office
Office européen des brevets

(11) **EP 3 740 760 B1**

(12) **EUROPEAN PATENT SPECIFICATION**

(45) Date of publication and mention
of the grant of the patent:
**31.05.2023 Bulletin 2023/22**

(21) Application number: **19704140.3**

(22) Date of filing: **21.01.2019**

(51) International Patent Classification (IPC):
***G01N 33/68*** *(2006.01)*     ***G01N 33/70*** *(2006.01)*

(52) Cooperative Patent Classification (CPC):
**G01N 33/6893; G01N 33/70;** G01N 2800/347

(86) International application number:
**PCT/US2019/014427**

(87) International publication number:
**WO 2019/144081 (25.07.2019 Gazette 2019/30)**

(54) **BIOMARKERS AND CLASSIFICATION ALGORITHMS FOR CHRONIC KIDNEY DISEASE IN CATS**

BIOMARKER UND KLASSIFIKATIONSALGORITHMEN FÜR CHRONISCHE NIERENERKRANKUNGEN IN KATZEN

BIOMARQUEURS ET ALGORITHM DE CLASSIFICATION POUR MALADIE RÉNALE CHRONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.01.2018 US 201862619681 P**
**14.07.2018 US 201862698046 P**

(43) Date of publication of application:
**25.11.2020 Bulletin 2020/48**

(73) Proprietor: **Mars, Incorporated**
**McLean, VA 22101 (US)**

(72) Inventors:
• **BRADLEY, Richard**
**Leicestershire LE14 4RT (GB)**
• **TAGKOPOULOS, Ilias**
**Davis, CA 95616 (US)**
• **BIOURGE, Vincent**
**30470 Aimargues (FR)**
• **FEUGIER, Alexandre**
**30470 Aimargues (FR)**
• **DELMOTTE, Sebastien**
**31560 Nailloux (FR)**

(74) Representative: **Carpmaels & Ransford LLP**
**One Southampton Row**
**London WC1B 5HA (GB)**

(56) References cited:
WO-A1-2012/177717     WO-A1-2013/190084
WO-A1-2016/077484     WO-A2-2012/033999
US-A1- 2013 122 528

• **MARTHA CANNON: "Diagnosis and investigation of chronic kidney disease in cats", IN PRACTICE, vol. 38, no. Suppl 3, 1 October 2016 (2016-10-01), pages 2-9, XP055571676, GB ISSN: 0263-841X, DOI: 10.1136/inp.i4914**
• **I. VAN HOEK ET AL: "Short- and long-term follow-up of glomerular and tubular renal markers of kidney function in hyperthyroid cats after treatment with radioiodine", DOMESTIC ANIMAL ENDOCRINOLOGY, vol. 36, no. 1, 1 January 2009 (2009-01-01), pages 45-56, XP055571689, AMSTERDAM, NL ISSN: 0739-7240, DOI: 10.1016/j.domaniend.2008.10.001**
• **L. GHYS ET AL: "Cystatin C: A New Renal Marker and Its Potential Use in Small Animal Medicine", JOURNAL OF VETERINARY INTERNAL MEDICINE, vol. 28, no. 4, 9 May 2014 (2014-05-09), pages 1152-1164, XP055570984, US ISSN: 0891-6640, DOI: 10.1111/jvim.12366**

**Description**

**CROSS-REFERENCE TO RELATED APPLICATIONS**

This application claims priority to United States Provisional Application No. 62/619,681, filed January 19, 2018, and United States Provisional Application No. 62/698,046, filed July 14, 2018.

**TECHNICAL FIELD**

The presently disclosed subject matter relates to methods of determining a feline's susceptibility to developing chronic kidney disease (CKD) and to methods of preventing and/or reducing a risk of developing CKD for a feline.

**BACKGROUND**

Chronic kidney disease (CKD), also known as chronic renal disease or chronic renal failure, is a progressive loss in renal function over a period of months or years. CKD can be caused by a variety of conditions and mechanisms, and it affects both humans and other mammals. CKD is a common cause of illness and death in aging felines. It is important to detect CKD as early as possible to begin treatment before significant damage occurs.
In cats suffering from renal disease, a scheme for staging CKD in cats and dogs has been developed by the International Renal Interest Society (IRIS) (*see also* Elliott et al., Dietary therapy for feline chronic kidney disease, Encyclopedia of feline clinical nutrition, 2nd edition, 2015). Staging is based initially on fasting blood creatinine concentration, assessed on at least two occasions in a stable cat. The cat is then substaged based on proteinuria and blood pressure. However, there remains a need in the art for methods of predicting, preventing and/or reducing a risk of CKD.

**SUMMARY**

In certain non-limiting embodiments, the presently disclosed subject matter provides a system for identifying a susceptibility to developing chronic kidney disease (CKD) for a feline, the system comprising: a processor; and a memory that stores code that, when executed by the processor, causes the computer system to: receive at least one input level of one or more biomarkers from the feline and optionally an input level of an age of the feline, wherein at least one of the one or more biomarkers comprises information relating to a urine specific gravity level, a creatinine level, a urine protein level, a blood urea nitrogen (BUN) or urea level, a white blood cell count (WBC), urine pH, or any combination thereof; analyze and transform the input level of the one or more biomarkers and optionally the input level of the by organizing and/or modifying each input level to derive a probability score or a classification label via a classification algorithm, wherein the classification algorithm comprises code developed from a training dataset, the training dataset comprising medical information relating to both a first plurality of biomarkers and optionally ages from a first set of sample felines and a second plurality of biomarkers and optionally ages from a second set of sample felines, wherein the classification algorithm is developed using a training algorithm; wherein the classification algorithm is one of a hard classifier, which determines the classification label of whether the feline is at risk of developing CKD, or a soft classifier, which determines the probability score of the feline developing CKD; generate an output, wherein the output is the classification label or the probability score; determine or categorize, based on the output, whether the feline is at risk of developing CKD; and determine a customized recommendation based on the determining or categorizing.
In certain embodiments, the code, when executed by the processor, further causes the system to display the determination or categorization and customized recommendation on a graphical user interface.
In certain embodiments, the system further comprises: a communication device for transmitting and receiving information; wherein: the at least one input level is received from a remote second system, via the communication device; and the code, when executed by the processor, further causes the system to transmit the determination or categorization and customized recommendation to the remote second system, via the communication device.
In certain embodiments, the system provides a customized recommendation of a dietary regimen and/or further monitoring the one or more biomarkers based on the output.
In certain non-limiting embodiments, the presently disclosed subject matter provides for a computer-implemented method of identifying a susceptibility to developing chronic kidney disease (CKD) for a feline, by performing the steps of: receiving at least one input level of one or more biomarkers from the feline and optionally an input level of an age of the feline, wherein at least one of the one or more biomarkers comprises information relating to a urine specific gravity level, a creatinine level, a urine protein level, a blood urea nitrogen (BUN) or urea level, a white blood cell count (WBC), urine pH, or any combinations thereof; analyzing and transforming the at least one input level of the one or more biomarkers and optionally the input level of the age by organizing and/or modifying each input level to derive a probability score or a classification label via a classification algorithm, wherein the classification algorithm comprises code developed from a training dataset, the training dataset comprising medical information relating to both a first plurality of biomarkers and optionally ages from a first set of sample felines and a second plurality of biomarkers and optionally ages from a second set of sample felines, wherein the classification algorithm is developed using a training algorithm; wherein the classification algorithm is one of a hard classifier, which determines the classification label of whether the feline is at risk of developing CKD, or a soft classifier, which determines the probability score of the feline developing CKD; generating an output, wherein the output is the classification label or the probability score; determining or categorizing, based on the output, whether the feline is at risk of developing CKD; and determining a customized recommendation based on the determining or categorizing.

In certain embodiments, the method further comprises the step of displaying the determination or categorization and customized recommendation on a graphical user interface.

In certain embodiments, the at least one input level is received from a remote second system, via a communication device; and further comprising the step of: transmitting the determination or categorization and customized recommendation to the remote second system, via the communication device.

In certain non-limiting embodiments, the presently disclosed subject matter provides for a non-transitory computer readable medium, storing instructions that, when executed by a processor, cause a computer system to execute the steps of any of methods disclosed herein.

In certain embodiments, the classification algorithm is developed using a supervised training algorithm under supervision of the one or more biomarkers and optionally the ages. In certain embodiments, the classification algorithm is developed using an unsupervised training algorithm.

In certain embodiments, the at least one input level comprise sequential measurements of the one or more biomarkers measured at different time points.

In certain embodiments, the first set of sample felines have been diagnosed with CKD and the second set of sample felines have not been diagnosed with CKD. In certain embodiments, the training dataset is stratified into 2 or more folds for cross validation. In certain embodiments, the training dataset is filtered by a set of inclusion and/or exclusion criteria.

In certain embodiments, the training algorithm comprises an algorithm selected from the group consisting of logistic regression, artificial neural network (ANN), recurrent neural network (RNN), K-nearest neighbor (KNN), Naive Bayes, support vector machine (SVM), random forest, AdaBoost and any combination thereof. In certain embodiments, the training algorithm comprises KNN with dynamic time warping (DTW). In certain embodiments, the training algorithm comprises RNN with long short-term memory (LSTM).

In certain embodiments, the classification algorithm comprises a regularization algorithm comprising 5% or more dropout to prevent overfitting.

In certain embodiments, the dietary regimen is selected from the group consisting of a low phosphorous diet, a low protein diet, a low sodium diet, a potassium supplement diet, a polyunsaturated fatty acids (PUFA) supplement diet, an anti-oxidant supplement diet, a vitamin B supplement diet, a liquid diet and any combination thereof.

In certain embodiments, the one or more biomarkers comprises information relating to a urine specific gravity level, a creatinine level and a blood urea nitrogen (BUN) or urea level. In certain embodiments, the one or more biomarkers comprises information relating to a urine specific gravity level, a creatinine level, a urine protein level, a blood urea nitrogen (BUN) or urea level, a white blood cell count (WBC) and urine pH. In certain embodiments, the method comprises receiving at least one input level of one or more biomarkers from the feline and an input level of an age of the feline. In certain embodiments, the method comprises receiving input levels of biomarkers comprising information relating to a urine specific gravity level, a creatinine level and a blood urea nitrogen (BUN) or urea level; and an input level of an age of the feline.

In certain embodiments, in any of the methods disclosed herein, the classification algorithm comprises a standard RNN algorithm. In certain embodiments, the input levels of the biomarkers and the age of the feline relate to medical records of one or more visit of the feline. In certain embodiments, the input levels of the biomarkers and the age of the feline relate to medical records of at least 2 visits of the feline. In certain embodiments, in any of the methods disclosed herein, the classification label or the probability score is transformed from a combination of intermediate probability scores, each of which is determined based on the input levels of the biomarkers and the age of the feline relating to a medical record of one visit of the feline.

In certain embodiments, the classification label or the probability score relates to the feline's status of contracting chronic kidney disease (CKD) at the time of the determination of the classification label or the probability score. In certain embodiments, the classification label or the probability score relates to the feline's risk of developing chronic kidney disease (CKD) after the determination of the classification label or the probability score. In certain embodiments, the classification label or the probability score relates to the feline's risk of developing chronic kidney disease (CKD) about 1 year after the determination of the classification label or the probability score. In certain embodiments, the classification label or the probability score relates to the feline's risk of developing chronic kidney disease (CKD) about 2 years after the determination of the classification label or the probability score.

In certain embodiments, in any of the methods disclosed herein, the customized recommendation comprises diagnosing the presence of a comorbidity in the feline. In certain embodiments, the comorbidity is selected from the group consisting of hyperthyroidism, diabetes mellitus, hepatopathy, underweight, murmur, arthritis, malaise, constipation, gastroenteritis, vomiting, inflammatory bowel disease, crystalluria, enteritis, urinary tract infection, upper respiratory disease, urinary tract disease, obesity, inappropriate elimination, cystitis, colitis and any combination thereof. In certain embodiments, the comorbidity is selected from the group consisting of hyperthyroidism, diabetes mellitus, hepatopathy, underweight, murmur and any combination thereof.

## BRIEF DESCRIPTION OF THE DRAWINGS

Figure 1 depicts a distribution of visits per cat age at the time of the visit.

Figures 2A-2C depict a hierarchical clustering and heatmap plot of the 61,160 records that comprise the dataset after min-max normalization and missing value imputation. Figure 2A depicts the dataset after the 1223 outliers have been removed; the 6 features that will be used for prediction are shown in red rectangular boxes. Figure 2B depicts the heatmap of the 6 features only. Figure 2C depicts the heatmap without removing the 1223 outliers.

Figure 3 depicts a scatterplot matrix for the 6 most informative variables. Visits with healthy and CKD cats are shown as green and red dots, respectively.

Figures 4A-4D depict PCA and t-SNE plots of healthy and CKD visits. Figure 4A depicts a PCA 2D plot of healthy and CKD visits. Figure 4B depicts a PCA 3D plot of healthy and CKD visits. Figure 4C depicts a t-SNE 2D plot of healthy and CKD visits. Figure 4D depicts a t-SNE 3D plot of healthy and CKD visits.

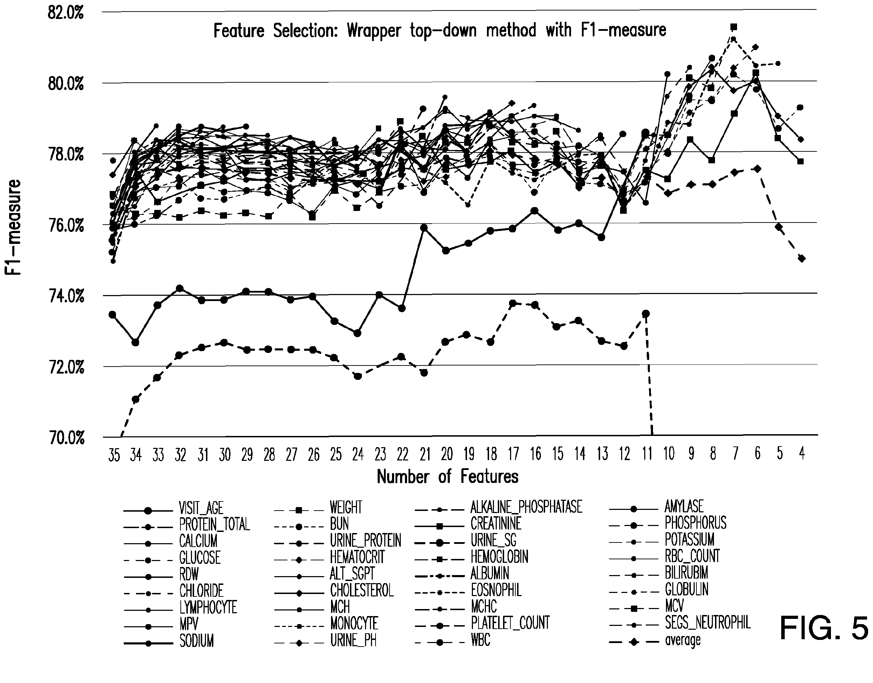

Figure 5 depicts feature selection with Recursive Feature Elimination Top-down wrapper method.

Figure 6 depicts optimal K parameter selection with all training data used.

Figures 7A-7B depict receiver operating characteristic curves (ROC curves) and precision-recall curves (PR curves) for K= 3 to 17 for the sampled dataset. Figure 7A depict PR curves for K= 3 to 17 for the sampled dataset. Figure 7B depicts ROC curves for K= 3 to 17 for the sampled dataset.

Figures 8A-8B depict ROC curves and PR curves for each individual temporal predictor and the Mixture of Experts (MOE). Figure 8A depicts PR curves for each individual temporal predictor and the Mixture of Experts (MOE). Figure 8B depicts ROC curves for each individual temporal predictor and the Mixture of Experts (MOE).

Figure 9 depicts Recurrent Neural Network architecture.

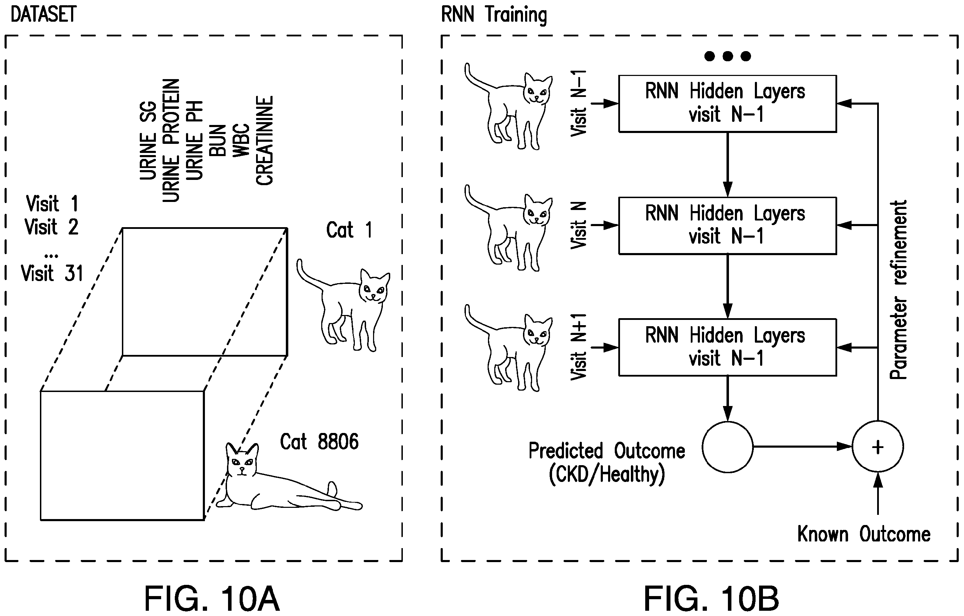

Figures 10A-10B depict schematics of machine learning processes. Figure 10A depicts structure of the training dataset to the RNN architecture. For each RNN time slice a vector of the six features for a unique cat are loaded. Figure 10B depicts training schema for the single output RNN (vanilla or LSTM). At each time slice a single visit/cat is loaded and the forward activation functions are calculated. At the last visit, the output is calculated (probability of CKD that is converted to a binary prediction) and then compared to the real label. Any difference between the true label and the prediction is backpropagated to refine the weights. The procedure is repeated for several epochs, with one epoch being a full utilization of the dataset.

Figure 11 depicts LSTM (top) and vanilla RNN (bottom) architectures with their 3 metrics. For each configuration, the first row represents the node distribution per layer and the subsequent 3 rows the F1 score, AUC ROC and AUC PR values, respectively. The best performers are highlighted in dark green boxes.

Figure 12 depicts F1-scores as a function of the number of nodes for LSTM and vanilla RNN (blue and orange circles, respectively).

Figures 13A-13D depict the features of a model based on RNN-LSTM algorithm. Figure 13A depicts RNN-LSTM architecture of the optimal configuration (3 LSTM layers, 7-7-7 with a dense Feed Forward layer at the end). Figure 13B depicts ROC curves for the 5-fold CV with AUC 0.93-0.96 (0.94 overall). Figure 13C depicts loss function vs. number of epochs. Figure 13D depicts PR curves for the 5-fold CV with AUC 0.89-0.94 (0.91 overall). Baseline performance is the prior probability of membership on the CKD class (26%) and is depicted by a star (*).

Figures 14A-14C depict the features of a model based on vanilla RNN algorithm. Figure 14A depicts an alternative, near-optimal implementation with a vanilla RNN Architecture (3 RNN layers, 3-5-3 with a final dense Feed Forward). Figure 14B depicts ROC curves for the 5-fold CV with AUC 0.93-0.95 (0.94 overall). Figure 14C depicts loss function vs. number of epochs. Figure 14D depicts PR curves for the 5-fold CV with AUC 0.90-0.93 (0.91 overall).

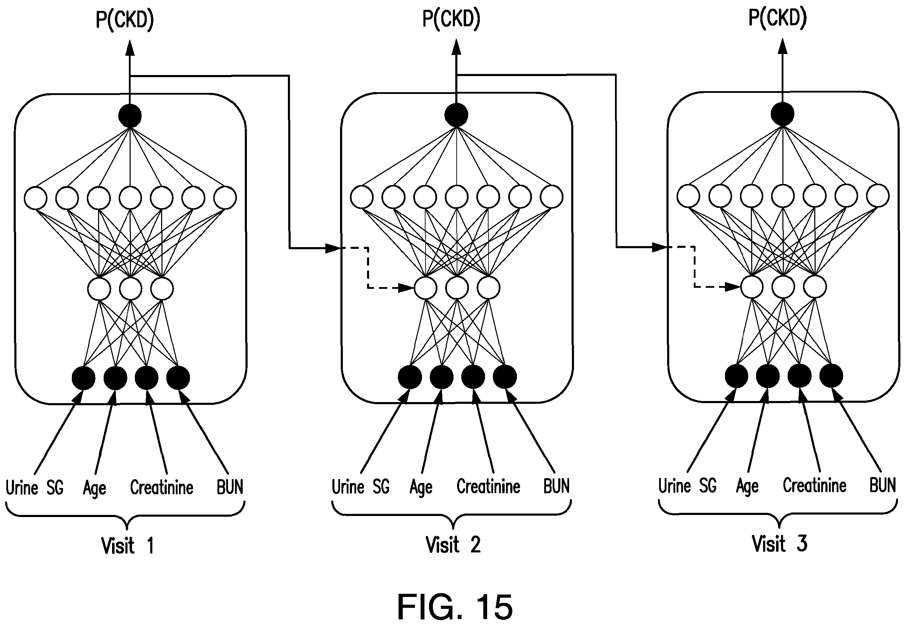

Figure 15 depicts schematic representation of recurrent neural network (RNN) approaches. In a standard RNN the input feature data at every visit (here as an example urine specific gravity (Urine SG), age, creatinine and blood urea nitrogen (BUN) are combined in nonlinear ways through 2 hidden layers with 3 and 7 nodes, respectively, and merged with the prior CKD probability - P(CKD) to yield an updated P(CKD). The weights and activation functions that define the nonlinear pattern are the same for every visit. The model output is P(CKD) at the last visit. A LSTM (long short-term memory) approach is conceptually similar but has additional mechanisms to forget part of the information from prior visits when combining these with the current visit information.

Figure 16 depicts distribution of age at evaluation (T0), creatinine, blood urea nitrogen and urine specific gravity in the study data set differentiated by CKD status.

Figures 17A-17H depict randomly picked electronic health records (EHRs) for individual cats with CKD statuses showing the observations for creatinine, blood urea nitrogen and urine specific gravity as a function of time before diagnosis (T0). A) and B) CKD status of "No CKD." C) and D) CKD status of "Probable CKD." E) to H) CKD status of "CKD".

Figure 18 depicts F1-score as a function of model architecture for RNN and LSTM prediction models.

Figure 19 depicts distribution of model probability outputs for the three different groups predicted at evaluation T0 in the test data set. A diagnosis probability p(CKD) of greater than 0.5 denotes a prediction of future CKD risk, and a prediction below 0.5 predicts low future CKD risk for that cat.

Figure 20 depicts model sensitivity with 95% confidence interval as a function of the number of visits before the time of diagnosis. Note that confidence intervals increase as there are less EHRs with large numbers of visits before the time of diagnosis.

Figure 21 depicts model sensitivity with 95% confidence intervals as a function of the time before diagnosis where the prediction was made only with the data up to that point.

Figure 22 depicts model specificity with 95% confidence intervals as a function of age at diagnosis.

## DETAILED DESCRIPTION

To date, there remains a need for methods of predicting, treating and/or preventing CKD. The present application relates to determining susceptibility of a feline to developing chronic kidney disease (CKD) and methods of preventing and/or reducing a risk of developing CKD for a feline, using biomarkers and, optionally, an age of the feline, wherein the biomarkers include, but are not limited to, urine specific gravity, creatinine, urine protein, blood urea nitrogen (BUN) (or urea), white blood cell count (WBC) and urine pH. For clarity and not by way of limitation, the detailed description of the presently disclosed subject matter is divided into the following subsections:

    1. Definitions;
    2. Biomarkers;
    3. Test methods;
    4. Treatment methods; and
    5. Device and system.

### 1. DEFINITIONS

The terms used in this specification generally have their ordinary meanings in the art, within the context of this invention and in the specific context where each term is used. Certain terms are discussed below, or elsewhere in the specification, to provide additional guidance to the practitioner in describing the methods and compositions of the invention and how to make and use them.

As used herein, the use of the word "a" or "an" when used in conjunction with the term "comprising" in the claims and/or the specification may mean "one," but it is also consistent with the meaning of "one or more," "at least one," and "one or more than one." Still further, the terms "having," "including," "containing" and "comprising" are interchangeable and one of skill in the art is cognizant that these terms are open ended terms.

The term "about" or "approximately" means within an acceptable error range for the particular value as determined by one of ordinary skill in the art, which will depend in part on how the value is measured or determined, i.e., the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

The term "effective treatment" or "effective amount" of a substance means the treatment or the amount of a substance that is sufficient to effect beneficial or desired results, including clinical results, and, as such, an "effective treatment" or an "effective amount" depends upon the context in which it is being applied. In the context of administering a composition to reduce a risk of CKD, and/or administering a composition to treat or delay the progression of CKD, an effective amount of a composition described herein is an amount sufficient to treat and/or ameliorate CKD, as well as decrease the symptoms and/or reduce the likelihood of developing CKD. An effective treatment described herein is a treatment sufficient to treat and/or ameliorate CKD, as well as decrease the symptoms and/or reduce the likelihood of CKD. The decrease can be a 10%, 20%, 30%, 40%, 50%, 60%, 70%, 80%, 90%, 95%, 98% or 99% decrease in severity of symptoms of CKD, or likelihood of CKD. An effective amount can be administered in one or more administrations. A likelihood of an effective treatment described herein is a probability of a treatment being effective, *i.e.,* sufficient to treat and/or ameliorate CKD, as well as decrease the symptoms.

As used herein, and as well understood in the art, "treatment" is an approach for obtaining beneficial or desired results, including clinical results. For purposes of this subject matter, beneficial or desired clinical results include, but are not limited to, alleviation or amelioration of one or more symptoms, diminishment of extent of disease, stabilized (i.e., not worsening) state of disease, prevention of disease, reducing the likelihood of developing disease, delay or slowing of disease progression, and/or amelioration or palliation of the disease state. The decrease can be a 10%, 20%, 30%, 40%, 50%, 60%, 70%, 80%, 90%, 95%, 98% or 99% decrease in severity of complications or symptoms. "Treatment" can also mean prolonging survival as compared to expected survival if not receiving treatment.

The term "pet food" or "pet food composition" or "pet food product" or "final pet food product" means a product or composition that is intended for consumption by, and provides certain nutritional benefit to a companion animal, such as a cat, a dog, a guinea pig, a rabbit, a bird or a horse. For example, but not by way of limitation, the companion animal can be a "domestic" dog, *e.g., Canis lupus familiaris.* In certain embodiments, the companion animal can be a "domestic" cat such as *Felis domesticus.* A "pet food" or "pet food composition" or "pet food product" or "final pet food product" includes any food, feed, snack, food supplement, liquid, beverage, treat, toy (chewable and/or consumable toys), meal substitute or meal replacement.

As used herein, the term "predetermined reference value" or "reference value" refers to a threshold level of a biomarker by comparing with which, a diagnosis of CKD can be made. The reference value can be a threshold value or a reference range. In certain embodiments, a reference value can be derived from ROC curve analysis, selecting the reference value as that which maximizes sensitivity while keeping the specificity above a user-defined threshold. A receiver operating characteristic curve, i.e. ROC curve, is a graphical plot that illustrates the diagnostic ability of a binary classifier system. In certain embodiments, the reference value can be selected as that which maximizes specificity while keeping the sensitivity above a user-defined threshold, for example, 80% sensitivity. In certain embodiments, a reference value can be the upper limit of the range of a biomarker levels produced from a population of healthy subjects, if the biomarker is increased in subjects having CKD, *i.e.,* the predetermined algorithm is positive logic. Conversely, a reference value can be the lower limit of the range of a biomarker levels or produced from a population of healthy subjects, if the biomarker is decreased in subjects having CKD, *i.e.,* the algorithm is negative logic.

The term "control population" means a control group of felines that do not have chronic kidney disease and that have not had any variables manipulated. The selection of the felines to be included in the control groups may be based on genetic background, average health status, age, history of nutrition, vaccination and/or prophylactic treatment. In certain embodiments, a control population can comprise a group of at least 3, preferably at least 10, or, more preferably, at least 50 felines with a similar genetic background, age and/or average health status.

The term "visit" means a meeting between a healthcare practitioner and a feline. In certain embodiments, a medical record is generated during or after a visit. In certain embodiments, an amount of one or more biomarkers is determined during a visit. In certain embodiments, a diagnosis of CKD is made during a visit. The practitioner can make a visit to the feline in a hospital and/or in a home or other location. A feline, taken by an owner, can make a visit to the practitioner in a clinic or an office.

The term "urine specific gravity" (a.k.a. urine SG or USG) measures the ratio of urine density compared to water density. It is a measure of the concentration of solutes in the urine, and it provides information on the ability of a kidney to concentrate urine.

## 2. BIOMARKERS

In certain non-limiting embodiments, the presently disclosed subject matter provides for biomarkers and methods of using the same to determine a feline's susceptibility to developing CKD.

The term "biomarker" as used herein, refers to any biological measurement, parameter, or combination thereof related to the development of a disease of interest. In particular, a biomarker for predicting CKD is one or more biological parameters related to the development of CKD. The prevention and/or treatment of kidney disease may be tailored, depending upon the risk of developing CKD indicated by the biomarkers The prediction of recovery can also be determined by monitoring the biomarkers.

In certain embodiments, the biomarker comprises at least one creatinine level, at least one at least one urine specific gravity level, at least one blood urea nitrogen (BUN) or urea level or any combination thereof. In certain embodiments, the biomarker comprises a urine specific gravity level, a creatinine level, a urine protein level, a blood urea nitrogen (BUN) or urea level, a white blood cell count (WBC), a urine pH or a combination thereof.

In certain embodiments, BUN and urea measurement is interchangeable. As BUN reflects only the nitrogen content of urea (molecular weight 28) and urea measurement reflects the whole molecule (molecular weight 60), urea measurement is 2.14 (60/28) times of BUN measurement.

In certain embodiments, the biomarker comprises the urine specific gravity level in a urine sample of the feline. In certain embodiments, the biomarker comprises the total creatinine level in the blood of the feline. In certain embodiments, the biomarker comprises the creatinine level in the serum of the feline. In certain embodiments, the biomarker comprises the creatinine in the plasma of the feline. In certain embodiments, the biomarker comprises the creatinine in a urine sample of the feline. In certain embodiments, the biomarker comprises the urine protein in a urine sample of the feline. In certain embodiments, the biomarker comprises the total urea in the blood of the feline. In certain embodiments, the biomarker comprises the urea in the serum of the feline. In certain embodiments, the biomarker comprises the urea in the plasma of the feline. In certain embodiments, the biomarker comprises the urea in a urine sample of the feline. In certain embodiments, the biomarker comprises the blood urea nitrogen (BUN) or urea in the blood of the feline. In certain embodiments, the biomarker comprises the white blood cell count (WBC) in the blood of the feline. In certain embodiments, the biomarker comprises the urine pH in a urine sample of the feline. In certain embodiments, a change in a level of a biomarker is associated with an increased risk of developing CKD.

With each biomarker, an increased or a decreased level of the biomarker can give information about a feline's susceptibility to developing CKD, depending on the particular biomarker. For example, in certain embodiments, a decreased level of urine specific gravity indicates an increased risk of developing CKD. In certain embodiments, an increased level of urine specific gravity indicates a decreased risk of developing CKD. In certain embodiments, a lower level of urine specific gravity compared to a predetermined reference value based on average levels of urine specific gravity in a control population indicates an increased risk of developing CKD. In certain embodiments, a higher level of urine specific gravity compared to a predetermined reference value based on average levels of urine specific gravity in a control population indicates a decreased risk of developing CKD. In certain embodiments, the average levels of urine specific gravity in a control population is between about 1.00 and about 1.1, between about 1.01 and about 1.09, between about 1.02 and about 1.08, or between about 1.03 and about 1.07. In certain embodiments, the average levels of urine specific gravity in a control population is between about 1.001 and about 1.08. In certain embodiments, the predetermined reference value of urine specific gravity is about 100%, about 99%, about 98%, about 97%, about 96%, about 95%, about 94%, about 93%, about 92%, about 91%, about 90%, about 89%, about 88%, about 87%, about 86%, about 85%, about 80%, about 75%, about 70% or less, or any intermediate percentage or range of the average level of urine specific gravity in a control population. In certain embodiments, the predetermined reference value of urine specific gravity is between about 99.9% and about 90%, between about 95% and about 90%, or between about 99% and about 92% of the average level of urine specific gravity in a control population. In certain embodiments, the predetermined reference value of urine specific gravity is between about 1.001 and about 1.08, between about 1.001 and about 1.07, between about 1.001 and about 1.06, between about 1.001 and about 1.05. or between about 1.001 and about 1.04. In certain embodiments, a feline's hydration status is considered to adjust the urine specific gravity level.

In certain embodiments, an increased level of creatinine indicates an increased risk of developing CKD. In certain embodiments, a decreased level of creatinine indicates a decreased risk of developing CKD. In certain embodiments, a higher level of creatinine compared to a predetermined reference value based on average levels of creatinine in a control population indicates an increased risk of developing CKD. In certain embodiments, a lower level of creatinine compared to a predetermined reference value based on average levels of creatinine in a control population indicates a decreased risk of developing CKD. In certain embodiments, the average levels of creatinine in a control population is between about 0.5 mg/dL and about 5 mg/dL, between about 0.8 mg/dL and about 3 mg/dL, between about 1 mg/dL and about 2.8 mg/dL, or between about 1.2 mg/dL and about 2.2 mg/dL. In certain embodiments, the average levels of creatinine in a control population is between about 0.8 mg/dL and about 2.4 mg/dL, In certain embodiments, the predetermined reference value of creatinine is about 100%, about 105%, about 110%, about 115%, about 120%, about 125%, about 130%, about 140%, about 150%, about 200%, about 250%, about 300%, about 400%, about 500% or more, or any intermediate percentage or range of the average level of creatinine in a control population. In certain embodiments, the predetermined reference value of creatinine is between about 100% and about 120%, between about 120% to about 150%, between about 150% and about 200%, or between about 200% and about 500% of the average level of creatinine in a control population. In certain embodiments, the predetermined reference value of creatinine is between about 0.5 mg/dL and about 3 mg/dL, between about 1 mg/dL and about 2.4 mg/dL, between about 1 mg/dL and about 2 mg/dL, or between about 1.2 mg/dL and about 1.8 mg/dL.

In certain embodiments, a decreased level of urine protein indicates an increased risk of developing CKD. In certain embodiments, an increased level of urine protein indicates a decreased risk of developing CKD. In certain embodiments, an increased level of urine protein indicates an increased risk of developing CKD. In certain embodiments, a decreased level of urine protein indicates a decreased risk of developing CKD. In certain embodiments, a lower level of urine protein compared to a predetermined reference value based on average levels of urine protein in a control population indicates an increased risk of developing CKD. In certain embodiments, a higher level of urine protein compared to a predetermined reference value based on average levels of urine protein in a control population indicates a decreased risk of developing CKD. In certain embodiments, a higher level of urine protein indicates infection or kidney damage. In certain embodiments, a historic bout of elevated urine protein indicates earlier infections and/or higher risk of kidney damage. In certain embodiments, current elevation of urine protein indicates higher risk of declining renal function and/or CKD. In certain embodiments, a feline exhibits a higher level of urine protein compared to a predetermined reference value at present, e.g., a higher level of urine protein is found in a current sample of the feline or in a recent medical record of the feline (e.g., a record made within about 1 week, about 2 weeks, about 3 weeks, about 4 weeks, about 5 weeks, about 10 weeks, about 3 months or about 6 months before practicing any one of the methods disclosed herein). In certain embodiments, a feline has exhibited a higher level of urine protein compared to a predetermined reference value in the past, e.g., a higher level of urine protein is found in a historic sample of the feline or in a historical medical record of the feline (e.g., a record made more than about 1 week, about 2 weeks, about 1 month, about 2 months, about 3 months or about 6 months before practicing any one of the methods disclosed herein). In certain embodiments, the average levels of urine protein in a control population is between about 0 mg/dL and about 50 mg/dL, between about 0 mg/dL and about 25 mg/dL, between about 0 mg/dL and about 10 mg/dL, or between about 0 mg/dL and about 5 mg/dL. In certain embodiments, the average levels of urine protein in a control population is between about 0 mg/dL and about 20 mg/dL. In certain embodiments, the predetermined reference value of urine protein is at least about 100%, about 110%, about 120%, about 130%, about 140%, about 150%, about 160%, about 170%, about 180%, about 190%, about 200%, about 250%, about 300%, about 400%, about 500%, about 1000%, about 2000%, about 5000%, about 10000% or more, or any intermediate percentage or range of the average level of urine protein in a control population. In certain embodiments, the predetermined reference value of urine protein is between about 100% and about 200%, between about 200% and about 500%, or between about 200% and about 1000% of the average level of urine protein in a control population. In certain embodiments, the predetermined reference value of urine protein is between about 0.001 mg/dL and about 100 mg/dL, between about 1 mg/dL and about 80 mg/dL, between about 5 mg/dL and about 70 mg/dL, between about 10 mg/dL and about 60 mg/dL, or between about 20 mg/dL and about 50 mg/dL.

In certain embodiments, an increased level of BUN or urea indicates an increased risk of developing CKD. In certain embodiments, a decreased level of BUN or urea indicates a decreased risk of developing CKD. In certain embodiments, a higher level of BUN or urea compared to a predetermined reference value based on average levels of BUN or urea in a control population indicates an increased risk of developing CKD. In certain embodiments, a lower level of BUN or urea compared to a predetermined reference value based on average levels of BUN or urea in a control population indicates a decreased risk of developing CKD. In certain embodiments, the average levels of BUN in a control population is between about 5 mg/dL and about 100 mg/dL, between about 10 mg/dL and about 50 mg/dL, between about 15 mg/dL and about 40 mg/dL, or between about 20 mg/dL and about 30 mg/dL. In certain embodiments, the average levels of BUN in a control population is between about 16 mg/dL and about 36 mg/dL. In certain embodiments, the average levels of urea in a control population is between about 10.7 mg/dL and about 214 mg/dL, between about 21.4 mg/dL and about 107 mg/dL, between about 32.1 mg/dL and about 85.6 mg/dL, or between about 42.8 mg/dL and about 64.2 mg/dL. In certain embodiments, the average levels of urea in a control population is between about 34.24 mg/dL and about 77.04 mg/dL. In certain embodiments, the predetermined reference value of BUN or urea is about 100%, about 105%, about 110%, about 115%, about 120%, about 125%, about 130%, about 140%, about 150%, about 200%, about 250%, about 300%, about 400%, about 500% or more, or any intermediate percentage or range of the average level of BUN or urea in a control population. In certain embodiments, the predetermined reference value of BUN or urea is between about 100% and about 120%, between about 120% to about 150%, between about 150% and about 200%, or between about 200% and about 500% of the average level of BUN or urea in a control population. In certain embodiments, the predetermined reference value of BUN is between about 10 mg/dL and about 100 mg/dL, between about 15 mg/dL and about 90 mg/dL, between about 20 mg/dL and about 80 mg/dL, between about 30 mg/dL and about 70 mg/dL, between about 40 mg/dL and about 70 mg/dL, or between about 40 mg/dL and about 60 mg/dL. In certain embodiments, the predetermined reference value of urea is between about 21.4 mg/dL and about 214 mg/dL, between about 32.1 mg/dL and about 192.6 mg/dL, between about 42.8 mg/dL and about 171.2 mg/dL, between about 64.2 mg/dL and about 149.8 mg/dL, between about 85.6 mg/dL and about 149.8 mg/dL, or between about 85.6 mg/dL and about 128.4 mg/dL.

In certain embodiments, a decreased level of WBC indicates an increased risk of developing CKD. In certain embodiments, an increased level of WBC indicates a decreased risk of developing CKD. In certain embodiments, an increased level of WBC indicates an increased risk of developing CKD. In certain embodiments, a decreased level of WBC indicates a decreased risk of developing CKD. In certain embodiments, WBC can be used by a prediction model to rule out other infections. In certain embodiments, WBC can be used by a prediction model to relate previous infections to future risk. In certain embodiments, WBC can be used by a prediction model to understand dehydration level and normalize the values of other biomarkers. In certain embodiments, a prediction model generated by machine learning process can interpret the WBC count according to the visit, the current and/or previous values of other biomarkers. In certain embodiments, a higher level of WBC compared to a predetermined reference value based on average levels of WBC in a control population indicates an increased risk of developing CKD. In certain embodiments, a higher level of WBC indicates infection or kidney damage. In certain embodiments, a historic bout of elevated WBC indicates earlier infections and/or higher risk of kidney damage. In certain embodiments, current elevation of WBC indicates higher risk of declining renal function and/or CKD. In certain embodiments, a feline exhibits a higher level of WBC compared to a predetermined reference value at present, e.g., a higher level of WBC is found in a current sample of the feline or in a recent medical record of the feline (e.g., a record made within about 1 week, about 2 weeks, about 3 weeks, about 4 weeks, about 5 weeks, about 10 weeks, about 3 months or about 6 months before practicing any one of the methods disclosed herein). In certain embodiments, a feline has exhibited a higher level of WBC compared to a predetermined reference value in the past, e.g., a higher level of WBC is found in a historic sample of the feline or in a historical medical record of the feline (e.g., a record made more than about 1 week, about 2 weeks, about 1 month, about 2 months, about 3 months or about 6 months before practicing any one of the methods disclosed herein). In certain embodiments, the average levels of WBC in a control population is between about $1 \times 10^9$ /L and about $60 \times 10^9$ /L, between about $2 \times 10^9$ /L and about $50 \times 10^9$ /L, between about $5 \times 10^9$ /L and about $30 \times 10^9$ /L, between about $6 \times 10^9$ /L and about $20 \times 10^9$ /L or between about $8 \times 10^9$ /L and about $16 \times 10^9$ /L. In certain embodiments, the average levels of WBC in a control population is between about $5.5 \times 10^9$ /L and about $19.5 \times 10^9$ /L. In certain embodiments, the predetermined reference value of WBC is about 100%, about 105%, about 110%, about 115%, about 120%, about 125%, about 130%, about 140%, about 150%, about 200%, about 250%, about 300%, about 400%, about 500% or more, or any intermediate percentage or range of the average level of WBC in a control population. In certain embodiments, the predetermined reference value of WBC is between about 100% and about 120%, between about 120% to about 150%, between about 150% and about 200%, or between about 200% and about 500% of the average level of WBC in a control population. In certain embodiments, the predetermined reference value of WBC is between about $2 \times 10^9$ /L and about $100 \times 10^9$ /L, between about $5 \times 10^9$ /L and about $80 \times 10^9$ /L, between about $10 \times 10^9$ /L and about $70 \times 10^9$ /L, between about $20 \times 10^9$ /L and about $60 \times 10^9$ /L or between about $30 \times 10^9$ /L and about $50 \times 10^9$ /L. In certain embodiments, a lower level of WBC compared to a predetermined reference value based on average levels of WBC in a control population indicates a decreased risk of developing CKD. In certain embodiments, the predetermined reference value of WBC is about 100%, about 95%, about 90%, about 85%, about 80%, about 75%, about 70%, about 60%, about 50% or less, or any intermediate percentage or range of the average level of WBC in a control population. In certain embodiments, the predetermined reference value of WBC is between about 100% and about 90%, between about 80% and about 60%, or between about 60% and about 40% of the average level of WBC in a control population.

In certain embodiments, a decreased level of urine pH indicates an increased risk of developing CKD. In certain embodiments, an increased level of urine pH indicates a decreased risk of developing CKD. In certain embodiments, a lower level of urine pH compared to a predetermined reference value based on average levels of urine pH in a control population indicates an increased risk of developing CKD. In certain embodiments, a higher level of urine pH compared to a predetermined reference value based on average levels of urine pH in a control population indicates a decreased risk of developing CKD. In certain embodiments, the average levels of urine pH in a control population is between about 4 and about 8.5, between about 5 and about 8, between about 5.2 and about 7.5, or between about 6 and about 7. In certain embodiments, the average levels of urine pH in a control population is between about 5.5 and about 7.5. In certain embodiments, the predetermined reference value of urine pH is about 100%, about 95%, about 90%, about 85%, about 80%, about 75%, about 70%, about 60%, about 50% or less, or any intermediate percentage or range of the average level of urine pH in a control population. In certain embodiments, the predetermined reference value of urine pH is between about 100% and about 80%, between about 80% and about 60%, or between about 60% and about 40% of the average level of urine pH in a control population. In certain embodiments, the predetermined reference value of urine pH is between about 3 and about 8, between about 4 and about 7.5, between about 4.5 and about 7, between about 4.5 and about 6.5, between about 5 and about 6.5, or between about 5 and about 6. In certain embodiments, a feline's diet and the handling of the urine sample of the feline is considered to adjust the urine specific gravity level.

In certain embodiments, an increased or a decreased level of a biomarker is detected at present, e.g., an increased or a decreased level of a biomarker is found in a current sample of a feline or in a recent medical record of the feline (e.g., a record made within about 1 week, about 2 weeks, about 3 weeks, about 4 weeks, about 5 weeks, about 10 weeks, about 3 months or about 6 months before practicing any one of the methods disclosed herein). In certain embodiments, a feline has exhibited an increased or a decreased level of a biomarker in the past, e.g., an increased or a decreased level of urine protein is found in a historic sample of the feline or in a historical medical record of the feline (e.g., a record made more than about 1 week, about 2 weeks, about 1 month, about 2 months, about 3 months or about 6 months before practicing any one of the methods disclosed herein).

In general, the ranges of the average levels for the biomarkers can account for 80-90% or more of the healthy, normal population. Therefore, about 5-10% of the population can have values above the higher end of an average/normal range, and about another 5-10 % of the population can have values below the low end of an average/normal range. However, these values can be normal for a particular feline. In certain embodiments, the actual ranges and validity of the biomarkers can be determined by each laboratory or testing, depending on the machine and/or on the population of felines tested to determine an average/normal range. Additionally, laboratory tests can be impacted by sample handling and machine maintenance/calibration. Updates to machines can also result in changes in the normal ranges. Any one of these factors can be considered for adjusting the average levels and/or the predetermined reference values of each biomarker.

In certain embodiments, the biomarker comprises at least one further biomarker. In certain embodiments, the at least one further biomarker is a biomarker identified in Table 1 in Example 1. In certain embodiments, the at least one further biomarker is selected from the group consisting of phosphate and parathyroid hormone (PTH), symmetric dimethyl arginine (SDMA), systolic blood pressure, potassium, total calcium, hyaluronic acid, death receptor 5, transforming growth factor β1, ferritin, beta globin, catalase, alpha globin, epidermal growth factor receptor pathway substrate 8, mucin isoform precursor, ezrin, delta globin, moesin, phosphoprotein isoform, annexin A2, myoglobin, hemopexin, serine proteinase inhibitor, serpine peptidase inhibitor, CD14 antigen precursor, fibronectin isoform preprotein, angiotensinogen preprotein, complement component precursor, carbonic anhydrase, uromodulin precursor, complement factor H, complement component 4 BP, heparan sulfate proteoglycan 2, olfactomedian-4, leucine rich alpha-2 glycoprotein, ring finger protein 167, inter-alpha globulin inhibitor H4, heparan sulfate proteoglycan 2, N-acylshingosine aminohydrolase, serine proteinase inhibitor clade A member 1, mucin 1, clusterin isoform 1, brain abundant membrane attached signal protein 1, dipeptidase 1, fibronectin 1 isoform 5 preprotein, angiotensinogen preproprotien, carbonic anhydrase, uromodulin precursor, Metalloproteinase inhibitor 2, Insulin-like growth factor-binding protein 7, Immunoglobulin A, Immunoglobulin G1, Immunoglobulin G2, Alpha-1 antitrypsin, Serum amyloid P component, Hepatocyte growth factor, Intercellular adhesion molecule 1, Beta-2-glycoprotein 1, Interleukin-1 beta, Neutrophil Elastase, Tumor necrosis factor receptor superfamily member 11B, Interleukin-11, Cathepsin D, C-C motif chemokine 24, C-X-C motif chemokine 6, C-C motif chemokine 13, C-X-C motif chemokines -1, -2, and -3, Matrilysin, Interleukin-2 receptor alpha chain, Insulin-like growth factor-binding protein 3, Macrophage colony-stimulating factor 1, apolipoprotein C-I, apolipoprotein C-II, fibrinogen alpha chain, fibrinogen A-alpha chain, kininogen, Inter-Alpha Inhibitor H4 (ITIH4), keratin Type I cytoskeletol 10 cystatin A, cystatin B, and any combination thereof. See for example U.S. Publication No. 2012/0077690 A1, U.S. Publication No. 2013/0323751 A1, EP 3,112,871 A1, EP 2,462,445 A1, and EP 3,054,301 A1.

In certain embodiments, the at least one further biomarker is in the blood of the feline. In certain embodiments, the at least one further biomarker is in the serum of the feline. In certain embodiments, the at least one further biomarker is in the plasma of the feline. In certain embodiments, the at least one further biomarker is in a urine of the feline.

In certain embodiments, the predetermined reference value of a biomarker can be based on an average amount of the biomarker in test samples in a control population. The control population can be a group of at least 3, preferably at least 10, more preferred at least 50 felines with a similar genetic background, age and average health status.

In certain embodiments, a predetermined reference value of a biomarker can be less than about 90%, about 80%, about 70%, about 60%, about 50%, about 40%, about 30%, about 20%, about 10%, about 5%, about 2%, or about 1%, of the average level of the biomarker in a control population. In certain embodiments, a predetermined reference value of a biomarker can be more than about 110%, about 120%, about 130%, about 140%, about 150%, about 160%, about 170%, about 180%, about 190%, about 200%, about 250%, about 300%, about 400%, about 500%, about 600%, about 700%, about 800%, about 900% or more of the average level of the biomarker in blood in a control population.

In certain embodiments, the amounts of the biomarkers in the feline can be detected and quantified by any means known in the art. In certain embodiments, the level of creatinine, urine protein, WBC, urea and/or BUN is determined by a fluorescence method or a luminescence method. In certain embodiments, the level of creatinine, urine protein, WBC, urea and/or BUN is determined by an antibody-based detection method, e.g., an enzyme-linked immunosorbent assay (ELISA), e.g., a sandwich ELISA. In certain embodiments, the level of urine protein is determined by using a urine albumin antibody. In certain embodiments, the level of urine specific gravity can be measured by refractometry, hydrometry and reagent strips. In certain embodiments, the level of urine pH can be measured by a pH test strip, or a pH meter and a pH probe. In certain embodiments, the level of WBC can be measured by flow cytometry.

In certain embodiments, other detection methods, such as other spectroscopic methods, chromatographic methods, labeling techniques, or quantitative chemical methods can be used. In certain embodiments, the level of a biomarker from a feline and a predetermined reference value of the biomarker are determined by the same method.

## 3. TEST METHODS

The presently disclosed subject matter provides test methods for determining susceptibility of a feline to developing chronic kidney disease (CKD).

In certain non-limiting embodiments, the method comprises: obtaining an amount of one or more biomarkers in the feline; and comparing the amount of each of the one or more biomarkers to a predetermined reference value. In certain embodiments, the predetermined reference value is based on an average amount of the biomarker in a sample in a control population. In certain embodiments, the one or more biomarkers comprises creatinine, urine specific gravity and BUN or urea. In certain embodiments, an amount of creatinine above a first predetermined value, an amount of urine specific gravity below a second predetermined reference value, and an amount of BUN or urea above a third predetermined reference value indicate a risk of CKD. In certain embodiments, the first predetermined reference value is between about 0.5 mg/dL and about 3 mg/dL, between about 1 mg/dL and about 2.4 mg/dL, between about 1 mg/dL and about 2 mg/dL, or between about 1.2 mg/dL and about 1.8 mg/dL. In certain embodiments, the second predetermined reference value is between about 1.001 and about 1.08, between about 1.001 and about 1.07, between about 1.001 and about 1.06, between about 1.001 and about 1.05. or between about 1.001 and about 1.04. In certain embodiments, when BUN measurement is used, the third predetermined reference value is between about 10 mg/dL and about 100 mg/dL, between about 15 mg/dL and about 90 mg/dL, between about 20 mg/dL and about 80 mg/dL, between about 30 mg/dL and about 70 mg/dL, between about 40 mg/dL and about 70 mg/dL, or between about 40 mg/dL and about 60 mg/dL. In certain embodiments, when urea measurement is used, the third predetermined reference value is between about 21.4 mg/dL and about 214 mg/dL, between about 32.1 mg/dL and about 192.6 mg/dL, between about 42.8 mg/dL and about 171.2 mg/dL, between about 64.2 mg/dL and about 149.8 mg/dL, between about 85.6 mg/dL and about 149.8 mg/dL, or between about 85.6 mg/dL and about 128.4 mg/dL.

In certain non-limiting embodiments, the one or more biomarkers comprises urine specific gravity, creatinine, urine protein, blood urea nitrogen (BUN) or urea, white blood cell count (WBC) and/or urine pH. In certain embodiments, an amount of creatinine above a first predetermined value, an amount of urine specific gravity below a second predetermined reference value, an amount of BUN or urea above a third predetermined reference value, an amount of urine protein above a fourth predetermined value, an amount of WBC above a fifth predetermined reference value, and an amount of urine pH below a sixth predetermined reference value indicate a risk of CKD. In certain embodiments, the first predetermined reference value is between about 0.5 mg/dL and about 3 mg/dL, between about 1 mg/dL and about 2.4 mg/dL, between about 1 mg/dL and about 2 mg/dL, or between about 1.2 mg/dL and about 1.8 mg/dL. In certain embodiments, the second predetermined reference value is between about 1.001 and about 1.08, between about 1.001 and about 1.07, between about 1.001 and about 1.06, between about 1.001 and about 1.05. or between about 1.001 and about 1.04. In certain embodiments, when BUN measurement is used, the third predetermined reference value is between about 10 mg/dL and about 100 mg/dL, between about 15 mg/dL and about 90 mg/dL, between about 20 mg/dL and about 80 mg/dL, between about 30 mg/dL and about 70 mg/dL, between about 40 mg/dL and about 70 mg/dL, or between about 40 mg/dL and about 60 mg/dL. In certain embodiments, when urea measurement is used, the third predetermined reference value is between about 21.4 mg/dL and about 214 mg/dL, between about 32.1 mg/dL and about 192.6 mg/dL, between about 42.8 mg/dL and about 171.2 mg/dL, between about 64.2 mg/dL and about 149.8 mg/dL, between about 85.6 mg/dL and about 149.8 mg/dL, or between about 85.6 mg/dL and about 128.4 mg/dL. In certain embodiments, the fourth predetermined reference value is between about 0.001 mg/dL and about 100 mg/dL, between about 1 mg/dL and about 80 mg/dL, between about 5 mg/dL and about 70 mg/dL, between about 10 mg/dL and about 60 mg/dL, or between about 20 mg/dL and about 50 mg/dL. In certain embodiments, the fifth predetermined reference value is between about $2 \times 10^9$ /L and about $100 \times 10^9$ /L, between about $5 \times 10^9$ /L and about $80 \times 10^9$ /L, between about $10 \times 10^9$ /L and about $70 \times 10^9$ /L, between about $20 \times 10^9$ /L and about $60 \times 10^9$ /L or between about $30 \times 10^9$ /L and about $50 \times 10^9$ /L. In certain embodiments, the sixth predetermined reference value is between about 3 and about 8, between about 4 and about 7.5, between about 4.5 and about 7, between about 4.5 and about 6.5, between about 5 and about 6.5, or between about 5 and about 6.

In certain non-limiting embodiments, the method of predicting a risk of chronic kidney disease (CKD) for a feline comprises: receiving at least one input level of one or more biomarkers from samples taken from the feline; analyzing and transforming the at least one input level of the one or more biomarkers to derive a probability score or a classification label via a classification algorithm; and generating an output. In certain embodiments, the method of predicting a risk of chronic kidney disease (CKD) for a feline comprises: receiving at least one input level of one or more biomarkers from samples taken from the feline and an input level of an age of the feline; analyzing and transforming the at least one input level of the one or more biomarkers and the input level of the age to derive a probability score or a classification label via a classification algorithm; and generating an output. In certain embodiments, the method further comprises determining a customized recommendation based on the determining or categorizing. In certain embodiments, the code, when executed by the processor, further causes the system to display the determination or categorization and customized recommendation on a graphical user interface. In certain embodiments, the age of the feline is the age when a method disclosed herein is carried out.

In certain embodiments, the at least one of the one or more biomarkers comprises information relating to a urine specific gravity level, a creatinine level, a urine protein level, a blood urea nitrogen (BUN) or urea level, a white blood cell count (WBC), urine pH, or a combination thereof. In certain embodiments, the biomarkers further comprise one or more parameters selected from Table 1 in Example 1. In certain embodiments, the analyzing and transforming the at least one input level of the one or more biomarkers and optionally the input level of the age comprises organizing and modifying each input level. In certain embodiments, the at least one input level is normalized. In certain embodiments, the at least one input level is transformed into composite levels of one or more biomarkers. In certain embodiments, the input level of the age is transformed into a composite level of the age. In certain embodiments, the at least one input level is transformed and/or adjusted according to biological information of the feline, e.g., weight, age, height, medical history, breed, etc. In certain embodiments, the at least one input level comprises sequential measurements of the one or more biomarkers measured at different time points.

In certain embodiments, the classification algorithm comprises code developed from a training dataset. In certain embodiments, the classification algorithm is developed using a machine learning technique, e.g., a training algorithm.

In certain embodiments, the classification algorithm is a hard classifier that determines the classification label of whether the feline is at risk of developing CKD or a soft classifier, which determines the probability score of the feline developing CKD.

In certain embodiments, the output is the classification label or the probability score.

In certain embodiments, the step of obtaining the data comprises measuring an amount of each of the one or more biomarkers in a sample from the feline. In certain embodiments, the step of obtaining the data from the test sample comprises receiving the data from a third party that has measured an amount of each of the one or more biomarkers in a sample from the feline to determine the data. In certain embodiments, the sample from the individual is a blood sample or a urine sample.

In certain embodiments, the training dataset comprising medical information relating to both a first plurality of biomarkers from a first set of sample felines and a second plurality of biomarkers from a second set of sample felines. In certain embodiments, the first set of sample felines have been diagnosed with CKD and the second set of sample felines have not been diagnosed with CKD. In certain embodiments, the training dataset comprising amounts of the biomarkers from felines that have been diagnosed with CKD and felines that have not been diagnosed with CKD. In certain embodiments, the first plurality of biomarkers comprises at least one of a urine specific gravity level, a creatinine level, a urine protein level, a blood urea nitrogen (BUN) or urea level, a white blood cell count (WBC), urine pH, or any combination thereof. In certain embodiments, the first plurality of biomarkers comprises any one of the biomarkers disclosed in the instant application. In certain embodiments, the second plurality of biomarkers comprises at least one of a urine specific gravity level, a creatinine level, a urine protein level, a blood urea nitrogen (BUN) or urea level, a white blood cell count (WBC), urine pH, or any combination thereof. In certain embodiments, the second plurality of biomarkers comprises any one of the biomarkers disclosed in the instant application.

In certain embodiments, if the data is classified as meaning a risk of CKD, the feline is predicted to have a greater likelihood of developing CKD as compared to if the data is classified as meaning a low risk of CKD.

In certain non-limiting embodiments, the method of determining susceptibility of a feline to developing chronic kidney disease (CKD) comprises:
obtaining data comprising amounts of a plurality of biomarkers in the feline and optionally an age of the feline; and performing an analysis on the data with an analytical algorithm, e.g., a classification algorithm, i.e., a classifier. In certain embodiments, the classification algorithm is developed by a machine learning algorithm. In certain embodiments, the classification algorithm is developed from a training dataset.

In certain non-limiting embodiments, a method of determining susceptibility of a feline to developing chronic kidney disease (CKD) comprises:

receiving at least one input level of one or more biomarkers from the feline, optionally receiving an input level of an age of the feline, wherein at least one of the one or more biomarkers comprises a urine specific gravity level, a creatinine level, a urine protein level, a blood urea nitrogen (BUN) or urea level, a white blood cell count (WBC), urine pH, or any combination thereof;

analyzing and transforming the at least one input level of the one or more biomarkers and optionally the input level of the age, by organizing and/or modifying each input level to derive a probability score or a classification label via a classification algorithm, wherein the classification algorithm comprises code developed from a training dataset, the training dataset comprising medical information relating to a first plurality of biomarkers and optionally ages from a first set of sample felines and a second plurality of biomarkers and optionally ages from a second set of sample felines, wherein the classification algorithm is developed using a training algorithm;

wherein the classification algorithm determines the classification label of whether the feline is at risk of developing CKD or determines the probability score of the feline developing CKD;

generating an output, wherein the output is the classification label or the probability score;

providing a customized recommendation, e.g., a dietary regimen and/or further monitoring the one or more biomarkers based on the output; and

displaying the output and/or customized recommendation on a graphical user interface.

In certain embodiments, the one or more biomarkers comprises information relating to a urine specific gravity level, a creatinine level and a blood urea nitrogen (BUN) or urea level. In certain embodiments, the one or more biomarkers comprises information relating to a urine specific gravity level, a creatinine level, a urine protein level, a blood urea nitrogen (BUN) or urea level, a white blood cell count (WBC) and urine pH.

In certain embodiments, the method comprises receiving at least one input level of one or more biomarkers from the feline and an input level of an age of the feline.

In certain embodiments, the method comprises receiving input levels of biomarkers comprising information relating to a urine specific gravity level, a creatinine level and a blood urea nitrogen (BUN) or urea level; and an input level of an age of the feline.

In certain embodiments, the classification algorithm comprises an algorithm selected from: a logistic regression algorithm, an artificial neural network algorithm (ANN), a recurrent neural network algorithm (RNN), a K-nearest neighbor algorithm (KNN), a Naive Bayes algorithm, a support vector machine algorithm (SVM), a random forest algorithm, an AdaBoost algorithm and any combination thereof. In certain embodiments, the classification algorithm comprises a regularization algorithm. In certain embodiments, a regularization algorithm prevents overfitting.

In certain embodiments, the classification algorithm comprises a standard RNN algorithm comprising an input layer, an output layer and a hidden layer. In certain embodiments, the RNN comprises vanilla nodes and/or layers. In certain embodiments, the RNN comprises long short-term memory (LSTM) nodes and/or layers. In certain embodiments, the RNN comprises about 1, about 2, about 3, about 4, about 5, about 6, about 7, about 8, about 9, about 10 or more hidden layers. In certain embodiments, the RNN comprises between about 1 and about 3, between about 2 and about 4, between about 3 and about 5, between about 5 and about 10, between about 1 and about 4, between about 1 and about 5, or between about 2 and about 6 hidden layers.

In certain embodiments, each layer comprises at least about 1, at least about 2, at least about 3, at least about 4, at least about 5, at least about 6, at least about 7, at least about 8, at least about 9, at least about 10, at least about 20, at least about 30, at least about 40, at least about 50, at least about 60, at least about 70, at least about 80, at least about 90, at least about 100, at least about 150, at least about 200, at least about 250, at least about 300, at least about 400, at least about 500 nodes, or any intermediate number or range of nodes. In certain embodiments, each layer comprises between about 2 and about 10, between about 2 and about 20, between about 3 and about 30, between about 2 and about 50, between about 3 and about 100, between about 4 and about 200, between about 5 and about 300, between about 10 and about 500, between about 2 and about 1000, between about 4 and about 500 nodes. In certain embodiments, each layer comprises between about 5 and about 300 nodes. In certain embodiments, each layer comprises between about 6 and about 250 nodes. In certain embodiments, each layer comprises between about 7 and about 200 nodes. In certain embodiments, a hidden layer comprises a tanh activation function.

In certain embodiments, the input levels of the biomarkers and the age of the feline relate to medical records of one or more visit of the feline. In certain embodiments, the input levels of the biomarkers and the age of the feline relate to medical records of at least about 2 visits, at least about 3 visits, at least about 4 visits, at least about 5 visits, at least about 6 visits, at least about 7 visits, at least about 8 visits, at least about 9 visits, at least about 10 visits or more of the feline. In certain embodiments, the input levels of the biomarkers and the age of the feline relate to medical records of between about 1 visit to about 10 visits, between about 2 visits to about 10 visits, between about 3 visits to about 10 visits, between about 1 visit to about 5 visits, between about 1 visit to about 3 visits, between about 2 visits to about 5 visits, between about 3 visits to about 5 visits of the feline.

In certain embodiments, the classification label or the probability score is transformed from a combination of intermediate probability scores, each of which is determined based on the input levels of the biomarkers and the age of the feline relating to a medical record of one visit of the feline.

In certain embodiments, the classification label or the probability score relates to the feline's status of contracting chronic kidney disease (CKD) at the time of the determination of the classification label or the probability score. In certain embodiments, the classification label or the probability score relates to the feline's risk of developing chronic kidney disease (CKD) after the determination of the classification label or the probability score.

In certain embodiments, the classification label or the probability score relates to the feline's risk of developing chronic kidney disease (CKD) about 1 month, about 2 months, about 3 months, about 4 months, about 5 months, about 6 months, about 7 months, about 8 months, about 9 months, about 10 months, about 11 months, about 12 months or more after the determination of the classification label or the probability score. In certain embodiments, the classification label or the probability score relates to the feline's risk of developing chronic kidney disease (CKD) about 1 year, about 2 years, about 3 years, about 4 years, about 5 years or more after the determination of the classification label or the probability score.

In certain embodiments, the classification label or the probability score relates to the feline's risk of developing chronic kidney disease (CKD) between about 1 month and about 12 months, between about 1 month and about 6 months, between about 1 month and about 3 months, between about 3 months and about 12 months, between about 6 months and about 12 months, between about 3 months and about 6 months after the determination of the classification label or the probability score. In certain embodiments, the classification label or the probability score relates to the feline's risk of developing chronic kidney disease (CKD) between about 1 year and about 5 years, between about 1 year and about 3 years, between about 1 year and about 2 years, between about 2 years and about 5 years, between about 2 years and about 3 years, between about 3 years and about 5 years after the determination of the classification label or the probability score.

In certain embodiments, the customized recommendation comprises diagnosing the presence of a comorbidity in the feline. In certain embodiments, the comorbidity is selected from the group consisting of hyperthyroidism, diabetes mellitus, hepatopathy, underweight, murmur, arthritis, malaise, constipation, gastroenteritis, vomiting, inflammatory bowel disease, crystalluria, enteritis, urinary tract infection, upper respiratory disease, urinary tract disease, obesity, inappropriate elimination, cystitis, colitis and any combination thereof. In certain embodiments, the comorbidity is selected from the group consisting of hyperthyroidism, diabetes mellitus, hepatopathy, underweight, murmur and any combination thereof.

In certain embodiments, the feline is a domestic cat.

*Training dataset*

In the presently disclosed subject matter, a training dataset includes medical records of plurality of felines. In certain embodiments, the medical records comprise an amount of a biomarker disclosed herein and optionally an age of a feline. In certain embodiments, the medical records comprise records of one or more visits of a feline. In certain embodiments, the medical records comprise records of at least two visits of a feline. In certain embodiments, the medical records comprise records of at least three visits of a feline at different time points. In certain embodiments, the medical records comprise records of at least four visits of a feline at different time points. In certain embodiments, the medical records comprise records of the most recent two visits of a feline at different time points. In certain embodiments, the medical records comprise records of the most recent three visits of a feline at different time points. In certain embodiments, the medical records comprise records of the most recent four visits of a feline at different time points. In certain embodiments, the medical records comprise records of the first and the last visits of a feline at different time points.

In certain embodiments, the medical records comprise records of at least about 100 different felines that have been diagnosed with CKD and at least about 100 different felines that have not been diagnosed with CKD. In certain embodiments, the medical records comprise records of at least about 200 different felines that have been diagnosed with CKD and at least about 200 different felines that have not been diagnosed with CKD. In certain embodiments, the medical records comprise records of at least about 500 different felines that have been diagnosed with CKD and at least about 500 different felines that have not been diagnosed with CKD. In certain embodiments, the medical records comprise records of at least about 1000 different felines that have been diagnosed with CKD and at least about 1000 different felines that have not been diagnosed with CKD. In certain embodiments, the medical records comprise records of at least about 2000 different felines that have been diagnosed with CKD and at least about 2000 different felines that have not been diagnosed with CKD. In certain embodiments, the medical records comprise records of at least about 5000 different felines that have been diagnosed with CKD and at least about 5000 different felines that have not been diagnosed with CKD.

In certain embodiments, the training dataset is stratified for cross validation. Cross validation is a process that assesses how the results (e.g., a classification algorithm) of a training algorithm can generalize to an independent dataset. A training dataset can be divided or stratified into 2 or more folds where one or more subsets are used to validate a classification algorithm trained by one or more different subsets. In certain embodiments, the training dataset is stratified into about 2 folds. In certain embodiments, the training dataset is stratified into about 3 folds. In certain embodiments, the training dataset is stratified into about 4 folds. In certain embodiments, the training dataset is stratified into about 5 folds. In certain embodiments, the training dataset is stratified into about 6, about 7, about 8, about 9, about 10, about 15, about 20, about 30, about 40, about 50 or more folds.

In certain embodiments, the training dataset is divided into subsets for different prediction models. In certain embodiments, a subset comprises the measures corresponding to individuals already diagnosed CKD during a given visit. In certain embodiments, a subset comprises the measurements corresponding to individuals diagnosed with CKD within 3 months after a given visit. In certain embodiments, a subset comprises the measurements corresponding to individuals diagnosed with CKD within 6 months after a given visit. In certain embodiments, a subset comprises the measurements corresponding to individuals diagnosed with CKD within 9 months after a given visit. In certain embodiments, a subset comprises the measurements corresponding to individuals diagnosed with CKD within 12 months after a given visit. In certain embodiments, a subset comprises the measurements corresponding to individuals diagnosed with CKD within 2 years after a given visit. In certain embodiments, a subset comprises the measurements corresponding to individuals diagnosed with CKD within 3 years after a given visit. In certain embodiments, a subset comprises the measurements corresponding to individuals diagnosed with CKD within 4 years after a given visit. In certain embodiments, a subset comprises the measurements corresponding to individuals diagnosed with CKD within five or more years after a given visit. In certain embodiments, the training dataset is divided into subsets comprising one or more subsets disclosed above.

In certain embodiments, if a record of a feline lacks an amount or a level of one or more biomarkers and/or lacks an age, the amount or level of the one or more biomarkers and/or an age is imputed. In certain embodiments, the imputation is carried out using a random forest implementation.

In certain embodiments, the training dataset is filtered by a set of inclusion and exclusion criteria. In certain embodiments, a visit count of a feline is no less than 2, no less than 3, no less than 4, or no less than 5 visits (e.g., not necessarily with any blood or urine data). In certain embodiments, the medical history of visits covers at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 1 year, at least about 2 years, at least about 3 years, at least about 4 years, at least about 5 or more years, In certain embodiments, a visit age of a feline is between about 1 and about 25 years, between about 1.5 and about 22 years, between about 2 and about 20 years (e.g., age less than 19.5 years averaged across all visits).

In certain embodiments, the breed of a feline is a predetermined breed. With respect to cats, the breed can be domestic short hair (DSH), domestic medium-haired (DMH), domestic long-haired (DLH), or general mixed breed cats.

In certain embodiments, the record of a feline comprises at least 2, 3, 4, 5 or more creatinine measures across at least about 1 year, at least about 2 years, at least about 3 years, at least about 4 years, at least about 5 or more years. In certain embodiments, the record of a feline comprises at least one creatinine measure within about 3, about 3.5, about 4, about 4.5, about 5, about 5.5, about 6, about 6.5, about 7, about 7.5, about 8, about 8.5, about 9 or more years before diagnosis of CKD. In certain embodiments, the record of a feline comprises at least one creatinine measure within about 3, about 3.5, about 4, about 4.5, about 5, about 5.5, about 6, about 6.5, about 7, about 7.5, about 8, about 8.5, about 9 or more years of having 2 more non-diagnosed years.

*Machine learning algorithm*

In certain embodiments, the machine learning algorithm comprises an algorithm having a learning style of any one or more of: supervised learning (e.g., using logistic regression, using back propagation neural networks), unsupervised learning (e.g., using an Apriori algorithm, using K-means clustering), semi-supervised learning, reinforcement learning (e.g., using a Q-leaming algorithm, using temporal difference learning), and any other suitable learning style.

In certain embodiments, the machine learning algorithm comprises any one or more of: a regression algorithm (e.g., ordinary least squares, logistic regression, stepwise regression, multivariate adaptive regression splines, locally estimated scatterplot smoothing, etc.), an instance-based method (e.g., k-nearest neighbor, learning vector quantization, self-organizing map, etc.), a regularization method (e.g., ridge regression, least absolute shrinkage and selection operator, elastic net, etc.), a decision tree learning method (e.g., classification and regression tree, iterative dichotomiser 3, C4.5, chi-squared automatic interaction detection, decision stump, random forest, multivariate adaptive regression splines, gradient boosting machines, etc.), a Bayesian method (e.g., naive Bayes, averaged one-dependence estimators, Bayesian belief network, etc.), a kernel method (e.g., a support vector machine, a radial basis function, a linear discriminate analysis, etc.), a clustering method (e.g., k-means clustering, expectation maximization, etc.), an associated rule learning algorithm (e.g., an Apriori algorithm, an Eclat algorithm, etc.), an artificial neural network model (e.g., a Perceptron method, a back-propagation method, a Hopfield network method, a self-organizing map method, a learning vector quantization method, etc.), a deep learning algorithm (e.g., a restricted Boltzmann machine, a deep belief network method, a convolution network method, a stacked auto-encoder method, etc.), a dimensionality reduction method (e.g., principal component analysis, partial lest squares regression, Sammon mapping, multidimensional scaling, projection pursuit, etc.), an ensemble method (e.g., boosting, bootstrapped aggregation, AdaBoost, stacked generalization, gradient boosting machine method, random forest method, etc.), a condition random field algorithm and any suitable form of algorithm.

In certain embodiments, the classification algorithm is trained using a supervised learning algorithm. In certain embodiments, the classification algorithm is trained using the algorithms selected from: a logistic regression algorithm, an artificial neural network algorithm (ANN), a recurrent neural network algorithm (RNN), a K-nearest neighbor algorithm (KNN), a Naive Bayes algorithm, a support vector machine algorithm (SVM), a random forest algorithm, an AdaBoost algorithm and a combination thereof. In certain embodiments, the classification algorithm is a regularization algorithm. In certain embodiments, a regularization algorithm prevents overfitting.

In certain embodiments, the classification algorithm is trained using KNN with dynamic time warping (DTW). In certain embodiments, the one or more biomarkers and/or the age is selected by a filter method, e.g., using Pearson correlation coefficient. In certain embodiments, the one or more biomarkers and/or the age is selected by a top-down wrapper method KNN-DTW. In certain embodiments, K is 7, e.g., 7 neighbors. In certain embodiments, the one or more biomarkers and/or the age is selected by a bottom-up wrapper. In certain embodiments, the one or more biomarkers comprises urine specific gravity, creatinine, urine protein, blood urea nitrogen (BUN) or urea, white blood cell count (WBC) and/or urine pH. In certain embodiments, the one or more biomarkers comprises one or more parameters in Tables 1 and 9. In certain embodiments, the classification algorithm is trained using stratified subsets of a training dataset to create a predictor that predict a risk of developing CKD after various time periods of a visit during which an amount of one or more biomarkers is determined. In certain embodiments, a predictor is created to predict a risk of developing CKD about 0 month, about 3 months, about 6 months, about 9 months, or about 12 months after an amount of a biomarker is determined. In certain embodiments, a predictor is created to predict a risk of developing CKD about 0 year, about 0.5 year, about 1 year, about 2 years, about 3 years, about 4 years, about 5 or more years after an amount of a biomarker is determined. In certain embodiments, a mixture of experts (MOE) approach is employed to train the classification algorithm, wherein an ensemble of predictors is combined, e.g., with simple voting or weighted voting. In certain embodiments, the classification algorithm is trained using a KNN algorithm, and wherein K is at least about 7. In certain embodiments, the classification algorithm is trained using a KNN algorithm, and wherein K is at least about 13. In certain embodiments, the classification algorithm is trained using a KNN algorithm, and wherein K is about 15. In certain embodiments, the classification algorithm is trained using a KNN algorithm, and wherein K is about 17.

In certain embodiments, the classification algorithm is trained using an RNN algorithm comprising an input layer, an output layer and a hidden layer. In certain embodiments, the RNN comprises vanilla nodes and/or layers. In certain embodiments, the RNN comprises long short-term memory (LSTM) nodes and/or layers. In certain embodiments, the RNN comprises about 1, about 2, about 3, about 4, about 5, about 6, about 7, about 8, about 9, about 10 or more hidden layers. In certain embodiments, the RNN comprises between about 1 and about 3, between about 2 and about 4, between about 3 and about 5, between about 5 and about 10, between about 1 and about 4, between about 1 and about 5, or between about 2 and about 6 hidden layers. In certain embodiments, each layer comprises at least about 1, at least about 2, at least about 3, at least about 4, at least about 5, at least about 6, at least about 7, at least about 8, at least about 9, at least about 10, at least about 20, at least about 30, at least about 40, at least about 50, at least about 60, at least about 70, at least about 80, at least about 90, at least about 100, at least about 150, at least about 200, at least about 250, at least about 300, at least about 400, at least about 500 nodes, or any intermediate number or range of nodes. In certain embodiments, each layer comprises between about 2 and about 50, between about 3 and about 100, between about 4 and about 200, between about 5 and about 300, between about 10 and about 500, between about 2 and about 1000, between about 4 and about 500 nodes. In certain embodiments, each layer comprises between about 5 and about 300 nodes. In certain embodiments, each layer comprises between about 6 and about 250 nodes. In certain embodiments, each layer comprises between about 7 and about 200 nodes. In certain embodiments, a hidden layer comprises a tanh activation function. In certain embodiments, an output layer comprises a softmax function. In certain embodiments, a binary cross-entropy can be used for loss calculation. In certain embodiments, the classification algorithm a regularization algorithm to prevent overfitting. In certain embodiments, a regularization algorithm causes about 5%, about 10%, about 15%, about 20%, about 25%, about 30%, about 35%, about 40% or any intermediate percentage or range of dropout to avoid overfilling. In certain embodiments, a regularization algorithm causes between about 5% and about 10%, between about 10% and about 20%, between about 20% and about 30%, or between about 30% and about 40% dropout to avoid overfilling.

In certain embodiments, subsequent steps can include assessing or validating the machine learning algorithm. For example, the machine learning algorithm can be updated based on the assessment/validation. In certain embodiments, the training dataset is stratified in to about 2 folds, about 3 folds, about 4 folds, about 5 folds, about 6 folds, about 7 folds, about 8 folds, about 9 folds, about 10 folds, about 20, about 30 folds, about 40 folds, about 50 folds or more folds, or any intermediate number of folds for cross validation.

In certain embodiments, performance of the classification algorithm is characterized by an area under the curve (AUC) ranging from about 0.50 to about 0.99. In certain embodiments, performance of the classification algorithm is characterized by an area under the curve (AUC) ranging from about 0.60 to about 0.99. In certain embodiments, performance of the classification algorithm is characterized by an area under the curve (AUC) ranging from about 0.70 to about 0.99. In certain embodiments, performance of the classification algorithm is characterized by an area under the curve (AUC) ranging from about 0.80 to about 0.99. In certain embodiments, performance of the classification algorithm is characterized by an area under the curve (AUC) ranging from about 0.80 to about 0.95.

*Linear method*

In certain non-limiting embodiments, the method of predicting a risk of chronic kidney disease (CKD) for a feline comprises: calculating a score based on an amount of one or more biomarker of the feline and comparing the score with a threshold value. In certain embodiments, the score is calculated by summing the product of each biomarker and a coefficient thereof. In certain embodiments, the coefficient of the one or more biomarker is determined by applying a linear discriminant analysis (LDA) to a dataset including medical records of plurality of felines, wherein the medical records comprise measurements of the one or more biomarker. In certain embodiments, the threshold value is determined by applying a linear discriminant analysis (LDA) to a dataset including medical records of plurality of felines, wherein the medical records comprise measurements of the one or more biomarker. In certain embodiments, the score being greater than the threshold value indicates a risk of CKD. In certain embodiments, the score being smaller than the threshold value indicates a risk of CKD.

In certain embodiments, the one or more biomarker comprises creatinine, urine specific gravity and/or BUN or urea. In certain embodiments, the amount of creatinine is measured in milligram per deciliter (mg/dL). In certain embodiments, the amount of urine specific gravity is measured as a ratio of the density of a urine sample to the density of water. In certain embodiments, the measurement of BUN or urea is measured in milligram per deciliter (mg/dL).

In certain embodiments, the coefficient of creatinine is between about 0.000001 to about 10, between about 0.00001 to about 1, between about 0.00005 to about 0.5, between about 0.0001 to about 0.10 or between about 0.0005 to about 0.05. In certain embodiments, the coefficient of creatinine is between about 0.001 to about 0.02, between about 0.002 to about 0.015, between about 0.003 to about 0.012, between about 0.004 to about 0.01, between about 0.005 to about 0.009, between about 0.0055 to about 0.0085, between about 0.0057 to about 0.0083 or between about 0.006 to about 0.007. In certain embodiments, the coefficient of creatinine is about 0.0057, about 0.0058, about 0.0061, about 0.0068, about 0.0069 or about 0.0083.

In certain embodiments, the coefficient of urine specific gravity is between about - 0.01 to about -1000, between about -0.05 to about -500, between about -0.1 to about -300 or between about -0.5 to about -200. In certain embodiments, the coefficient of urine specific gravity is between about -1 to about -100, between about -5 to about -80, between about -10 to about -70, between about -15 to about -60, between about -20 to about -50, between about -25 to about -45 or between about -30 to about -40. In certain embodiments, the coefficient of creatinine is about -25.7343, about -36.9897, about -40.0563, about -44.3369, about - 47.042 or about -49.9186.

In certain embodiments, the coefficient of urea is between about 0.00001 to about 100, between about 0.0001 to about 10, between about 0.0005 to about 5, between about 0.001 to about 1 or between about 0.005 to about 0.8. In certain embodiments, the coefficient of urea is between about 0.01 to about 0.5, between about 0.02 to about 0.4, between about 0.03 to about 0.3, between about 0.04 to about 0.2, between about 0.05 to about 0.15, between about 0.06 to about 0.12, between about 0.07 to about 0.11 or between about 0.08 to about 0.1. In certain embodiments, the coefficient of urea is about 0.0659, about 0.1044, about 0.1077, about 0.1085, about 0.1137 or about 0.1182. In certain embodiments, when BUN measurement is used, the coefficient of urea is multiplied by 2.14 times.

In certain embodiments, the score is calculated by the formula as follows:

$$\text{Score} = \text{the measurement of creatinine} \times \text{the coefficient of creatinine} + \text{the measurement of urine specific gravity} \times \text{the coefficient of urine specific gravity} + \text{the measurement of BUN or urea} \times \text{the coefficient of BUN or urea}.$$

In certain embodiments, the threshold value is between about -0.01 to about -1000, between about -0.05 to about -500, between about -0.1 to about -300 or between about -0.5 to about -200. In certain embodiments, the threshold value is between about -1 to about -100, between about -5 to about -80, between about -10 to about -70, between about -15 to about - 60, between about -20 to about -50, between about -25 to about -45 or between about -30 to about -40. In certain embodiments, the threshold value is about -38.7128, about -22.603, about -34.8051, about -42.7709, about -45.625 or about -48.7966.

In certain embodiments, the threshold value and the coefficients of creatinine, urine specific gravity and urea is selected according to Table 19 in Example 4. In certain embodiments, when BUN measurement is used, the coefficient of urea is multiplied by 2.14 times.

In certain embodiments, the score being greater than the threshold value indicates a risk of CKD. In certain embodiments, the score being smaller than the threshold value indicates an absence of risk of CKD.

In certain embodiments, the method predicts risk of CKD about 0 month, about 3 months, about 6 months, about 9 months, about 12 months, about 18 months and/or about 24 months after an amount of a biomarker is determined. In certain embodiments, the method predicts a risk of developing CKD about 0 year, about 0.5 year, about 1 year, about 2 years, about 3 years, about 4 years, about 5 and/or more years after an amount of a biomarker is determined.

4. TREATMENT METHODS

Sometimes, methods of treating, preventing or reducing a risk of developing chronic kidney disease (CKD) for a feline comprise providing a feline owner with a dietary regimen to treat or prevent CKD for a feline.

The compositions and methods of the presently disclosed subject matter can be useful for a variety of feline animals, e.g. domestic cats.

In certain non-limiting embodiments, the feline is at risk of chronic kidney disease.

In certain non-limiting embodiments, the feline is not known to be at risk of chronic kidney disease.

In certain non-limiting embodiments, the feline has been diagnosed with chronic kidney disease.

In certain non-limiting embodiments, the feline is not known to have chronic kidney disease.

Sometimes, methods of treating, preventing and/or reducing a risk of developing chronic kidney disease (CKD) for a feline comprise: determining whether the feline is at a risk of developing CKD using any of the prediction methods disclosed herein, where if the feline is at a risk of developing CKD, the method comprises a further analysis of one or more biomarkers disclosed in the instant application. In certain embodiments, the further analysis of the one or more biomarkers comprises determining an amount of the one or more biomarkers in a sample from the feline. In certain embodiments, the one or more biomarkers comprises urine specific gravity, creatinine, urine protein, blood urea nitrogen (BUN) or urea, white blood cell count (WBC) and/or urine pH. In certain embodiments, the method further comprises a reanalysis of the risk of developing CKD using any one of the prediction methods disclosed in the instant application and using the newly obtained measurements of the biomarkers and optionally an age of the feline.

In certain embodiments, the one or more biomarkers comprises symmetric dimethylarginine (SDMA), urine specific gravity and/or creatinine. In certain embodiments, the method further comprises diagnosing whether the feline has CKD. Any standard CKD diagnosing method can be used, e.g., a staging method developed by the International Renal Interest Society (IRIS) (www.iris-kidney.com; *see also* Elliott et al., Dietary therapy for feline chronic kidney disease, Encyclopedia of feline clinical nutrition, 2nd edition, 2015). In certain embodiments, the diagnosing method is according to the staging criteria described in Example 3 and/or Table 17 below.

In certain non-limiting embodiments, the presently disclosed subject matter provides methods of treating or preventing chronic kidney disease (CKD) for a feline, wherein the method comprises: determining whether the feline is at a risk of developing CKD using any of the prediction methods disclosed herein, where if the feline is determined to be at a risk of developing CKD, the method further comprises prescribing a treatment regimen to the feline.

In certain embodiments, the treatment regimen comprises at least one treatment regimen selected from: a dietary therapy, hemodialysis, renal replacement therapy, withdrawal of kidney damaging compounds, kidney transplantation, delaying or avoiding kidney damaging procedures, modifying diuretic administration, and a combination thereof. In certain embodiments, the treatment regimen comprises at least one treatment regimen selected from: reducing phosphate intake, reducing protein intake, administering polyunsaturated fatty acids, administering a phosphate binder therapy, administering potassium, reducing dietary sodium intake, administering alkali supplements, and a combination thereof. See for example, Jonathan D. Foster, Update on Mineral and Bone Disorders in Chronic Kidney Disease, Vet Clin North Am Small Anim Pract. 2016 Nov;46(6):1131-49.

In certain embodiments, the treatment regimen is a dietary therapy. In certain embodiments, the dietary therapy comprises a diet selected from: a low phosphorous diet; a low protein diet; a low sodium diet; a potassium supplement diet; a polyunsaturated fatty acid (PUFA, e.g., long chain omega-3 fatty acids) supplement diet; an anti-oxidant supplement diet; a vitamin B supplement diet; a liquid diet; and a combination thereof.

In certain embodiments, a low phosphorous diet comprises between about 0.01% and about 5%, between about 0.1% and about 2%, between about 0.1% and about 1%, between about 0.05% and about 2%, or between about 0.5% and about 1.5% phosphorous on a weight by weight basis of a pet food. In certain embodiments, a low phosphorous diet comprises about 0.01%, about 0.05%, about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 3%, about 4%, about 5% phosphate, or any intermediate percentage or range of phosphate on a weight by weight basis of a pet food. In certain embodiments, a low phosphorous diet comprises about 0.1 g/1000 kcal, about 0.2 g/1000 kcal, about 0.3 g/1000 kcal, about 0.4 g/1000 kcal, about 0.5 g/1000 kcal, about 0.6 g/1000 kcal, about 0.7 g/1000 kcal, about 0.8 g/1000 kcal, about 0.9 g/1000 kcal, about 1.0 g/1000 kcal, about 1.1 g/1000 kcal, about 1.2 g/1000 kcal, about 1.3 g/1000 kcal, about 1.4 g/1000 kcal, about 1.5 g/1000 kcal, about 1.6 g/1000 kcal, about 1.7 g/1000 kcal, about 1.8 g/1000 kcal, about 1.9 g/1000 kcal, about 2.0 g/1000 kcal, about 2.1 g/1000 kcal, about 2.2 g/1000 kcal, about 2.5 g/1000 kcal, about 2.8 g/1000 kcal, about 3.0 g/1000 kcal, about 3.5 g/1000 kcal, about 4 g/1000 kcal, about 5 g/1000 kcal, about 10 g/1000 kcal, about 15 g/1000 kcal, about 20 g/1000 kcal, or any intermediate percentage or range of phosphate. In certain embodiments, a low phosphorous diet comprises between about 0.1 g/1000 kcal and about 0.5 g/1000 kcal, between about 0.5 g/1000 kcal and about 1.0 g/1000 kcal, between about 1.0 g/1000 kcal and about 2.0 g/1000 kcal, between about 2.0 g/1000 kcal and about 5.0 g/1000 kcal, between about 0.01 g/1000 kcal and about 0.1 g/1000 kcal, between about 0.05 g/1000 kcal and about 1.0 g/1000 kcal, between about 0.1 g/1000 kcal and about 1 g/1000 kcal, between about 0.1 g/1000 kcal and about 2 g/1000 kcal, between about 1 g/1000 kcal and 2 g/1000 kcal of phosphate. In certain embodiments, a low phosphorous diet comprises about 0.5% phosphate on a weight by weight basis of a pet food. (e.g., about 1.2 g/1000 kcal for the dry renal diet or about 1.0 g/1000 kcal for the wet renal diet). In certain embodiments, a low phosphorous diet comprises about 0.9 or 1% phosphate on a weight by weight basis of a pet food (e.g., about 1.8 g/1000 kcal for the dry maintenance diet or about 2.3 g/1000 kcal for the wet maintenance diet).

In certain embodiments, a low sodium diet comprises between about 0.00001% and about 5%, between about 0.0001% and about 1%, between about 0.001% and about 0.1%, or between about 0.001% and about 0.05% sodium on a weight by weight basis of a pet food. In certain embodiments, a low sodium diet comprises about 0.01%, about 0.05%, about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 3%, about 4%, about 5% sodium, or any intermediate percentage or range of sodium on a weight by weight basis of a pet food. In certain embodiments, a low sodium diet comprises about 1 mg/kg/day, about 2 mg/kg/day, about 3 mg/kg/day, about 4 mg/kg/day, about 5 mg/kg/day, about 6 mg/kg/day, about 7 mg/kg/day, about 8 mg/kg/day, about 9 mg/kg/day, about 10 mg/kg/day, about 15 mg/kg/day, about 20 mg/kg/day, about 30 mg/kg/day, about 40 mg/kg/day, about 50 mg/kg/day, about 60 mg/kg/day, about 70 mg/kg/day, about 80 mg/kg/day, about 90 mg/kg/day, about 100 mg/kg/day about 120 mg/kg/day, about 150 mg/kg/day, or any intermediate amount or range of sodium. In certain embodiments, a low sodium diet comprises between about 1 mg/1000 kcal and about 50 mg/1000 kcal, between about 2 mg/1000 kcal and about 20 mg/1000 kcal, between about 5 mg/1000 kcal and about 50 mg/1000 kcal, between about 1 mg/1000 kcal and about 10 mg/1000 kcal, between about 0.1 mg/1000 kcal and about 5 mg/1000 kcal, between about 0.1 mg/1000 kcal and about 10 mg/1000 kcal, between about 0.1 mg/1000 kcal and about 20 mg/1000 kcal, between about 0.1 mg/1000 kcal and about 40 mg/1000 kcal, between about 10 mg/1000 kcal and 20 mg/1000 kcal of sodium. In certain embodiments, a low sodium diet comprises about 0.4 to about 0.9 mmol/kg/day, or about 9.2 to about 20.7 mg/kg/day. In certain embodiments, a low sodium diet comprises about 2 mmol/kg/day or about 46 mg/kg/day.

In certain embodiments, a potassium supplement diet comprises between about 0.00001% and about 5%, between about 0.0001% and about 1%, between about 0.001% and about 0.1%, or between about 0.001% and about 0.05% potassium supplement on a weight by weight basis of a pet food in addition to the potassium existing in the pet food. In certain embodiments, a potassium supplement diet comprises about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 3%, about 4%, about 5% or more potassium supplement on a weight by weight basis of a pet food in addition to the potassium existing in the pet food, or any intermediate percentage or range of potassium supplement in addition to the potassium existing in a pet food on a weight by weight basis of a pet food. In certain embodiments, a potassium supplement diet comprises about 1 mg/kg/day, about 2 mg/kg/day, about 3 mg/kg/day, about 4 mg/kg/day, about 5 mg/kg/day, about 6 mg/kg/day, about 7 mg/kg/day, about 8 mg/kg/day, about 9 mg/kg/day, about 10 mg/kg/day, about 15 mg/kg/day, about 20 mg/kg/day, about 30 mg/kg/day, about 40 mg/kg/day, about 50 mg/kg/day, about 60 mg/kg/day, about 70 mg/kg/day, about 80 mg/kg/day, about 90 mg/kg/day, about 100 mg/kg/day or more, or any intermediate amount or range of potassium supplement in addition to the potassium existing in a pet food. In certain embodiments, a potassium supplement diet comprises between about 1 mg/1000 kcal and about 10 mg/1000 kcal, between about 2 mg/1000 kcal and about 20 mg/1000 kcal, between about 5 mg/1000 kcal and about 50 mg/1000 kcal, between about 1 mg/1000 kcal and about 10 mg/1000 kcal, between about 0.1 mg/1000 kcal and about 5 mg/1000 kcal, between about 0.1 mg/1000 kcal and about 10 mg/1000 kcal, between about 0.1 mg/1000 kcal and about 20 mg/1000 kcal, between about 0.1 mg/1000 kcal and about 40 mg/1000 kcal, between about 10 mg/1000 kcal and 20 mg/1000 kcal of potassium supplement in addition to the potassium existing in a pet food.

In certain embodiments, a low protein diet comprises between about 0.0001% and about 20%, between about 0.001% and about 10%, between about 0.01% and about 5%, between about 0.05% and about 2%, or between about 0.01% and about 1% protein on a weight by weight basis of a pet food. In certain embodiments, a low protein diet comprises about 0.01%, about 0.05%, about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 3%, about 4%, about 5%, about 10%, about 15%, about 20% protein, or any intermediate percentage or range of protein on a weight by weight basis of a pet food. In certain embodiments, a low protein diet comprises about 1 g/kg/day, about 2 g/kg/day, about 3 g/kg/day, about 4 g/kg/day, about 5 g/kg/day, about 6 g/kg/day, about 7 g/kg/day, about 8 g/kg/day, about 9 g/kg/day, about 10 g/kg/day, about 15 g/kg/day, about 20 g/kg/day or any intermediate amount or range of protein. In certain embodiments, a low protein diet comprises between about 1 g/kg/day and about 20 g/kg/day, between about 1 g/kg/day and about 50 g/kg/day, between about 2 g/kg/day and about 30 g/kg/day, between about 2 g/kg/day and about 10 g/kg/day, between about 2 g/kg/day and about 8 g/kg/day, between about 5 g/kg/day and about 20 g/kg/day or any intermediate amount or range of protein. In certain embodiments, a low protein diet comprises about 4 to about 6 g/kg/day or about 5 to about 5.5 g/kg/day.

In certain embodiments, a PUFA supplement diet comprises between about 0.01% and about 30%, between about 0.1% and about 20%, between about 1% and about 10%, between about 0.1% and about 5%, or between about 1% and about 10% PUFA supplement in addition to the PUFA existing in a pet food on a weight by weight basis of a pet food. In certain embodiments, a PUFA supplement diet comprises about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 3%, about 4%, about 5%, about 10%, about 15%, about 20%, about 25%, about 30% or more PUFA supplement in addition to the PUFA existing in a pet food, or any intermediate percentage or range of PUFA supplement in addition to the PUFA existing in a pet food on a weight by weight basis of a pet food. In certain embodiments, a PUFA supplement diet comprises about 0.1 g/kg/day, about 0.5 g/kg/day, about 1 g/kg/day about 1 g/kg/day, about 2 g/kg/day, about 3 g/kg/day, about 4 g/kg/day, about 5 g/kg/day, about 6 g/kg/day, about 7 g/kg/day, about 8 g/kg/day, about 9 g/kg/day, about 10 g/kg/day, about 15 g/kg/day, about 20 g/kg/day, about 30 g/kg/day, about 40 g/kg/day, about 50 g/kg/day, about 60 g/kg/day, about 70 g/kg/day, about 80 g/kg/day, about 90 g/kg/day, about 100 g/kg/day or any intermediate amount or range of PUFA supplement in addition to the PUFA existing in a pet food. In certain embodiments, a PUFA supplement diet comprises between about 0.1 g/kg/day and about 20 g/kg/day, between about 1 g/kg/day and about 100 g/kg/day, between about 2 g/kg/day and about 200 g/kg/day, between about 5 g/kg/day and about 150 g/kg/day, between about 10 g/kg/day and about 100 g/kg/day, between about 5 g/kg/day and about 50 g/kg/day or any intermediate amount or range of PUFA supplement in addition to the PUFA existing in a pet food. In certain embodiments, a PUFA supplement diet comprises n-6 PUFA (e.g., plant oils). In certain embodiments, a PUFA supplement diet comprises n-3 PUFA (e.g., fish oils). In certain embodiments, a PUFA supplement diet comprises eicosapentaenoic acid (EPA) and/or docosahexaenoic acid (DHA).

In certain embodiments, an anti-oxidant supplement diet comprises between about 0.001% and about 5%, between about 0.01% and about 1%, between about 0.01% and about 2%, between about 0.1% and about 1%, or between about 1% and about 5% anti-oxidant existing in a pet food on a weight by weight basis of a pet food. In certain embodiments, an anti-oxidant supplement diet comprises about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 3%, about 4%, about 5% or more anti-oxidant supplement, or any intermediate percentage or range of anti-oxidant supplement, in addition to the anti-oxidant existing in a pet food on a weight by weight basis of a pet food. In certain embodiments, an anti-oxidant supplement diet comprises about 1 mg/kg/day, about 2 mg/kg/day, about 3 mg/kg/day, about 4 mg/kg/day, about 5 mg/kg/day, about 6 mg/kg/day, about 7 mg/kg/day, about 8 mg/kg/day, about 9 mg/kg/day, about 10 mg/kg/day, about 15 mg/kg/day, about 20 mg/kg/day, about 30 mg/kg/day, about 40 mg/kg/day, about 50 mg/kg/day, about 60 mg/kg/day, about 70 mg/kg/day, about 80 mg/kg/day, about 90 mg/kg/day, about 100 mg/kg/day or more, or any intermediate amount or range of anti-oxidant supplement in addition to the anti-oxidant existing in a pet food. In certain embodiments, an anti-oxidant supplement diet comprises between about 1 mg/kg/day and about 20 mg/kg/day, between about 1 mg/kg/day and about 100 mg/kg/day, between about 2 mg/kg/day and about 200 mg/kg/day, between about 5 mg/kg/day and about 150 mg/kg/day, between about 10 mg/kg/day and about 100 mg/kg/day, between about 5 mg/kg/day and about 50 mg/kg/day or any intermediate amount or range of anti-oxidant supplement in addition to the anti-oxidant existing in a pet food. In certain embodiments, the anti-oxidant is selected from the group consisting of vitamin E, vitamin C, taurine, carotenoids, flavanols and any combination thereof. In certain embodiments, a flavanol can be catechin, epicatechin, epigallocatechin galate, procyanidins, tannins or any combination thereof. In certain embodiments, the anti-oxidant supplement diet comprises a plant that has a high flavanol concentration, e.g., cocoa, grapes, and green tea.

In certain embodiments, a vitamin B supplement diet comprises vitamin B1 (thiamine), vitamin B2 (riboflavin), vitamin B3 (niacin or nicotinamide riboside), vitamin B5 (pantothenic acid),vitamin B6 (pyridoxine, pyridoxal or pyridoxamine), vitamin B7 (biotin),vitamin B9 (folate),vitamin B12 (cobalamins, e.g., cyanocobalamin or methylcobalamin), or any combination thereof. In certain embodiments, a vitamin B supplement diet comprises between about 0.001% and about 2%, between about 0.01% and about 1%, between about 0.05% and about 1%, between about 0.001% and about 0.1%, or between about 0.01% and about 0.2%, vitamin Bs in addition to the vitamin Bs existing in a pet food on a weight by weight basis of a pet food. In certain embodiments, an vitamin B supplement diet comprises about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2% or more vitamin Bs, or any intermediate percentage or range of vitamin B supplement, in addition to the vitamin Bs existing in a pet food on a weight by weight basis of a pet food. In certain embodiments, a vitamin B supplement diet comprises about 1 mg/kg/day, about 2 mg/kg/day, about 3 mg/kg/day, about 4 mg/kg/day, about 5 mg/kg/day, about 6 mg/kg/day, about 7 mg/kg/day, about 8 mg/kg/day, about 9 mg/kg/day, about 10 mg/kg/day, about 15 mg/kg/day, about 20 mg/kg/day, about 30 mg/kg/day, about 40 mg/kg/day, about 50 mg/kg/day, about 60 mg/kg/day, about 70 mg/kg/day, about 80 mg/kg/day, about 90 mg/kg/day, about 100 mg/kg/day or more, or any intermediate amount or range of vitamin B supplement in addition to the vitamin Bs existing in a pet food. In certain embodiments, a vitamin B supplement diet comprises between about 1 mg/kg/day and about 20 mg/kg/day, between about 1 mg/kg/day and about 100 mg/kg/day, between about 2 mg/kg/day and about 200 mg/kg/day, between about 5 mg/kg/day and about 150 mg/kg/day, between about 10 mg/kg/day and about 100 mg/kg/day, between about 5 mg/kg/day and about 50 mg/kg/day or any intermediate amount or range of vitamin B supplement in addition to the vitamin Bs existing in a pet food.

In certain embodiments, the dietary therapy can be any dietary therapy in the field. See for example, Elliott et al., Dietary therapy for feline chronic kidney disease, Encyclopedia of feline clinical nutrition, 2nd edition, 2015, and Elliott et al., Chronic renal disease: the importance of nutrition, Encyclopedia of feline clinical nutrition, 2nd edition, 2015.

5. Devices, Systems and Applications

In certain non-limiting embodiments, the presently disclosed subject matter also provides a device, a system and an application for the method(s) disclosed in the instant application, e.g., for determining susceptibility or reducing a risk of developing CKD for a feline. The device, system and/or application enable a user, such as a caretaker or owner to evaluate the risk of developing CKD and take action by themselves, or with the aid of a healthcare professional/veterinarian to evaluate risk of developing CKD for a feline and administer suitable treatment to the feline, if needed.

In certain embodiments, a device is used to carry out the method(s) disclosed in the instant application. In certain embodiments, the device is configured to accept a user input. In certain embodiments, the user input comprises levels of a plurality of biomarkers in the feline according to step of receiving input information, e.g., levels of one or more biomarkers, of a method disclosed in the instant application, and optionally an input level of an age of the feline. In certain embodiments, the plurality of biomarkers comprise urine specific gravity, creatinine, urine protein, blood urea nitrogen (BUN) or urea, white blood cell count (WBC) or urine pH. In certain embodiments, the device automatically (or on request) performs an analysis and transformation step of a method disclosed in the instant application, e.g., analyzing and transforming the input information of the one or more biomarkers optionally the input level of the age to derive a probability score or a classification label. In certain embodiments, the analysis and transformation step is performed using a classification algorithm developed according to any methods disclosed in the instant application. The analysis provides a classification of a risk of developing CKD in the feline, and provides output information.

In certain embodiments, the device provides a message with the output of step (b). In certain embodiments, the message comprises a warning, wherein the feline is determined as at a risk of developing CKD. In certain embodiments, the results of the method(s) are provided by the device in a user interface. In certain embodiments, the device provides a recommendation of treatment/prevention suggestions according to a treatment/prevention method disclosed in the instant application, e.g., a diet and/or a dietary regime.

In certain embodiments, the device may be specially constructed for the required purposes, or it may comprise a general purpose computer selectively activated or reconfigured by a computer program/application stored in the computer. In certain embodiments, the computer program/application comprises code for carrying out any one of the methods disclosed herein. Such a computer program/application may be stored in a computer readable storage medium, such as, but is not limited to, read-only memories (ROMs), random access memories (RAMs), EPROMs, EEPROMs, flash memory, magnetic or optical cards, any type of disk including floppy disks, optical disks, CD-ROMs, and magnetic-optical disks, or any type of media suitable for storing electronic instructions, and each coupled to a computer system interconnect.

In certain embodiment, the device comprises a processor that executes an application that directs the device to provide data fields for entry of user input relating to a step of receiving input information and an analysis and transformation step. In certain embodiment, the application uses the processor to evaluate the risk of the feline developing CKD in certain period of time after a measurement of a biomarker. In certain embodiments, the application is an easily navigable application, e.g., online, to carry out any method(s) disclosed in the instant application.

In certain embodiment, the device is a tablet, smartphone, desktop computer, laptop computer or personal digital assistant. In certain embodiment, the device is a mobile device, such as a smartphone and a tablet.

In certain embodiments, a system is also provided for the method(s) disclosed in the instant application, of determining whether am feline is at a risk of developing CKD. In certain embodiments, the system comprises a database connected to a remotely located device disclosed herein. In certain embodiments, the device comprises a processor executing an analysis that evaluates a determination according to the method(s) disclosed in the instant application. In certain embodiment, the system and/or the device further comprises a communication device for transmitting and receiving information. In certain embodiment, at least one input level of a biomarker and optionally an input level of an age is received from a remote second system, via the communication device. In certain embodiment, the system and/or the device transmits the determination or categorization and customized recommendation to the remote second system, via the communication device.

Unless specifically stated otherwise as apparent from the following discussion, it is appreciated that throughout the description, discussions utilizing terms such as "processing" or "computing" or "calculating" or "determining" or "displaying" or "analyzing" or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission, or display devices.

The algorithms and displays presented herein are not inherently related to any particular computer or other device. Various general purpose systems may be used with the application in accordance with the teachings herein, or it may prove convenient to construct a more specialized device to perform the required method operations. The structure for a variety of these systems will appear from the description above. In addition, the present embodiments are not described with reference to any particular programming language, and various examples may thus be implemented using a variety of programming languages. All preferred features and/or embodiments of the methods and the diets/dietary regimes disclosed in the instant application apply to the device, the system and the application.

## EXAMPLES

The presently disclosed subject matter will be better understood by reference to the following Example, which is provided as exemplary of the invention, and not by way of limitation.

## Example 1

A prediction model was built and validated using over 600,000 data points from more than 70,000 cats in a veterinary database. Information from the routinely measured blood and urine parameters was used. The model used thousands of computer cores over hundreds of hours, to learn the patterns of blood and urine chemistry for the cats, which remained healthy and those who developed CKD. This knowledge is then applied to each new cat which the model sees, and it predicts if the cat has a risk of developing CKD based on whether it has similarities with the historic Cases or Controls.

### *Methods*

#### *Inclusion / Exclusion Criteria*

Basic inclusion criteria for data:

1. Visit Count in database is no less than 3 visits for a cat (not necessarily with any blood or urine data);
2. Visit Duration is no less than 2 years, i.e., a cat has been seen for at least 2 years (not necessarily with blood/urine);
3. Visit Age is between 1.5 and 22 years (age less than 19.5 years averaged across all visits);
4. Breed is domestic short hair (DSH), domestic medium-haired (DMH) or domestic long-haired (DLH), i.e. general mixed breed cats;
5. At least 3 creatinine measures across at least 2 years (some of these measures may not be in the dataset if they are in the last 2 years for "healthy" cats, or after diagnoses for CKD cases); and
6. At least one creatinine measure within 6.5 years before diagnosis or 6.5 years of having 2 more non-diagnosed years. This ensures that the model saw at least one creatinine data point.

Further criteria:

1. In certain models, data was filtered to allow only cats with at least 3 visits containing creatinine values within a 3.5 year window of the diagnosis or healthy data cutoff. Phase 3 additionally allowed either 1 or 2 visits into the dataset to help the model predict better with single and double visits.
2. Data of certain models used a random half of the cats in the database, and split them randomly in half again for Training and Test.
3. The cats in certain models were randomly assigned to Training or Blind Test set by their Pet ID in a sequential manner i.e. two from every three sequential numbers across time become Training data. The remainder were used for the Blind Test. Certain models used all appropriate cats in the database.
4. For certain models, around 18,500 cats have been separated from the Controls as they have been identified as "at risk" from a combination of medical note scoring and heuristic analysis of blood urea nitrogen (BUN), creatinine and urine specific gravity (urine SG or USG) values. This is further detailed below. The Training data was then filtered for > 0 creatinine points between 0 and 3.5 years, and > 0 USG data points.

Cases are defined as having one of the diagnoses listed below in Table 2 at some point during their history as recorded in the veterinary database. Cats with only a diagnosis in their medical notes are not included as Cases at present, as there is no consistent use of the medical notes and the numbers are far too high to manually classify the cats. Acute Renal Failure has been included as the blood chemistry may be similar. We will test this to see if we need to remove ARF from the data set and only train/test on CRF.

Controls are defined as cats that have not been diagnosed with the listed kidney diseases at any point in their lives. They may have any other disease. The last two full years of their data for the model were removed (only during training/testing) so they remained free of CKD for two years from the last data point given to the model. This is because they could have been developing CKD but had not yet been diagnosed, although their blood chemistry may have been altering. Controls are then further cleaned by a heuristic approach described below.

*Blood and Urine Analytes Tested During Modelling*

The parameters in bold were selected for the current model. Additional parameters can be checked to see if model performance improves, e.g., urine glucose.

**Table 1.**

| *PARAMETER* |
| --- |
| ALT/SGPT (ALT) |
| Albumin (ALB) |
| Alkaline Phosphatase (ALKP) |
| Amylase (AMYL) |
| **BUN** |
| Bilirubin, Total (TBIL) |
| Calcium (CA) |
| Chloride |
| Cholesterol (CHOL) |
| **Creatinine (CREA)** |
| Eosinophil, % |
| Globulin (GLOB) |
| Glucose (GLU) - blood |
| Hematocrit (HCT) |
| Hemoglobin (HGB) |
| Lymphocyte, % |
| MCH (mean corpuscular hemoglobin) |
| MCHC (mean corpuscular hemoglobin concentration) |
| MCV (mean corpuscular volume) |
| MPV (mean platelet volume) |
| Monocyte, % |
| Phosphorus (PHOS) |
| Platelet Count (PLT) |
| Potassium |
| Protein, Total (TP) |
| RBC Count (RBC) |
| RDW |
| Segs, Neutrophil, % |
| Sodium |
| **Urine Protein** |
| **Urine Specific Gravity** |
| **Urine pH** |
| **WBC** |

*Diagnoses Included/Excluded*

The ailments in bold in Table 2 were classed as CKD diagnoses for the purpose of certain models, even though some may be acute. "Renal Failure, Chronic" is by far the most common of these diagnoses. The ailments not in bold in Table 2 were noted but were included in the models as Controls if there was not also a diagnosis from the red category at some point in the cat's life.

During final testing of certain models, predictions were made across all these diagnoses, and a second set of predictions was carried out using only "healthy" cats and those with the diagnosis of "Renal Failure, Chronic" (i.e. excluding all cats which had any of the other diseases in the table below from the Cases and Controls). It was found that the predictions were more accurate when the other diseases were removed.

During all the training, the diagnosis status was not investigated and all cats with an "AILMENT ID" in the bold category were assumed to be at least suspected of CKD by the veterinarian and included in the Cases. The final stage of Testing used a refined subset of cats which had more "confirmed" diagnoses (i.e. the diagnosis was not later marked as "resolved", "changed" or invalid).

**Table 2.**

| AILMENT |
| --- |
| **Nephritis** |
| **Renal Disease, Additional Day** |
| **Renal Disease, Cystic** |
| **Renal Failure, Acute** |
| **Renal Failure, Chronic** |
| Urinary System Trauma |
| Urinary Tract Disease, Feline |
| Urinary Tract Infection |

*Datasets*

Datasets have been generated, and blind testing was run. The datasets have been produced from a cleaned and augmented copy of a veterinary database, with pet visits dating back to 1995. Several iterations of datasets and models were built. Dataset sizes for training are summarized in Table 3.

**Table 3**

| Dataset | Total | Cases | Controls | Percent Cases | Rows of Data |
| --- | --- | --- | --- | --- | --- |
| Earlier versions | 8,810 | 2,095 | 6,715 | 23.78 | 61,159 |
| Later versions | 50,408 | 11,250 | 39,158 | 22.32 | 121,703 |

*Heuristics for Cleaning the Control Group*

Cats which did not have a formal diagnosis of CKD and would have been classed as Controls were analysed for evidence of renal issues. Levels of urine specific gravity, creatinine and BUN across their life were analysed by the algorithm below. In addition, certain keywords e.g. renal, K/D, azotemia, CKD were referenced from the medical notes. The medical notes were also scored by a text analysis algorithm which had been trained on the medical notes of Cases and Controls. The combination of these factors was used to filter cats out of the Controls who had a risk of heading towards CKD or already had CKD but only had it recorded in the medical notes. Cats classed as either "3" or "4" below were removed from the training and test sets and will be assessed separately.

This algorithm is overly conservative in terms of sometimes removing cats from the Controls who were probably true controls or had other diseases which could elevate the parameters under investigation. However, it was deemed more important to have clean Cases and Controls to train and test the models. It can be useful to also analyze comorbidities and other diseases which could be mistaken for CKD.

Exemplary heuristic algorithm:

```
max (case when ail_k.Diag_Age_First is not null then '0 Diagnosed CKD' else
        case when (URINE_SG_MIN < 1.025 and (CREATININE_MAX > 2.4 or
        BUN MAX >= 36 or PREDICTION_MAX > 0.4 or RENAL_NOTES_TOT > 1))
            or (CREATININE MAX > 3 and BUN_MAX >= 40 )
            then '3 CKD'
            else case when (URINE_SG_MIN <= 1.035 and (CREATININE MAX >
        1.8 or BUN_MAX >= 32))
                or (CREATININE_MAX > 2.4 and BLTN MAX > 36 )
                or (CREATININE_MAX > 1.8 and BUN MAX >= 32 and (
                PREDICTION_COUNT > 1 or RENAL_NOTES_TOT > 1 ) )
            then '2 CKD Risk'
            else '1 Normal' end
        end end) OVER (partition by enc.pet _id) Renal_Filter, -- Filter based on
        medical notes and blood chem - select only
'0 Diagnosed CKD' or '1 Normal' for modelling
        datasets.
    URINE_SG_MIN is the lowest value of USG seen for that cat across all visits
    CREATININE_MAX is the highest value of creatinine seen for that cat across all visits
    BUN_MAX is the highest value of BUN seen for that cat across all visits
    PREDICTION_MAX is the highest score for any medical note from the scoring
    algorithm used to see if CKD related words were in the notes
    PREDICTION_COUNT is the number of medical notes scored as being related to CKD
    RENAL_NOTES_TOT is the number of medical notes containing any of the words
    ('renal', 'K/D', 'azotemia', 'CKD' 'CRF')
```

## *Results*

### *Summary of the prediction model*

The model uses 6 factors which were selected for their predictive rather than diagnostic capabilities. These are: urine specific gravity, creatinine, urine protein, blood urea nitrogen (BUN), white blood cell count (WBC), urine pH. Urine specific gravity, creatinine and BUN are known to be diagnostic for CKD and are used in IRIS staging of the disease. Urine protein, WBC and urine pH are more novel and help the model to predict future disease. WBC can be used by the model in some cases to rule out other infections, and can be used to understand dehydration level and normalize the other values.

The model looks at changes in these parameters over time. For example, it can pick up a reduction in urine specific gravity, urine pH and WBC count over time as an indication of reducing renal function, even if none of these factors are outside of the normal range. This allows the veterinarian to look at the cat's medical history in more detail and begin early treatment or arrange further tests if needed.

### *Accuracy of the model*

The model was validated using the historic data of tens of thousands of cats from the veterinary database. It was shown to be effective at predicting future CKD in these cats, without giving a high number of false positives. The model performed best with several (two or more) visits with blood and urine data, and became more precise with three or more visits. Pets which had been on the Wellness plan over a period of time can get the most benefit from this model.

The model was shown to have an accuracy of over 95% with ideal data, meaning that its predictions on historic cats in the veterinary database were correct more than 9 out of 10 times. Its sensitivity (ability to predict the disease in cats that have it) was highest between 0.5 and 1 year before diagnosis, where it generally picked up more than 79% of the cats which would be diagnosed in the future. However, it had good predictive power much earlier before diagnosis, and was still able to correctly predict future diagnosis of CKD over 50% of the time when it saw data as far as three years before the cat was finally diagnosed. Performance up to 4 years before formal diagnosis also appears to be surprisingly good. This ability to highlight even some of the cats which were at risk very early, combined with the low false positive rate, can give veterinarians confidence in investigating these cats who may not otherwise have been spotted until the problem was severe and less treatable. This can give the opportunity to begin interventions very early for many cats which could then stabilize the condition before it becomes more severe, potentially prolonging the cat's healthy lifespan. It also gives an opportunity to develop diets specifically tailored for this early phase of the disease, which can stabilize the cat without need for other interventions.

Table 4 shows the results for the six-biomarker model run on blinded longitudinal data (previously unseen data across multiple visits) from the veterinary database. Cats were split into Cases and Controls based on their diagnosis, and also because they had blood and urine data which was consistent with either IRIS Stage 0 or Stage 3+. This removed a lot of the ambiguous cats and the model predicted extremely well on the remainder. The false positive rate for this subset of cats was less than 1%. Prediction at 3.5 years before diagnosis shows high accuracy. There were insufficient number of cats with 4+ years of longitudinal data.

**Table 4.**

| Years From Diagnosi s | Sensitivit y | Specificit y | Accurac y | True Positiv e | True Negativ e | False Positiv e | False Negativ e | Tota l Cats |
|---|---|---|---|---|---|---|---|---|
| 0 | 99.1 | 99.4 | 99.4 | 1391 | 5574 | 32 | 13 | 7,01 0 |
| 1 | 82.9 | 99.1 | 95.9 | 483 | 2313 | 20 | 100 | 2,91 5 |
| 2 | 68.7 | 99.4 | 93.3 | 244 | 1411 | 8 | 111 | 1,77 4 |
| 3 | 57.4 | 99.8 | 91.5 | 77 | 539 | 0 | 57 | 674 |
| 3.5 | 61.2 | 96.6 | 89.5 | 44 | 278 | 10 | 28 | 359 |

Table 5 shows the same analysis, but with the model only seeing single visits (i.e., cross sectional). As there were more single visits, predictions are shown as far as 4 years before the cats were diagnosed. The model performed extremely well on single visit data, with the accuracy nearly as good as the multiple visits. This was partly because the single-visit data was limited to visits with both a creatinine and USG measure, whereas the longitudinal model was predicting on quite a lot of missing data. The longitudinal model predictions would improve with more complete data (more Wellness visits per pet).

For reference, a Sensitivity of around 20% at random would be expected, so 47% at 4 years was far better than random, and the Specificity was extremely high (false positives around 1%) on these cleaned data.

**Table 5.**

| Years From Diagnosi s | Sensitivit y | Specificit y | Accurac y | True Positiv e | True Negativ e | False Positiv e | False Negativ e | Tota l Cats |
|---|---|---|---|---|---|---|---|---|
| 0 | 99.0 | 98.9 | 98.9 | 1094 | 4363 | 49 | 11 | 5,51 7 |
| 1 | 83.4 | 99.2 | 96.0 | 382 | 1818 | 15 | 76 | 2,29 2 |
| 2 | 70.2 | 99.0 | 93.2 | 242 | 1363 | 14 | 103 | 1,72 3 |
| 3 | 56.7 | 98.4 | 90.1 | 134 | 929 | 15 | 102 | 1,18 0 |
| 4 | 47.3 | 99.2 | 88.8 | 57 | 482 | 4 | 64 | 607 |

For completeness, the same predictions were included in Tables 6 and 7 for the whole dataset (i.e. cats with reasonable quality data, but only based on veterinary diagnosis captured in the database, not cleaned by blood chemistry staging). Some of these individual cats had very high blood chemistry and sometimes low urine specific gravity. The clinicians had often commented in the medical notes about possible kidney disease but had not necessarily made a formal diagnosis on all of these cats because of insufficient evidence. Therefore, the model sometimes predicted CKD in these additional cats but there was no official diagnosis of CKD. This led to a slight increase in false positives and lower apparent accuracy across the whole uncleaned dataset. There can also be comorbidities in some of these like hyperthyroidism which can make diagnosis difficult.

Results from longitudinal predictions are shown in Table 6, with all data including Cases with lower creatinine, and Controls with high creatinine. The Controls in this dataset contained a large percentage with Creatinine > 1.6 mg/dL (140 $\mu$mol/L).

Generally the Sensitivity remained high, but the Specificity and Accuracy dropped when the more ambiguous data were introduced, due to the false positive rate increasing. However, the results remained very powerful and robust.

**Table 6.**

| Years From Diagnosi s | Sensitivit y | Specificit y | Accurac y | True Positiv e | True Negativ e | False Positiv e | False Negativ e | Total Cats |
|---|---|---|---|---|---|---|---|---|
| 0 | 96.9 | 90.1 | 91.5 | 5653 | 21064 | 2303 | 181 | 29,20 1 |
| 1 | 79.3 | 88.8 | 86.9 | 3475 | 15564 | 1968 | 909 | 21,91 6 |
| 2 | 63.3 | 87.4 | 82.6 | 1723 | 9497 | 1371 | 997 | 13,58 8 |
| 3 | 52.5 | 85.8 | 79.1 | 505 | 3309 | 548 | 458 | 4,820 |
| 3.5 | 54.4 | 85.5 | 79.3 | 87 | 545 | 93 | 73 | 797 |

Table 7 shows the results of cross sectional (single visit) predictions with all data including Cases with lower creatinine, and Controls with high creatinine. The Controls in this dataset contained a large percentage with Creatinine > 1.6 mg/dL (140 $\mu$mol/L).

**Table 7.**

| Years From Diagnosi s | Sensitivit y | Specificit y | Accurac y | True Positiv e | True Negativ e | False Positiv e | False Negativ e | Total Cats |
|---|---|---|---|---|---|---|---|---|
| 0 | 96.8 | 85.8 | 88.0 | 4011 | 14247 | 2365 | 134 | 20,75 7 |
| 1 | 79.8 | 87.2 | 85.7 | 2800 | 12257 | 1795 | 708 | 17,55 9 |
| 2 | 64.2 | 87.2 | 82.6 | 1629 | 8849 | 1295 | 909 | 12,68 2 |
| 3 | 53.1 | 88.8 | 81.6 | 880 | 5870 | 741 | 777 | 8,268 |
| 4 | 43.5 | 85.8 | 77.3 | 347 | 2741 | 454 | 452 | 3,995 |

*Model Building*

*Dataset*

*1. Raw data*

A training dataset for 61,159 feline visit records for 8,806 unique cats from the veterinary database (6,711 healthy control and 2,095 cats that have/develop CKD) was used. There are 35 features from demographics, blood chemistry, hematology and urine levels (Table 8). Healthy controls have visit entries up to 2 years before the last (undiagnosed) visit, while CKD cats have visits up to 1 month after the visit that led to the CKD diagnosis.

**Table 8.**

| VISIT_AGE | WEIGHT | ALKALINE_PH OSPHATASE | AMYLAS E. | PROTEIN _TOTAL | BUN |
|---|---|---|---|---|---|
| CREATINI NE | PHOSPHOR US | CALCIUM | URINE_P ROTEIN | URINE_S G | POTASSIU M |
| GLUCOSE | HEMATOC RIT | HEMOGLOBIN | RBC_CO UNT | RDW | ALT_SGPT |
| ALBUMIN | BILIRUBIN | CHLORIDE | CHOLEST EROL | EOSINOP HIL | GLOBULI N |

(continued)

| VISIT_AGE | WEIGHT | ALKALINE_PH OSPHATASE | AMYLAS E. | PROTEIN _TOTAL | BUN |
|---|---|---|---|---|---|
| LYMPHOC YTE | MCH | MCHC | MCV | MPV | MONOCYT E |
| PLATELET _COUNT | SEGS_NEU TROPHIL | SODIUM | URINE_P H | WBC | **DIAG_AG E_FIRST** |
| Features in the veterinary dataset. Demographic (underlined), blood/urine and age of cat when first diagnosed with CKD, if at all (bold). | | | | | |

*2. Pre-processing*

Missing values were imputed using a Random Forest implementation [1]. Felines missing the URINE_SG value from all visits were deleted (10.1% of records). Min-max normalization for each feature was applied [2].

*3. Data overview*

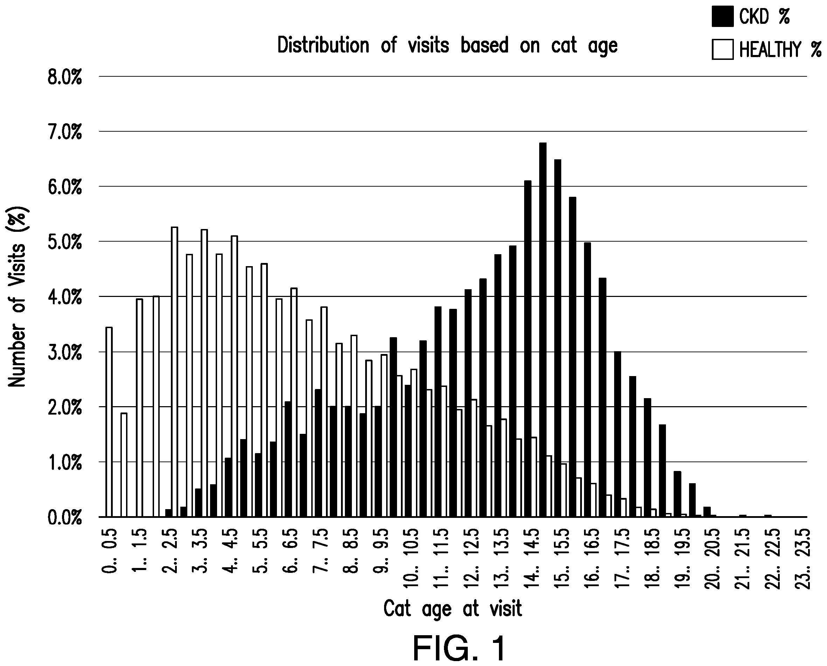

Figure 1 depicts the age distribution of CKD cats, both the age first diagnosed (red) as well as the age distribution of healthy cats (green). The median for healthy and CKD visits are 5.8±4.17 and 13.5±3.80 respectively.

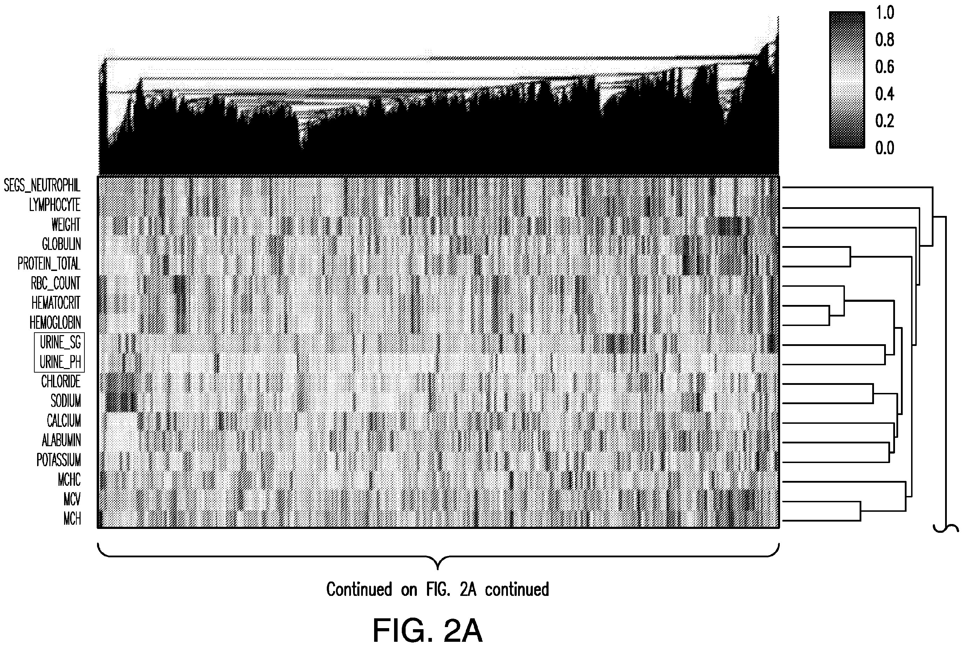

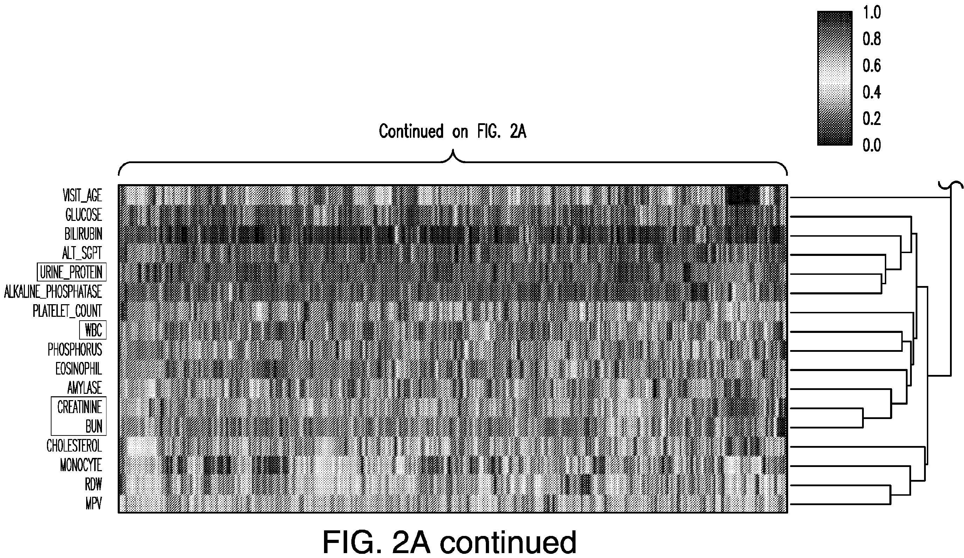

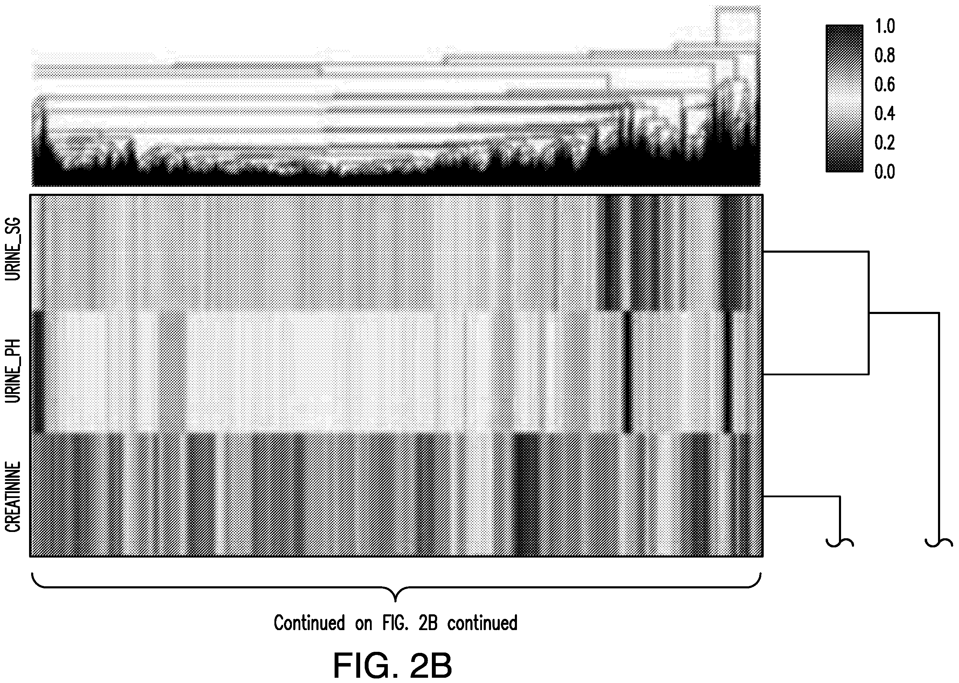

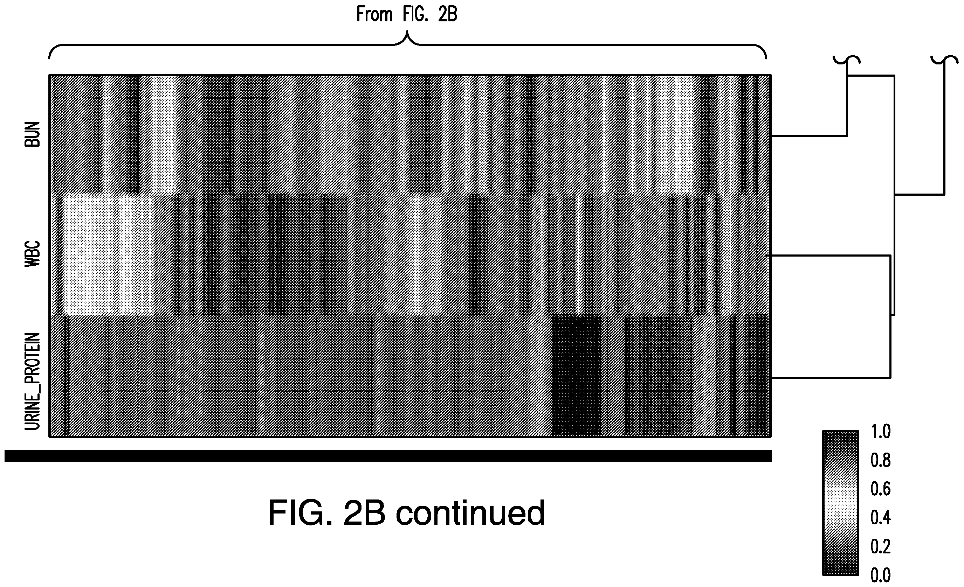

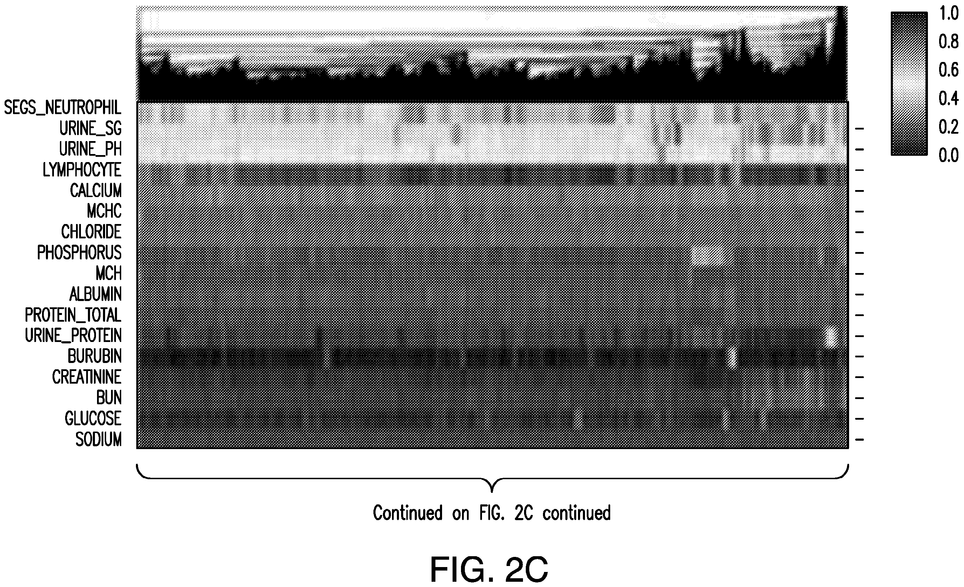

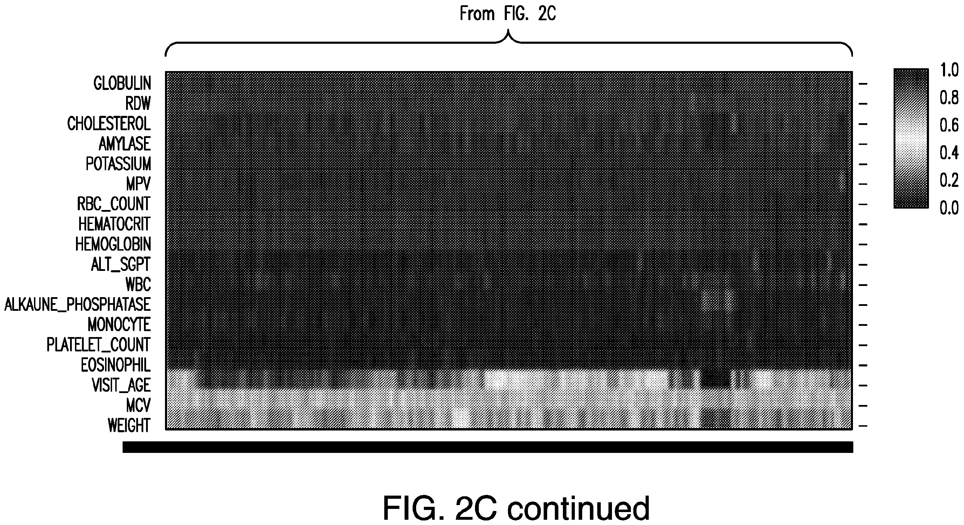

Figure 2 shows the result of hierarchical clustering (entire dataset) after min-max normalization and missing value imputation. The presence of a few outliers masks the variability of the data range (Figure 2C), so those extreme values were removed for visualization purposes (1223 values). The resulting heatmap and hierarchical clustering (agglomerative) is shown in Figure 2A. The 6 features (Urine_sg, Urine_proterin, Urine_pH, WBC, Creatinine, BUN) that were found to be the most informative in feature selection are highlighted in red and are also shown in Figure 2B. Hierarchical clustering put Creatinine and BUN together, as well as Urine_SG and Urine_pH together, arguing of the high correlation of their values in the respective samples. Urine_Protein and WBC are closer to the Creatinine/BLTN cluster.

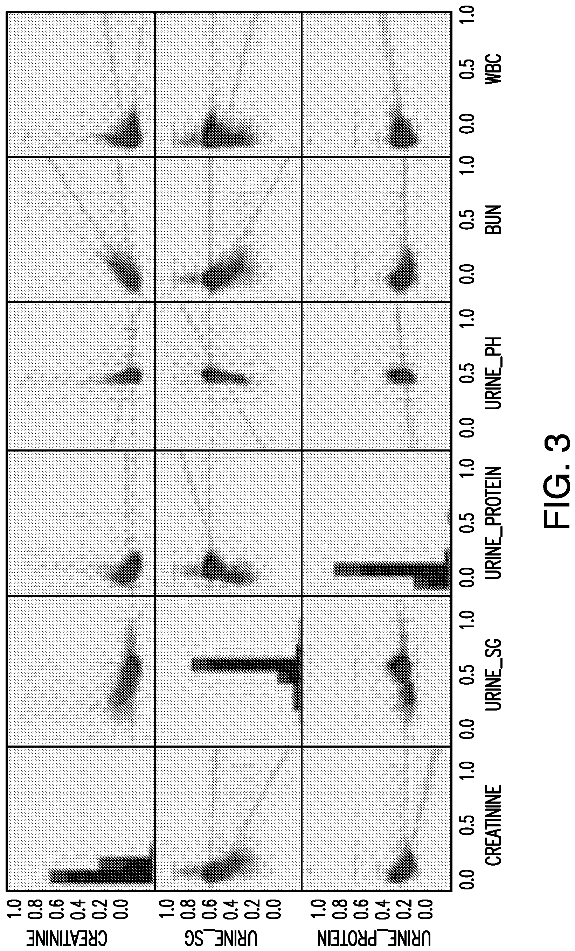

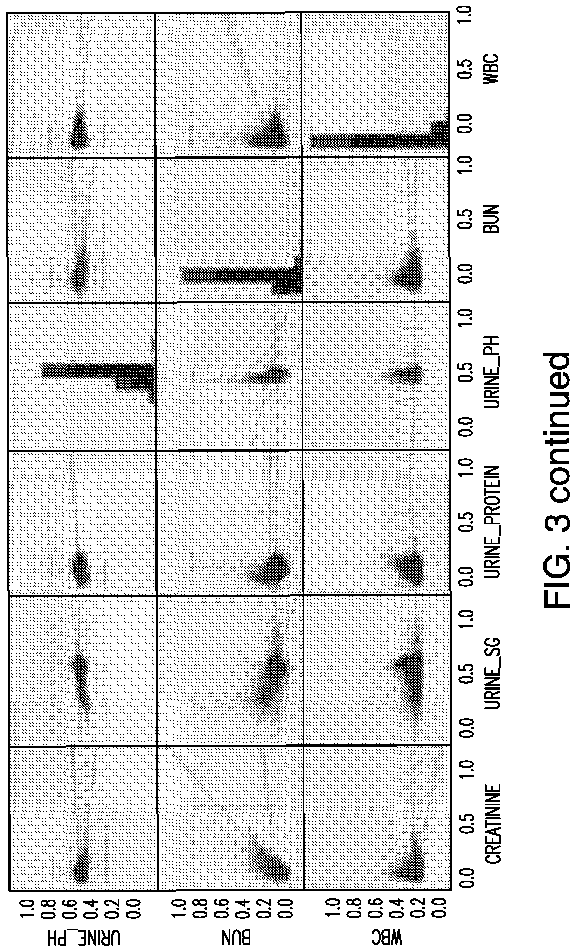

Figure 3 depicts the scatterplot matrix and histograms of the 6 most informative features. The large range of each variable can be attributed to outliers, the high overlap on variable values between healthy (green) and CKD (red) visits, which can obscure the prediction task.

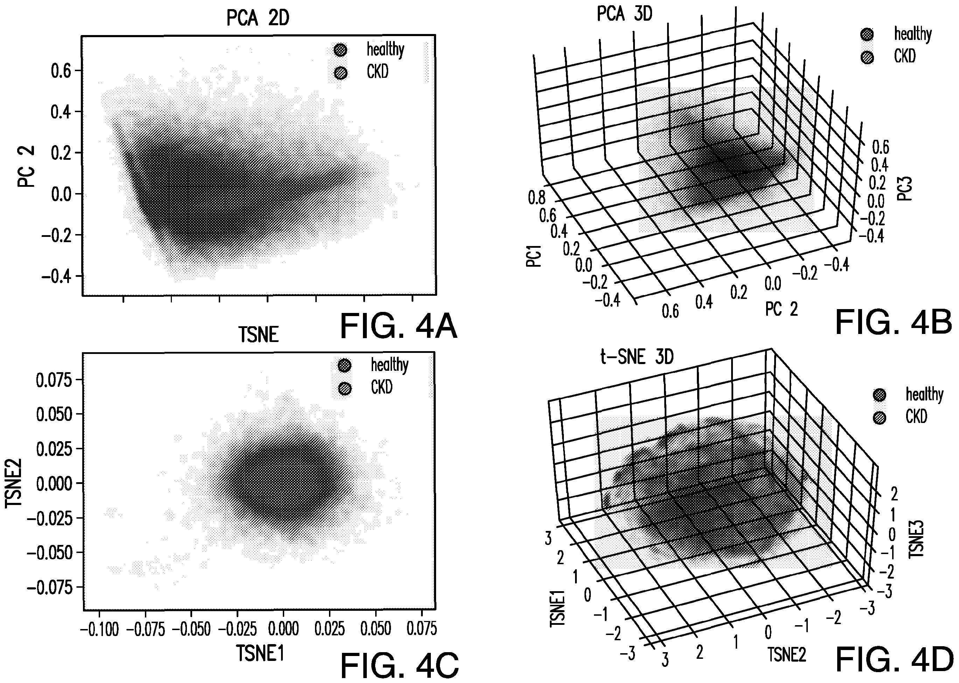

Figures 4A and 4B project the dataset into a feature space by performing PCA (linear) and t-SNE (non-linear) dimensionality reduction, respectively. Table 9 lists ranked features based on the PCA and t-SNE results and compares them to the ranking based on the feature selection methods (filter, wrapper).

*Supervised learning*

*1. Training and testing datasets*

The question to be answered by the predictor was "given a cat's record, will it have CKD within the next 2 years?" The dataset needed to be processed further to be ready for training and testing of the methods. It was done by first constructing a pan-cat dataset, that was the superset of all possible visit trajectories and then creating sampled datasets by sampling it with replacement.

For a cat with N visits, its trajectory was defined as the temporally ordered list of visits. A reduced trajectory was defined as any ordered subset of visits, where the last K visits were removed, where K was a number from 1 to N. In other words, if the cat's visit history can be thought as a string, with each element in the string corresponding to a visit, a reduced trajectory would be any prefix of the string and there can be up to N-1 possible prefixes (trajectories). If the original dataset were extended to include all possible reduced trajectories for CKD cats with removed visits up to 2 years before diagnosis, then an augmented dataset was created which was call the Pan-cat dataset.

Sampled dataset was defined as the subset of the Pan-cat dataset where a single trajectory for each CKD cat was randomly selected. Note that the records of healthy cats were identical to the initial dataset. A large number of sampled datasets were created by using a random number generator with different seeds, so that a different trajectory (a different number of visits) was chosen for each pet id (sampling with replacement).

The reason that the sampled dataset was needed to train and test the predictors was the following: For each cat that has CKD, the initial dataset contains data from the beginning of the pet's history up to a month after the diagnosis. If a predictor was trained using this dataset, the predictor would learn to identify whether an undiagnosed cat would have been diagnosed with CKD a month ago, which had little value. However, when using a sampled dataset, a predictor learns the patterns for cats that would be diagnosed with CKD at any point in the next 2 years.

*2. Feature selection*

Features were selected by using a filter method (Pearson Correlation Coefficient; PCC) and a top-down wrapper method KNN-DTW with K=7 neighbors, 25% of the training data, 3- fold cross-validation and F1-measure as the selection criterion (Figure 4). A bottom-up wrapper for the first 6 features was also in agreement with the results [3]. As shown in Table 9, the top features were Urine Specific Gravity, Creatinine, Urine Protein, Blood Urea Nitrogen (BUN), WBC and Urine pH. Interestingly, visit age was highly correlated to the output label, however the neither of the wrapper methods (top-down or bottom-up) picked it as a significant feature. A closer examination of the data shows that this feature had similar information (yet somewhat at a lower degree) to that in creatinine, so the inclusion of the later rendered the former less valuable.

**Table 9.**

| Feature | RANK | PCC | P-VALUE | PC1 |
|---|---|---|---|---|
| URINE_SG | 1 | -0.42 | 0 | 0.31 |
| CREATININE | 2 | 0.31 | 0 | 0.09 |
| URINE_PROTEIN | 3 | -0.08 | 5.9E-54 | 0.06 |
| BUN | 4 | 0.31 | 0 | 0.10 |
| WBC | 5 | 0.04 | 6.8E-17 | 0.00 |
| URINE_PH | 6 | -0.14 | 2.5E-169 | 0.05 |
| MCV | 7 | -0.04 | 2.1E-12 | 0.03 |
| AMYLASE | 8 | 0.13 | 1.3E-142 | 0.04 |
| BILIRUBIN | 9 | 0.00 | 4.4E-01 | 0.01 |
| LYMPHOCYTE | 10 | -0.17 | 3.1E-242 | 0.14 |
| VISIT_AGE | 11 | 0.54 | 0 | 0.88 |
| SEGS_NEUTROPHIL | 12 | 0.16 | 4.3E-222 | 0.27 |
| PHOSPHORUS | 13 | -0.06 | 1.9E-28 | 0.05 |
| MCH | 14 | 0.00 | 4.2E-01 | 0.01 |
| ALBUMIN | 15 | -0.08 | 5.5E-60 | 0.03 |
| GLOBULIN | 16 | 0.13 | 1.3E-155 | 0.03 |
| HEMATOCRIT | 17 | -0.12 | 1.7E-118 | 0.01 |
| PLATELET_COUNT | 18 | -0.03 | 4.3E-07 | 0.00 |
| EOSINOPHIL | 19 | 0.02 | 3.8E-06 | 0.00 |
| HEMOGLOBIN | 20 | -0.09 | 1.9E-64 | 0.01 |
| CALCIUM | 21 | 0.01 | 3.8E-03 | 0.01 |
| WEIGHT | 22 | -0.01 | 1.1E-01 | 0.02 |
| MCHC | 23 | 0.06 | 3.4E-33 | 0.00 |
| ALT_SGPT | 24 | 0.06 | 6.2E-34 | 0.02 |
| RBC_COUNT | 25 | -0.06 | 6.9E-36 | 0.02 |
| MONOCYTE | 26 | -0.01 | 5.2E-03 | 0.02 |
| CHLORIDE | 27 | 0.03 | 1.6E-10 | 0.00 |
| RDW | 28 | 0.13 | 6.7E-135 | 0.01 |
| SODIUM | 29 | -0.04 | 1.5E-12 | 0.00 |
| PROTEIN_TOTAL | 30 | 0.08 | 4.6E-55 | 0.01 |
| POTASSIUM | 31 | -0.01 | 1.5E-02 | 0.01 |
| MPV | 32 | 0.02 | 2.9E-03 | 0.00 |
| ALKALINE_PHOSPHATASE | 33 | -0.01 | 1.4E-01 | 0.02 |
| CHOLESTEROL | 34 | 0.16 | 1.6E-224 | 0.03 |
| GLUCOSE | 35 | 0.11 | 1.2E-106 | 0.04 |

Table 9 shows feature analysis and selection. The 35 features in the dataset were ranked based on the Wrapper top-down elimination (1, most informative; 35, least informative). It also shows the Pearson correlation coefficient of each feature with the CKD output, the p-value and the weight of the feature in PC1.

*3. Time-series prediction*

K-Nearest Neighbor (KNN) with Dynamic Time Warping (DTW): KNN-DTW was used with Euclidean distance as a metric [4] [5]. 5-fold cross validation was used to find the optimal K. To do so, the last {0,3,6,9,12,18, 24} months of the history of CKD cats (both train and test) were removed to create predictors that answer the following question:

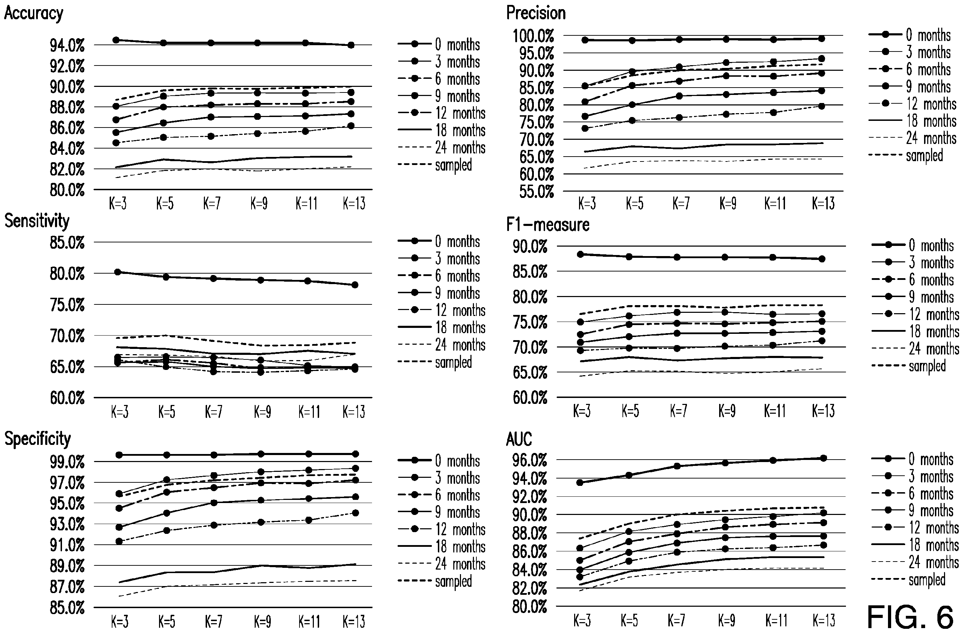

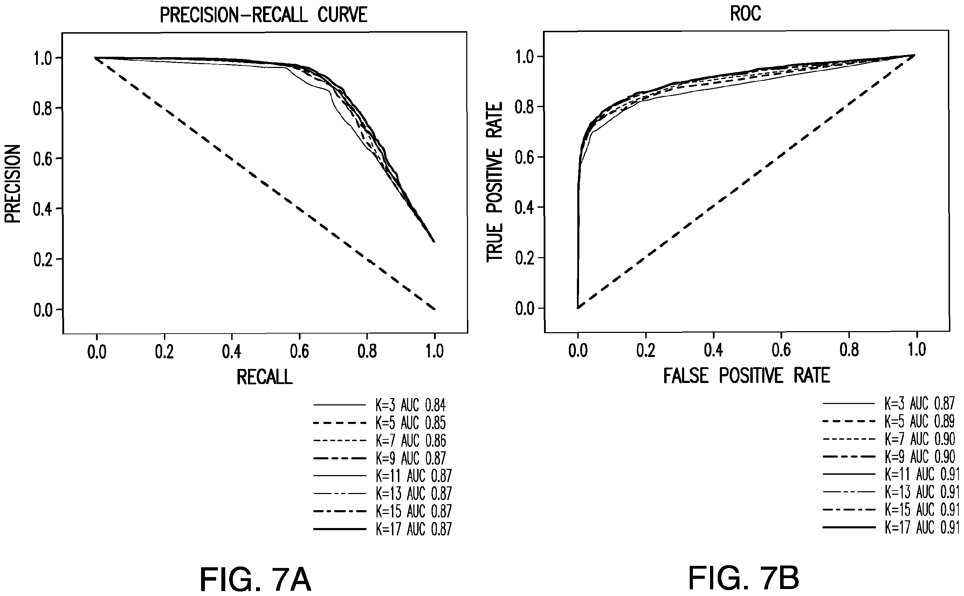

"Will my cat have CKD in X months from now?" A predictor was also trained and evaluated based on the "sampled dataset", which includes random trajectories for each cat by removing the last {0,3,6,9,12,18, 24} months and trains the predictor to answer the original question ("Will my cat have CKD within the next 2 years?"). As shown in Figure 6, after K=7 there was only a slight increase on the metrics, with performance increasing asymptotically up to K=13. In the case of sampled dataset, the runs were continued for K equal to 15 and 17 and a very slight difference (AUC ROC is 91.0% and 91.1%, respectively) was observed. As such, the final predictor was based on the sampled dataset with K=17 with its confusion table in Table 10 and ROC/PR shown in Figure 7.

**Table 10.**

Confusion Matrix for best KNN-DTW configuration with K=17

| KNN-DTW K=17 | | Known | | | | |
|---|---|---|---|---|---|---|
| | | **CKD** | **Healthy** | **Total** | | |
| **Pred** | **CKD** | 1452 | 227 | 1679 | 86.5% | **Precision** |
| | **Healthy** | 520 | 5213 | 5733 | 90.0% | **NPV** |
| | **Total** | 1972 | 5440 | | | |

| 73.6% | 95.8% | 13.5% | 89.9% | 79.5% |
|---|---|---|---|---|
| **Sensitivity** | **Specificity** | **FDR** | **Accuracy** | **F1** |

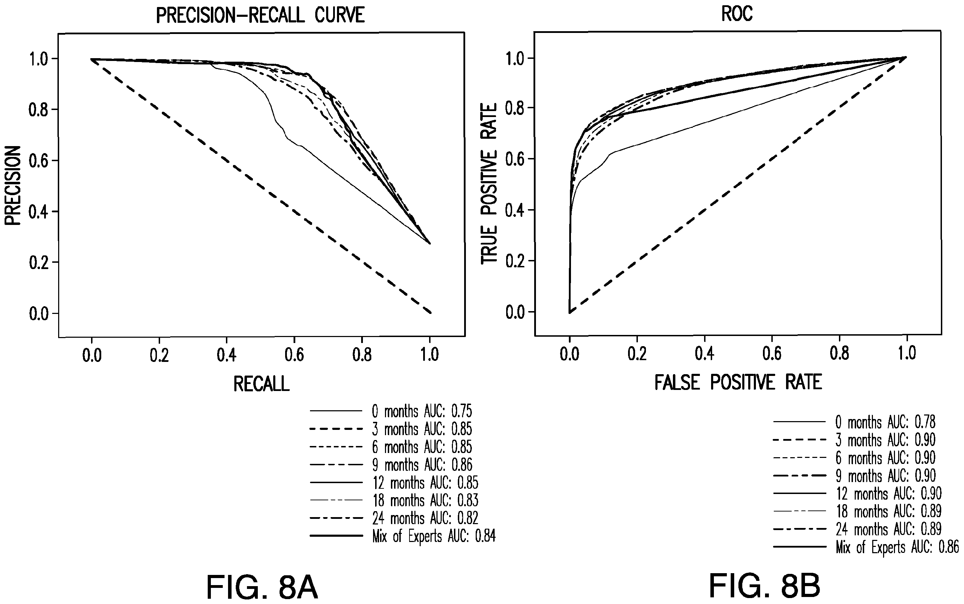

Mixture of Experts (MOE): Next, whether an Ensemble learning technique, where each individual KNN-DTW predictor trained to predict CKD for {0, 3, 6, 9, 12, 18, 24} was explored. An MOE meta-predictor was explored with either simple or weighted voting. The ROC/PR results for all predictors are shown in Figure 8. While the AUC was significantly lower than the individual predictors, the F1-measure was the highest.

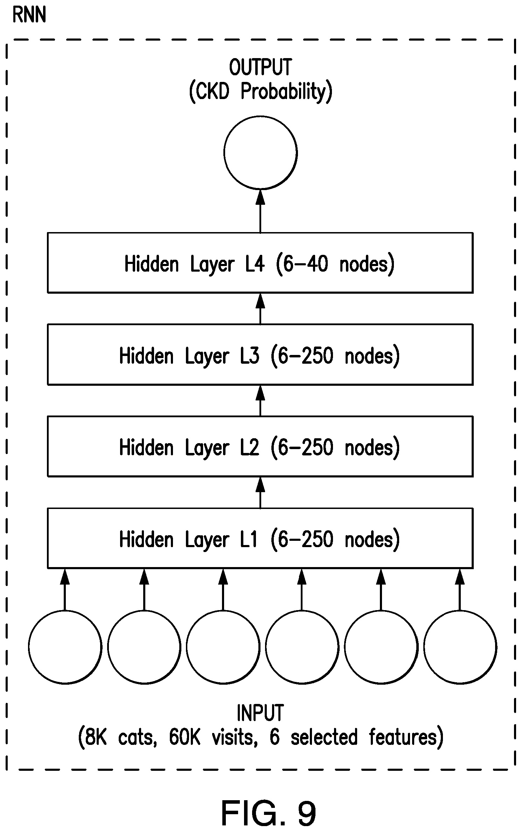

Recurrent Neural Networks with Long Short-Term Memory (RNN-LSTM): the architecture showing in Figure 9 was used for training recurrent neural networks (RNN). Different configurations of 1-4 hidden layers and 6-250 nodes per layer were ran. Tanh was used as activation function in the hidden layers and softmax (sigmoid here since binary classification) at the output layer. Binary cross-entropy was used for loss calculation and 20% dropout was considered to avoid overfitting [6]. Backpropagation through time was used for training with the RMSprop gradient descent optimization algorithm. In addition and on parallel with the vanilla RNN structure, the Long Short-Term Memory (LSTM) cell structure were explored to cope with vanishing gradients.

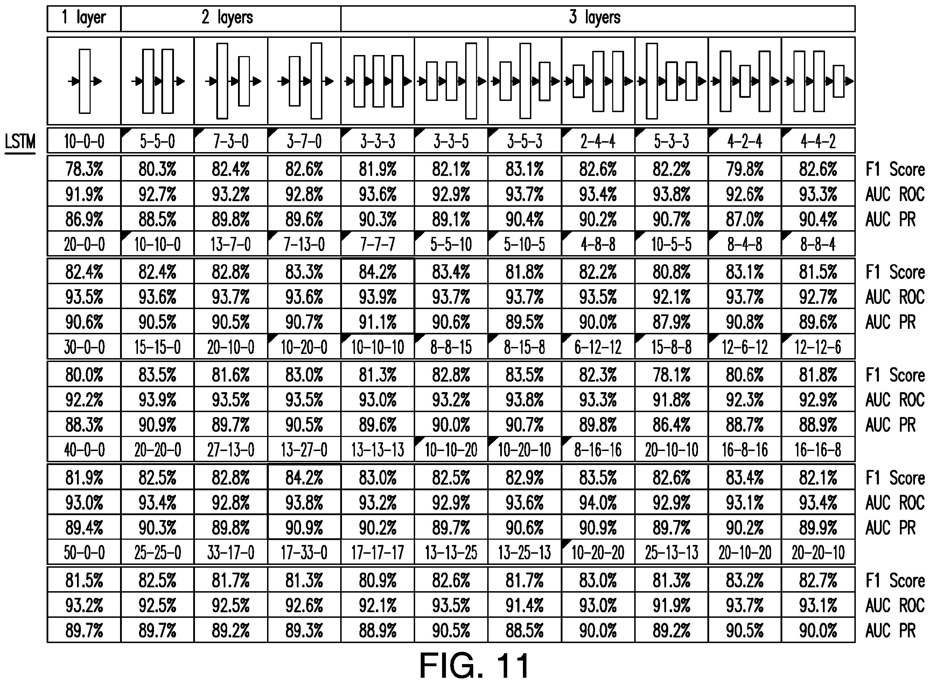

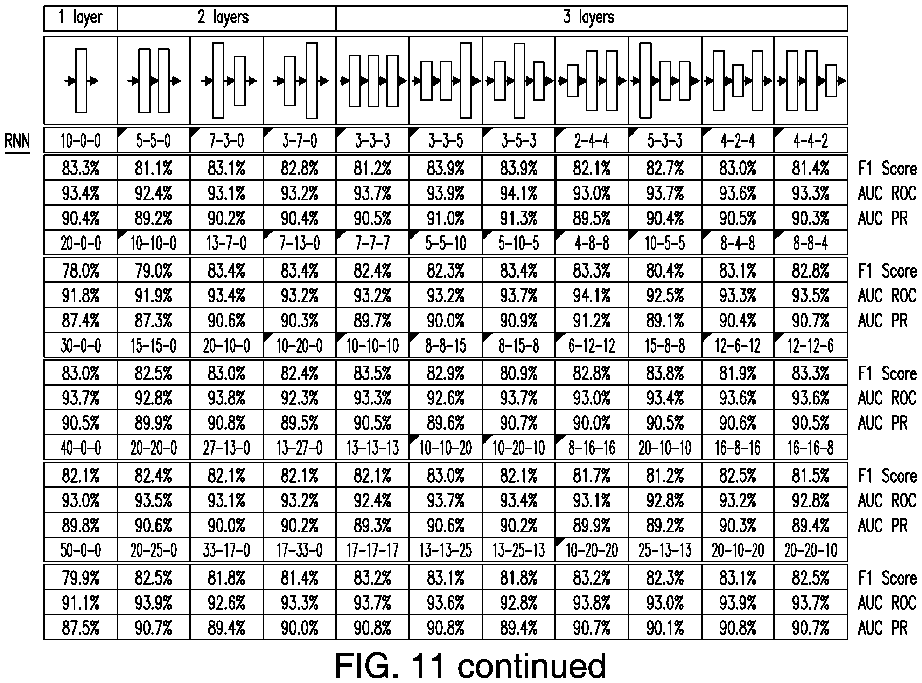

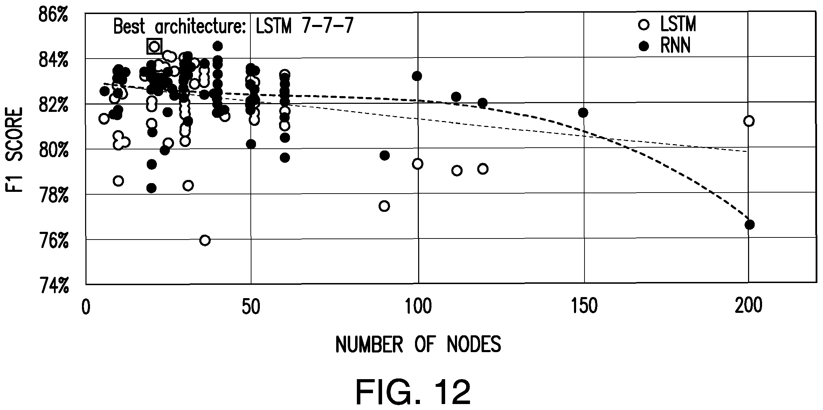

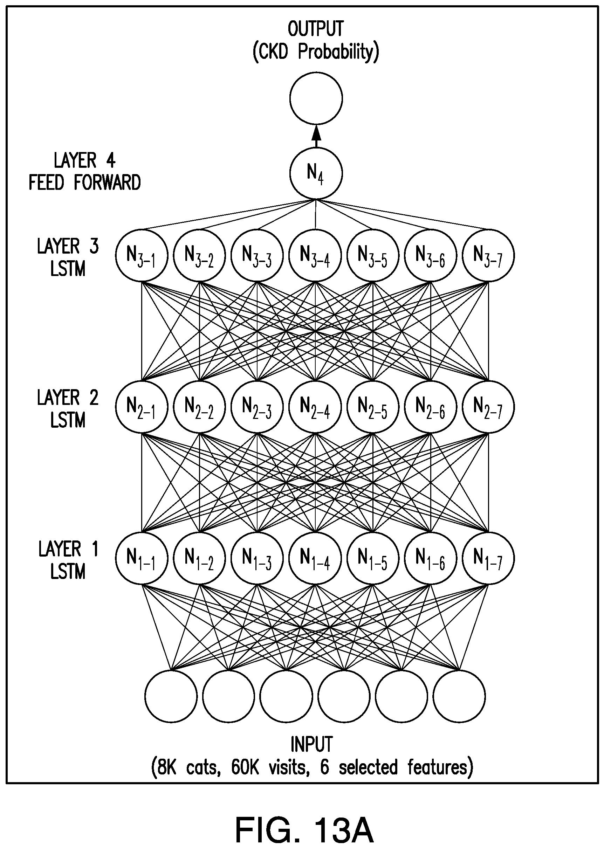

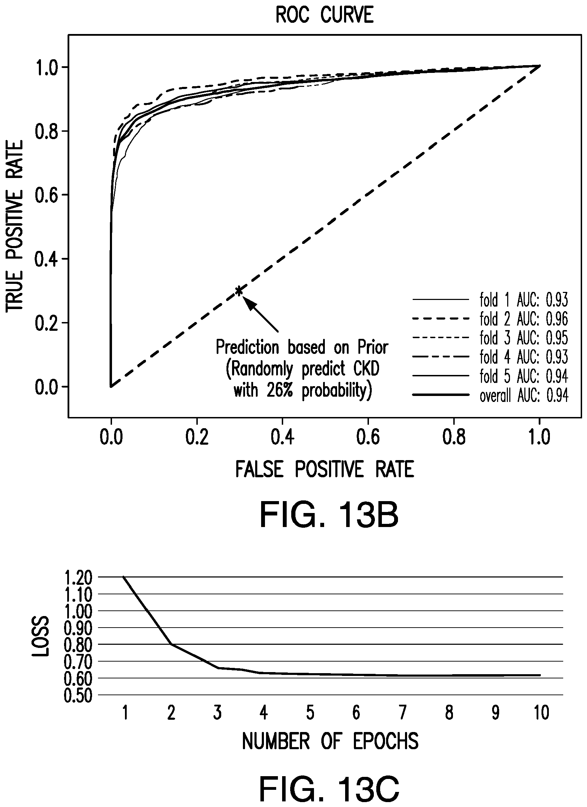

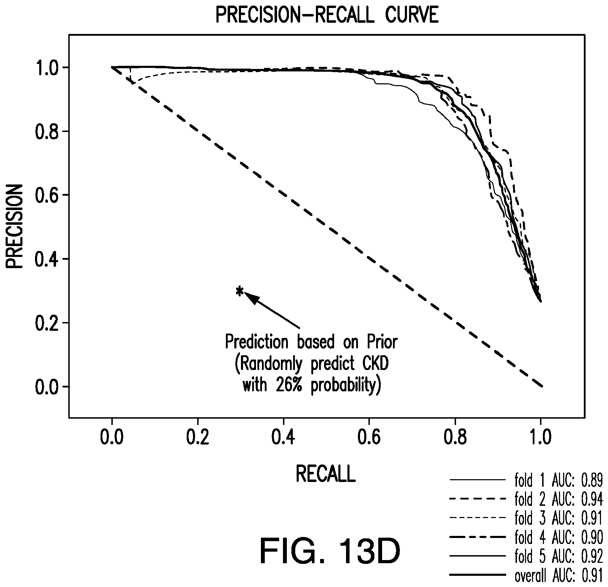

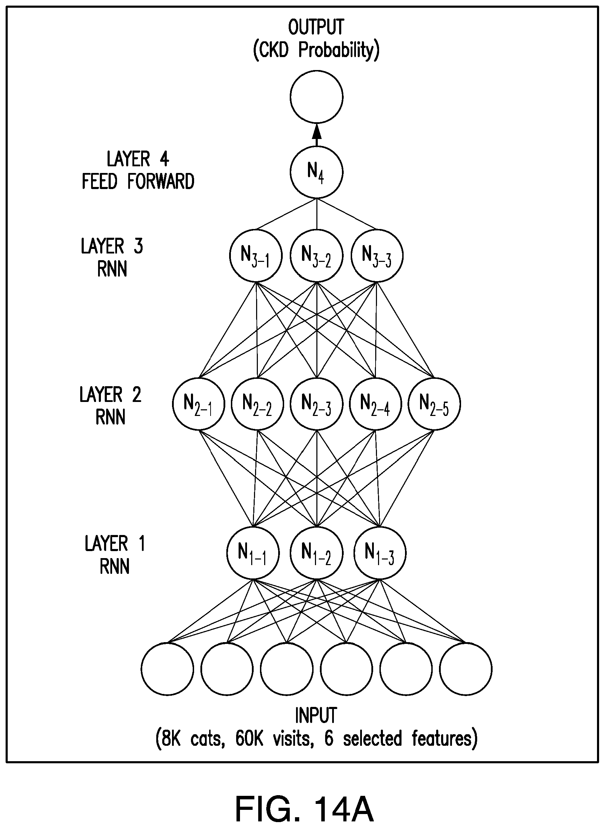

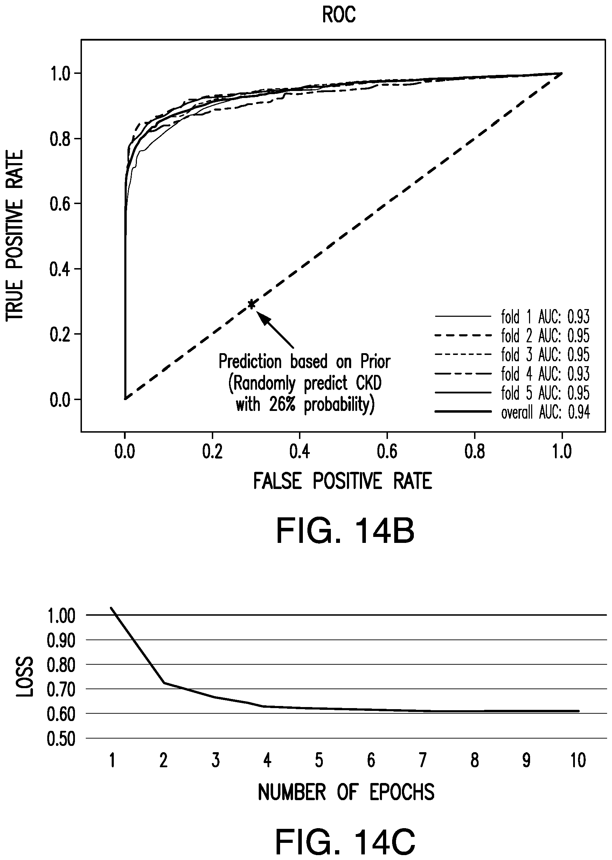

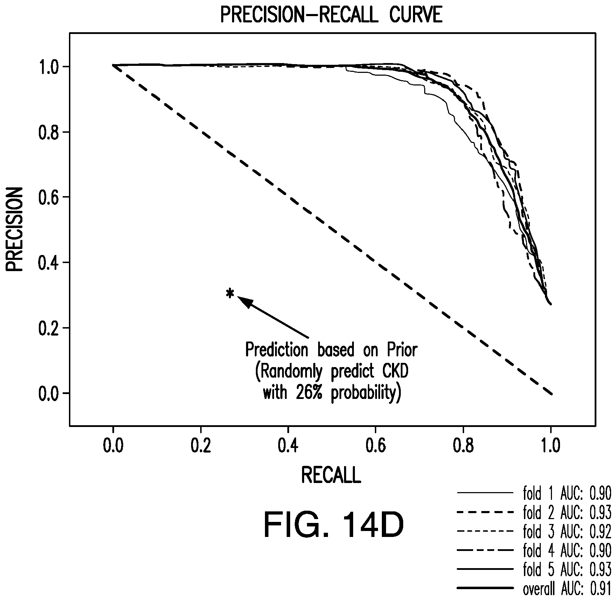

Figure 10 depicts the way the dataset was structured as an input to the RNN (Figure 10A) and the way the RNN was trained through time (Figure 10B). Different configurations were explored by performing a randomized parameter sweep on the number of nodes per layer and the number of layers (Figure 11). Figure 12 shows how the F1 measure changes as a function of the total number of nodes. The best two configurations after 5-fold cross validation were a 3-layer RNN- LSTM (Figure 13) and a 3-layer Vanilla RNN (Figure 14). The confusion tables for these two implementations are shown in Tables 11 and 12. Loss drops exponentially within the first 5 epochs and quickly saturates after that (Figure 13C, 14C). The robustness of the architectures was tested by calculating the various metrics over the different folds. After considering all parameters, the recommendation is to proceed with the 7-7-7 RNN-LSTM architecture.

## Table 11.

Confusion Matrix for best RNN-LSTM configuration

| LSTM | | Known | | | | |
|---|---|---|---|---|---|---|
| 7-7-7 | | CKD | Health | Total | | |
| Predict | CKD | 1560 | 173 | 1733 | 90.0% | Precision |
| | Healthy | 412 | 5267 | 5679 | 92.7% | NPV |
| | Total | 1972 | 5440 | | | |
| | | 79.1% | 96.8% | 10.0% | 92.1% | 84.2% |
| | | Sensitvity | Specificity | FDR | Accuracy | F1 |

## Table 12.

Confusion Matrix for best Vanilla RNN configuration

| RNN | | Known | | | | |
|---|---|---|---|---|---|---|
| 3-5-3 | | CKD | Health | Total | | |
| Predict | CKD | 1582 | 217 | 1799 | 87.9% | Precision |
| | Healthy | 390 | 5223 | 5613 | 93.1% | NPV |

| | Total | 1972 | 5440 | | | |
|---|---|---|---|---|---|---|
| | | 80.2% | 96.0% | 12.1% | 91.8% | 83.9% |
| | | Sensitvity | Specificity | FDR | Accuracy | F1 |

*Summary of Model Building*

Two methods for longitudinal analysis: K- Nearest Neighbors with Dynamic Time Warping (KNN-DTW) and Recurrent Neural Networks (RNN) either vanilla or with Long Short-Term Memory cells (RNN-LSTM) were used. The dataset had 61,159 feline visit records for 8,806 unique cats from the veterinary database (6,711 healthy control and 2,095 cats that have/develop CKD). There were 35 features from demographics, blood chemistry, hematology and urine levels.

From the hundreds of predictors built, the two best were (a) KNN-DTW with K=17 neighbors (AUC ROC=0.91; AUC PR= 0.87; F1=0.795) and (b) RNN-LSTM with 3 LSTM layers (7-7-7) and 1 dense layer (AUC ROC=0.94; AUC PR= 0.91; F1=0.842). The Mixture of Experts configuration achieved a slightly lower performance but better stability. There was a clear separation of the data in 3D space following (non)linear dimensionality reduction. The top 6 features were sufficient for classification. Weight was not a good predictor, nor change in weight (absolute or relative). Interestingly, while visit age had a high correlation with the CKD onset, it was not used in the classification.

Only 6 features were needed for gain all the information that the data can provide for prediction: Urine SG, Creatinine, Urine Protein, BUN, WBC, Urine pH, ordered based on their information content. A final KNN-IDT and RNN-LSTM predictors were provided. The pre-trained RNN predictor calculated faster and performs better than the KNN predictor. The predictors achieved 0.94 AUCROC, 0.91 AUCPR and 0.842 F1, with accuracy, precision, recall, specificity all at high numbers. This performance was measured in a realistic scenario when cats have CKD at a random, stratified point within the next two years. When tested with cats that had CKD in a fixed time range, performance ranges with an accuracy of ~0.95 to ~0.83 for cats with CKD within 0-3 month to 21-24 months from now, respectively. Further optimization was not expected to move the performance to more than 5% within this project cycle. Higher quantity/quality of data would boost performance in the future.

## *References*

[1] Stekhoven, Daniel J. "MissForest - nonparametric missing value imputation for mixed- type data." Oxford Journal's Bioinformatics 28.1 (2012) 2012, 112-118
[2] http://scikit-learn.org/stable/modules/generated/sklearn.preprocessing.MinMaxScaler
[3] Granitto, Pablo M., et al. "Recursive feature elimination with random forest for PTR- MS analysis of agroindustrial products." Chemometrics and Intelligent Laboratory Systems 83.2 (2006): 83-90.
[4] Giorgino, Toni. "Computing and visualizing dynamic time warping alignments in R: the dtw package." Journal of statistical Software 31.7 (2009): 1-24.
[5] Tan, Songbo. "Neighbor-weighted k-nearest neighbor for unbalanced text corpus." Expert Systems with Applications 28.4 (2005): 667-671.
[6] Srivastava, Nitish, et al. "Dropout: a simple way to prevent neural networks from overfitting." Journal of Machine Learning Research 15.1 (2014): 1929-1958.

## **Example 2**

This example was to develop a predictive modeling system of azotemia (AZO) in cats based on urinary measures, and to implement the system in software for veterinary use. The predictive modeling system comprises of 5 independent mathematical models, allowing predicting the probability of azotemia 0, 90, 180, 270 and 360 days after the measurement, respectively. These models are based on a logistic equation that predicts the probability of a feline becoming azotemia in a given period from three blood parameters: creatinine, urine specific gravity and urea. Each of these models is associated with a decision threshold corresponding to the probability beyond which the individual will be predicted to be positive. This limit was determined by the ROC curve of each model and the Youden method. The predictive modeling system integrates a Bayesian evaluation system taking into account the history of the measurements of each cat and making it possible to refine the predictions by increasing the number of measurements.

In this example, data independent from those used to construct the initial models were used to: 1) validate the initial models; 2) improve the initial models; and 3) test a supervised neural network (ANN) approach as an alternative to the logistic equation approach.

## *Methods*

### *1. Data*

The new data comes from the veterinary database. The raw file has 58,292 lines corresponding to 8422 unique individuals followed at regular intervals. Three variables are measured: creatinine, urine specific gravity and urea. However, not all individuals consistently displayed a value for each of the three variables. Since models were based on the use of all three variables simultaneously, individuals with missing values were removal from the study. After this process, there remained 18,976 lines for 7051 individuals.

The following Table 13 shows visiting age and age of diagnosis values, before and after removal of incomplete individuals.

**Table 13.**

|  | *Before removal of incomplete individuals* |  | *After removal of incomplete individuals* |  |
|---|---|---|---|---|
|  | **Age visit** | **AZO diagnosis age** | **Age visit** | **AZO diagnosis age** |
| **min** | 0059 | 1.97 | 0.20 | 1.97 |
| **max** | 22.23 | 21.41 | 21.41 | 21.41 |
| **average** | 6.78 | 13.25 | 7.47 | 13.41 |
| **median** | 6.04 | 13.99 | 6.76 | 14.1 |

The removal of incomplete individuals had little influence on the age distribution characteristics, especially regarding the age of diagnosis of the disease. In total, out of 7051 selected individuals (18,976 measurements), 5348 were never diagnosed AZO and 1703 were. Out of the 18,976 measures, 1,302 were negative and 5933 were associated with individuals diagnosed positive during their follow-up.

The dataset was separated into two parts by random drawing to create the following:

1. A validation data set consisting of 9,469 measures, out of which 6,521 were negative and 2,948 were associated with individuals diagnosed positive during their follow-up. This dataset was then used both to validate initial models and to validate updated models and ANNs.

2. A set of learning data, consisting of 9506 measures, out of which 6521 were negative and 2985 were associated with individuals diagnosed positive during their follow-up. This dataset was then used to update the initial models in a new learning phase, but also to adjust the ANNs. For this purpose, the data from the initial study were added to this learning game, with 459 negative measures (170 unique individuals) and 244 measures associated with individuals diagnosed positive during their follow-up (56 unique individuals).

Certain individuals had their measurements shared between the validation and learning phases.

Afterwards, the learning dataset is divided into several subsets built to match the 5 models:

1. The measurements corresponding to individuals already diagnosed AZO during a given visit;
2. The measurements corresponding to individuals diagnosed with AZO within 3 months after a given visit;
3. The measurements corresponding to individuals diagnosed with AZO within 6 months after a given visit;
4. The measurements corresponding to individuals diagnosed with AZO within 9 months after a given visit; and
5. The measurements corresponding to individuals diagnosed with AZO within 12 months after a given visit.

To each of these subsets, the measurements corresponding to all individuals never diagnosed with AZO (providing the negatives for the models) are added.

## 2. Validation of initial models

In a first phase, all the new measures were projected in the initial models. For each measurement (visit), a prediction was made by each model (10, t3, t6, t9, t12), a search on the data of the corresponding individual was carried out to know if it was diagnosed AZO on the prediction period of the model (0 months, 3 months, 6 months, 9 months, 12 months). This made it possible to measure the quality of prediction.

For example on January 1, a measurement was made, and it was negative (no CKD on January 1). Model T0 predicts a negative and Model T3 predicts however CKD. For Model T3, there is an error if: the cat never becomes sick, or the cat becomes sick but after March 1; and there is no mistake if: the cat becomes sick before the March 1 even if the measure of January 1 said that it was negative.

Then, the sensitivity and specificity of the model under validation were calculated based on the number of true and false positives and negatives.

## 3. Update of the initial models

In a second phase, the learning dataset was used to re-adjust the initial logistic models (see original study report). Once the models were adjusted, the decision threshold to classify an individual as predicted or non-ill patient was calculated using the Youden index. The validation dataset was then projected into these updated models to verify matching of the predictions by calculating the sensitivity and specificity of the models in validation.

## 4. Neural network approach

The general approach was the same as before: using the learning dataset to adjust the models then projection of the validation data and calculating the sensitivity and specificity of validation. The adjustment phase of the neural networks was based on the coupling of a factorial plan on the parameters of the networks with an approach by 10-folds cross-validation.

The procedure was the following :

1. Pattern adjustment was repeated 5 times using:

- The learning data subset;
- All input variables;
- A complete factorial design to set the best set of parameters (intrinsic to the neural networks) for each model (model tuning);

- A 10-folds partition of the dataset, generated randomly at each draw: the technique of k- folds cross-validation consists of carrying out training based on 9 of the 10 scores, validating the 10th partition, and then redoing this process by exchanging validation score with a learning partition, and so on until all partitions were used for learning and validation. Thus, it was ensured that the model was not trained by a particular configuration of learning/validation data. Therefore, 10 weight adjustments were obtained which would be assembled to form an overall model with the best parameter set of the neural network.

2. Calculation of the Youden index for validation of the models established in such a manner, upon each repetition.
3. Selection of the best model for each repetition.
4. The final model was an overall model of the 5 best models which were composed themselves, of 10 networks of assembled neurons. As a result, in total, the final prediction model comprises 50 networks that were assembled to give a final prediction. The Youden index was calculated based on the result of this assembly to form the decision threshold during projection of the validation dataset (subset not used in this adjustment phase).

### Results

Results of the projection of all new data in the initial models are shown in Table 14.

**Table 14.**

| | True Positive | True Negative | False Positive | False Negative | Sensitivity | Specificity |
|---|---|---|---|---|---|---|
| $T_0$ (0 days) | 992 | 13508 | 4404 | 71 | 93 % | 75 % |
| $T_3$ (90 days) | 1149 | 10365 | 7398 | 63 | 95 % | 58 % |
| $T_6$ (180 days) | 1302 | 12168 | 5366 | 139 | 90 % | 69 % |
| $T_9$ (270 days) | 1554 | 12233 | 4978 | 210 | 88 % | 71 % |
| $T_{12}$ (360 days) | 1540 | 12611 | 4600 | 224 | 87 % | 73 % |
| *Restatement of sensitivity/specificity couples of the initial models during the training phase: T0 = 94191, T3 = 86/97.6, T6 = 83/86, T 9 = 77/83, and T12 = 84/76.* | | | | | | |

Considering the fact that initial training of the models had been carried out based on no more than 703 measurements covering 226 individuals, the results of the projection of 18,976 new measurements (7051 different individuals) which were not used to build the model may be considered to be very good. The specificity was higher or equivalent to 90% up to 180 days (T6) and 88 and 87% at T9 and T12, respectively. Specificity was lower, although it remained above 70%, at T0, T9, and T12. It was 69% at T6. Only the validation specificity of model T3 was much lower (58%). To appraise the quality of the results, it must also be kept in mind that it was not so much the value of sensitivity or specificity alone that is important, but rather the sensitivity-specificity couple, since the two parameters were interdependent: once one was decreased, the other one was increased.

Results of the projection of the new validation data in the updated initial models with the new training data are shown in Table 15. All models were improved with this subsequent addition of new data. All "Sensitivity + Specificity" sums were improved and, in particular, all specificity values increase.

**Table 15.**

| | True Positive | True Negative | False Positive | False Negative | Sensitivity | Specificity |
|---|---|---|---|---|---|---|
| $T_0$ (0 days) | **507** | 7105 | 1826 | 31 | 94% | 80% |
| $T_3$ (90 days) | 589 | 6193 | 2655 | 32 | 95% | 70% |
| $T_6$ (180 days) | 664 | 6636 | 2109 | 60 | 92% | 76% |
| $T_9$ (270 days) | 784 | 6882 | 1697 | 106 | 88% | 80% |
| $T_{12}$ (360 days) | 839 | 7155 | 1284 | 191 | 81% | 85% |

Results of projection of new validation data in neural networks models updated with new learning data are shown in Table 16.

**Table 16.**

| | True Positive | True Negative | False Positive | False Negative | Sensitivity | Specificity |
|---|---|---|---|---|---|---|
| $T_0$ (0 days) | 482 | 8095 | 836 | 56 | 90% | 91% |
| $T_3$ (90 days) | 575 | 7379 | 1469 | 46 | 93% | 83% |
| $T_6$ (180 days) | 643 | 7499 | 1246 | 81 | 89% | 86% |
| $T_9$ (270 days) | 729 | 7696 | 883 | 161 | 82% | 90% |
| $T_{12}$ (360 days) | 779 | 7724 | 715 | 251 | 76% | 92% |

ANN results were also very satisfactory since all the models present a "Sensitivity + Specificity" sum, which were superior one by one to those models by logistic regression. It can be noted that it was the specificity that was significantly improved on all the models.

***Discussion***

Updating the data made it possible to significantly improve the quality of the models. This improvement can be considered, at the same time, to be a quantitative improvement, with the improvement of the sensitivity/specificity couples through the addition of new data, and a qualitative improvement, considering that the importance of the number of new data that were used for training should consolidate and stabilize the models.

It is recommended to explore the methodological improvement of the models based on logistic regression (randomization of the training/validation data sets) and the construction of a comprehensive model combining the neuron network approach and that based on logistic regression in order to combine the strengths of the two approaches: the neuron networks provide better specificity, and the logistic models have better sensitivity.

## Example 3

This example relates to method of diagnosing CKD using baseline serum creatinine level for cats with creatinine levels within the laboratory reference interval.

If a cat has prior visits with bloodwork (+/- urinalysis), the baseline of serum creatinine for the cat can be established. The following criteria must be met to establish the baseline:

a. At the time of current visit, the cat has at least 2 creatinine results that were obtained in the previous 2 years. If available, it is recommended to use all creatinine results available during that time period that meet criteria (b)-(d);
b. Cat is over 1 year of age during each of those visits with creatinine results;
c. Cat is spayed/neutered at least 2 months before the first creatinine result to be used in the baseline; and
d. Cat must be otherwise healthy and not have any concurrent illness (e.g., hyperthyroid, diabetes).

Pre-test fasting is not necessary for evaluation of serum creatinine.

Using the previous creatinine results that meet the above criteria, the baseline creatinine level can be established by calculating the mean creatinine value.

Accordingly, diagnosis of CKD can be made using Table 17.

**Table 17.**

| State | Defined by: |
|---|---|
| At-risk | If any of the criteria regarding creatinine below are met, but there is no urine specific gravity and/or SDMA results available within the past 30 days. |
| Early CKD (IRIS CKD, Stage 1) | Creatinine < 1.6 mg/dL, but see $\geq$20% increase in serum creatinine from baseline; USG < 1.035; and SDMA > 14 $\mu$g/dL |

(continued)

| State | Defined by: |
|---|---|
| CKD, Stage 2 | Creatinine = 1.6-2.8 mg/dL;<br>USG< 1.035; and<br>SDMA = 15-25 $\mu$g/dL |
| CKD, Stage 3 | Creatinine = 2.8 - 5.0 mg/dL;<br>USG< 1.035; and<br>SDMA >25 $\mu$g/dL |
| CKD, Stage 4 | Creatinine >5.0 mg/dL;<br>USG< 1.035; and<br>SDMA $\geq$ 45 $\mu$g/dL |
| Healthy or Subclinical renal disease not detected | Not meeting any of the above criteria |

**Example 4**

This example relates to simplified rules to establish a typology of cats suffering from/not suffering from azotemia (AZO) in addition to the predictive modeling system constructed by machine learning in Example 2.

The instant predictive modeling system consists of six models to predict azotemia in cats. Each model is associated with the period of time which has elapsed since an initial point in time during which the disease can be triggered: 0 month (t0), 3 months (t3), 6 months (t6), 9 months (t9), 12 months (t12), and 24 months (t24).

The data which served to calibrate and validate the AZO-Predict models through neuron networks in Example 2 were used for developing the rule. The performances of the various proposed rules were tested by calculating their AUC, their sensitivity, and their specificity. Contrary to Example 2, no cross-validation were carried out, i.e., all data were used to establish the rule and calculate the performances of the models.

Table 18 shows the performances of optimized AZO-Predict models constructed by machine learning process.

**Table 18.** Performances of optimized ANN models selected for all repetitions. SE: sensitivity, SP: specificity.

| | SE | SP |
|---|---|---|
| t0 | 0.93 | 0.90 |
| t3 | 0.91 | 0.84 |
| t6 | 0.78 | 0.89 |
| t9 | 0.82 | 0.84 |
| t12 | 0.82 | 0.81 |
| t24 | 0.73 | 0.83 |

*Simplified Predictive Rule*

The simplified predictive rule is based on the application of Linear Discriminant Analysis (LDA), which provides a linear model to calculate a score designated SC1, whose value permits predicting the disease.

Three variables measured during a visit, i.e. creatinine concentration (Creat; measured in gm/dL), urine specific gravity (UrineSG), and urea (Urea; measured in gm/dL) were used in the simplified predictive rule, as well as the coefficients thereof, i.e., a(Creat), b(UrineSG), and c(Urea). These coefficients were the result of the application of the LDA on the data for each and every time of prediction (t0, t3, t6, t9, 112, t24). The values of the coefficients are shown in Table 19 below.

Threshold coefficients were used to determine, based on the SC1 value, whether the cat would be ill or not. The Threshold values were the result of the application of the LDA, and the values for each and every time of prediction are shown in Table 3.

The simplified predictive rule is summarized below:

1- SC1 = a(Creat) x Creat + b(UrineSG) x UrinesSG + c(Urea) x Urea

2- If SC1 > Threshold, the cat is predicted to be ill, if SC1 ≤ Threshold, the cat is predicted not to be ill.

**Table 19.** Summary of values used and performances of the simplified predictive rule for each and every time of prediction

|  | a(Creat) | b(UrineSG) | c(Urea) | Threshold | SE | SP |
|---|---|---|---|---|---|---|
| **t0** | 0.0068 | -40.0563 | 0.0659 | -38.7128 | 0.89 | 0.89 |
| **t3** | 0.0083 | -25.7343 | 0.1182 | -22.6030 | 0.80 | 0.87 |
| **t6** | 0.0069 | -36.9897 | 0.1137 | -34.8051 | 0.77 | 0.84 |
| **t9** | 0.0061 | -44.3368 | 0.1077 | -42.7709 | 0.77 | 0.83 |
| **t12** | 0.0057 | -47.0420 | 0.1085 | -45.6250 | 0.74 | 0.85 |
| **t24** | 0.0058 | -49.9186 | 0.1044 | -48.7966 | 0.70 | 0.84 |

The performances were comparable to the performances of optimized AZO-Predict models constructed by machine learning process shown in Table 18.

**Example 5**

The prediction model developed according to Example 1 based on six biomarkers was further improved. The selection criteria were refined for the tens of thousands of predictions made on the cats at different time points (i.e. with different amounts of data removed).

Table 20 shows the results for the improved model run in Longitudinal mode (across multiple visits) on blinded data from the veterinary database, where the cats were split into Cases and Controls based on their diagnosis and had blood and urine data which is consistent with either IRIS Stage 0 or Stage 3+. This removed a lot of the ambiguous cats, and the model predicted well on the remainder. The false positive rate for this subset of cats was less than 1%. Prediction up to 3 years had high accuracy.

**Table 20.**

| Years from Diagnosis | Sensitivity | Specificity | Accuracy | Total Cats |
|---|---|---|---|---|
| 0 | 99.1 | 99.4 | 99.4 | 7,010 |
| 1 | 82.9 | 99.1 | 95.9 | 2,915 |
| 2 | 68.7 | 99.4 | 93.3 | 1,774 |
| 3 | 57.4 | 99.8 | 91.5 | 674 |

Table 21 shows the results of the same analysis, but with the model only seeing single visits. As there were more single visits the predictions out to 4 years are shown. The model performed well on single visit data, with the accuracy comparable to the multiple visits. One reason was that the single-visit data were limited to having a creatinine and USG measure, whereas the Longitudinal model was predicting on significant amount of missing data. Therefore, the Longitudinal model would improve with more complete data (more Wellness visits per pet). For reference, a Sensitivity of around 20% was expected at random, so 47% at 4 years was significantly better than random, and the specificity was high (false positives around 1%).

**Table 21.**

| Years from Diagnosis | Sensitivity | Specificity | Accuracy | Total Cats |
|---|---|---|---|---|
| 0 | 99.0 | 98.9 | 98.9 | 5,517 |
| 1 | 83.4 | 99.2 | 96.0 | 2,292 |
| 2 | 70.2 | 99.0 | 93.2 | 1,723 |
| 3 | 56.7 | 98.4 | 90.1 | 1,180 |

(continued)

| Years from Diagnosis | Sensitivity | Specificity | Accuracy | Total Cats |
|---|---|---|---|---|
| 4 | 47.3 | 99.2 | 88.8 | 607 |

The performance of the same predictions for the whole dataset are shown below (i.e. cats with reasonable quality data, but only based on veterinary diagnosis captured in the database, not cleaned by blood chemistry sense-checking). Certain individual cats had very high blood chemistry and sometimes low urine pH. Veterinary physicians commented in certain medical notes regarding possible kidney disease but had not necessarily made a formal diagnosis. Therefore, the model sometimes predicts CKD in these additional cats when there was no diagnosis of CKD. This led to a slight increase in false positives and lower apparent accuracy across the whole uncleaned dataset. It was reckoned that certain borderline cases were where the model can help the clinicians to make an earlier decision. There could also be comorbidities in certain cases like hyperthyroidism which can make diagnosis difficult.

Performance of longitudinal models with all data including Cases with lower creatinine, and Controls with high creatinine is shown in Table 22. The Controls in this dataset contained a large percentage with Creatinine > 1.6mg/dl (140 $\mu$mol/l). Generally the Sensitivity remained high, but the Specificity and Accuracy dropped when the more ambiguous data were introduced, due to the false positive rate increasing. However, the results remained robust.

**Table 22.**

| Years from Diagnosis | Sensitivity | Specificity | Accuracy | Total Cats |
|---|---|---|---|---|
| 0 | 96.9 | 90.1 | 91.5 | 29,201 |
| **1** | **79.3** | **88.8** | **86.9** | 21,916 |
| **2** | **63.3** | **87.4** | **82.6** | 13,588 |
| **3** | **52.5** | **85.8** | **79.1** | 4,820 |
| **3.5** | **54.4** | **85.5** | **79.3** | 797 |

The performance of cross sectional (single visit) models with all data including Cases with lower creatinine, and Controls with high creatinine is shown in Table 23. The Controls in this dataset contained a large percentage with Creatinine > 1.6mg/dl (140 $\mu$mol/l)

**Table 23**

| Years from Diagnosis | Sensitivity | Specificity | Accuracy | Total Cats |
|---|---|---|---|---|
| 0 | 96.8 | 85.8 | 88.0 | 20,757 |
| 1 | 79.8 | 87.2 | 85.7 | 17,559 |
| 2 | 64.2 | 87.2 | 82.6 | 12,682 |
| 3 | 53.1 | 88.8 | 81.6 | 8,268 |
| 4 | 43.5 | 85.8 | 77.3 | 3,995 |

## Example 6

Data from a second veterinary database (63,500 cats, 177,500 visits) were used to further test and improve the prediction model. The data were processed to produce clean 'Cases' and 'Controls based on either the last visit being IRIS Stage 3 and previous visits being below IRIS Stage 3, or remaining at IRIS Stage 0 for all visits. Cats were defined as having a lifetime (across the 2 or more years of results for them in the second veterinary database) kidney IRIS Stage of either:

- Stage 3 if creatinine > 2.8 and urine_SG <= 1.035 within a 3-day period
- Stage 0 if creatinine_max < 1.6 and urine_SG_min >= 1.035 across all recorded visits

All other combinations of levels were staged but not included in this prediction set. It was known that IRIS staging without other clinical signs was not perfect in terms of diagnosis of CKD, although there was a high level of correlation. However, no other clinical information was available for most of the cats in the second veterinary database. Up to the visit prior to reaching Stage 3 (when the cat was staged below 3) were given to the model, which predicted the likelihood of the next visit being classed as stage 3. This would give clinicians the opportunity to intervene if the risk was seen to be high.

Examples of the performance are shown below. First, the model performed well across all major cat breeds as shown in Table 24.

**Table 24.**

| Breed | Sensitivity | Specificity | Accuracy | Total Cats |
|---|---|---|---|---|
| Abyssinian | 94.74 | 97.47 | 96.94 | 98 |
| Himalayan | 99.97 | 96.15 | 96.55 | 29 |
| Maine Coon | 99.9 | 97.87 | 97.91 | 48 |
| Mixed Breed | 93.71 | 98.76 | 98.15 | 2483 |
| Other Purebred | 94.07 | 97.61 | 97.04 | 845 |
| Persian | 99.98 | 100 | 100 | 55 |
| Ragdoll | 80 | 95.83 | 93.1 | 29 |
| Siamese | 92 | 98.68 | 97.03 | 101 |

Where there were more than one historic creatinine and USG value, the model was predicting the future state (at an average of 6 months) with above 98% accuracy.
The accuracy dropped slightly with only one historic creatinine value, but was still above 96%.

**Table 25.** For cats with > 1 creatinine value and > 1 USG value

| Sensitivity | Specificity | Accuracy | Total Cats |
|---|---|---|---|
| 92.36 | 99.25 | 98.33 | 3543 |

**Table 26.** For cats with only 1 creatinine value - USG not selected

| Sensitivity | Specificity | Accuracy | Total Cats |
|---|---|---|---|
| 84.88 | 99.04 | 96.69 | 3506 |

The maximum Stage the cats had reached by the point of prediction was calculated when predicting the progression to Stage 3 at a later visit as shown in Table 27. Of the cats that had only reached Stage 0 at the time of prediction, 8 of the 3080 went on to get CKD (as defined by reaching Stage 3 at the next visit). The model only predicted one of these 8. It was reckoned that certain cases were acute failure, which advanced from Stage 0 to 3 in 6 to 9 months. The model correctly predicted 3049 of the Stage 0s to remain below Stage 3, and only falsely predicted 23. Hence an accuracy of 99% on the ones which started at Stage 0. For the cats that were Stage 0.5 at the visit before they reached Stage 3, the model correctly predicted 4 of the 16. Again, this can be a fast progression for CKD. For the cats that were Stage 1 and above at the prior visit (or before), the model predicted the Cases with an accuracy from 86% to 100% as the Stage at the earlier visit increased.

The intermediate stages between 0 and 3 were defined using an algorithm based on increased creatinine and decreased USG, but with severity too low to be classified as IRIS Stage 3. Most of them would be in the normal ranges, or only exceeding in one analyte, e.g. Stage 2.5 has low USG, but creatinine is high in the normal range at 2.6 to 2.8. For Stage 2, creatinine is 2 to 2.6 with low USG.

**Table 27.**

| Stage at Prediction | Sensitivity | Specificity | Accuracy | Total Cats | Years _from Diagnosis |
|---|---|---|---|---|---|
| 0 | 12.5 | 99.25 | 99.03 | 3080 | -0.74 |
| 0.5 | 25 | | 25 | 16 | -0.65 |

(continued)

| Stage at Prediction | Sensitivity | Specificity | Accuracy | Total Cats | Years _from Diagnosis |
|---|---|---|---|---|---|
| 1 | 86.3 | | 86.3 | 73 | -0.58 |
| 1.5 | 90.74 | | 90.74 | 54 | -0.45 |
| 2 | 99.07 | | 99.06 | 214 | -0.46 |
| 2.5 | 100 | | 100 | 106 | -0.43 |

Table 28 shows the same analysis, but done on cats with only one creatinine measure before the Stage 3 visit (i.e. predicted on only 1 creatinine measure + the other analytes).

**Table 28.**

| Stage at Prediction | Sensitivity | Specificity | Accuracy | Total Cats | Years_ from Diagnosis |
|---|---|---|---|---|---|
| 0 | 5.71 | 99.04 | 97.94 | 2959 | -0.77 |
| 0.5 | 28.57 | | 28.57 | 35 | -0.95 |
| 1 | 79.63 | | 79.63 | 108 | -0.66 |
| 1.5 | 90.28 | | 90.28 | 72 | -0.63 |
| 2 | 99.58 | | 99.58 | 236 | -0.71 |
| 2.5 | 100 | | 100 | 96 | -0.64 |

To be clear, the data did not necessarily mean that every cat that was at Stage 2 would progress to Stage 3 within 9 months. For this validation, cats were selected as being known to reach Stage 3, then chosen for the visit prior to reaching Stage 3. Therefore, for all these Cases, they were expected to reach Stage 3 at the next visit, and were used to test if the model would predict correctly or would predict a false negative. For the Controls, it was tested whether it would predict a false positive.

If cats with a data point at Stage 2 were randomly chosen without specifying that the next data point should be Stage 3, a similar ability to predict either steady state or progression would be observed. Therefore, not all Stage 2 cats would necessarily progress to Stage 3 in a short time (although from looking at thousands of cats, it appeared that progression was more frequent than expected), but that the model was effective at spotting the cats that would progress (and those that won't) from the mid to late Stages, but understandably may not easily spot Stage 0 or 0.5 cats that would progress rapidly to Stage 3, as the nature of the disease in those cats was probably different.

**Example 7**

The prediction model based on six biomarkers described in Examples 1, 5 and 6 was further improved with even more cats at a higher data quality level. The predictive ability on purebred cats in the veterinary database was verified. The model performance (e.g. accuracy) on blind data from the veterinary database has increased by around 1%. The 1% accuracy increase represents a big reduction in false positive rate in most cases e.g. a 40% decrease from 2.6 to 1.5% false positives.

The total cats for training was 53,590 cats, and over 300,000 visits with chemistry data. The total cats for blind testing was 150,000 cats, and over 700,000 visits with chemistry data.

Table 29 shows the prediction accuracy at 1 year before diagnosis for mixed breeds and all of the common breeds in the veterinary database, using relatively uncleaned data. The slight variations in accuracy were caused by random variation due to low numbers of cats in certain groups (e.g. 86 Red Tabby cats compared to 25,248 DSH). Apparent accuracy on Siamese and Himalayan cats was slightly lower due to a higher prevalence of CKD in these breeds. However, the Sensitivity and Specificity were both high.

**Table 29.**

| Breed | Sensitivity | Specificity | Accuracy | Total Cats |
|---|---|---|---|---|
| American Short Hair | 71.13 | 94.59 | 89.05 | 411 |
| Bengal | 61.54 | 97.32 | 89.89 | 188 |

(continued)

| Breed | Sensitivity | Specificity | Accuracy | Total Cats |
|---|---|---|---|---|
| DLH | 71.2 | 97.77 | 90.06 | 4106 |
| DMH | 68.69 | 97.96 | 91.16 | 4975 |
| DSH | 69.1 | 98.17 | 91.51 | 25248 |
| Himalayan | 64.49 | 98.37 | 87.84 | 444 |
| Maine Coon | 72.22 | 97.14 | 90.55 | 476 |
| Manx | 76.47 | 100 | 93.75 | 128 |
| Persian | 65.79 | 98.27 | 89.59 | 711 |
| Ragdoll | 73.44 | 98.7 | 93.2 | 294 |
| Red Tabby | 71.43 | 98.61 | 94.19 | 86 |
| Russian Blue | 70.69 | 99.37 | 91.67 | 216 |
| Siamese | 69.6 | 97.52 | 88.4 | 1078 |
| Tortoise-Shell Persian | 67.44 | 97.7 | 87.69 | 130 |

## Example 8

Using a new methodology to select appropriate variables for predictive modelling, a new and simpler cat CKD model was developed, which is named CKD4. CKD4, which was developed on data from the veterinary database, uses Creatinine, BUN, Urine Specific Gravity and Age, and uses longitudinal data across multiple visits, though single visits can also be used. In comparison, the CKD3 models disclosed in Examples 2 and 4 use Creatinine, BUN and Urine Specific Gravity, and data from a single visit; and the CKD6 models disclosed in Examples 1 and 5-7 use Creatinine, BUN, Urine Specific Gravity, Urine pH, Urine Protein and WBC count, and uses longitudinal data across multiple visits, though single visits can also be used.

A benefit of CKD4 is that is it less demanding in terms of needing blood count data, urine pH or urine protein. Therefore, it is applicable in many more clinics and visits where these data have not been collected.

Comparing the performance of CKD6 and the simpler CKD4 on longitudinal blind data (150,000 cats in the veterinary database), the models disagreed on only around 4% of predictions. Of these predictions, CKD4 was better at predicting the Controls (83% correct vs. 33%). CKD6 was better at predicting the Cases (77% vs. 20%). On longitudinal data CKD4 was less than 1% worse in terms of accuracy compared to CKD6. However, CKD4 had a better positive predictive value (PPV) of 94.9% vs. 92% at 1 year from diagnosis, which indicated that its sensitivity was slightly lower, but its specificity was higher (Table 30). CKD6 performed slightly better at more distant times before diagnosis, e.g., 2.5 years or longer.

On single visit data, the performance was reversed. Both models only disagreed on 4.9% of predictions. Overall, CKD4 was between 2 and 3% more accurate than CKD6. At 1 year before diagnosis, where BUN, Creatinine and USG data were available from a single visit, CKD4 was 92% accurate with a PPV of 89% (Table 31). CKD4 performed slightly better at more distant times before diagnosis, e.g., 2.5 years or longer.

In the tables below, "Time Split" refers to years before the official diagnosis listed in the veterinary database, e.g., Time Split 2 indicates predicting risk 2 years before official diagnosis. The two models were compared on blind data at each time point.

**Table 30.** Comparison of both models on blind longitudinal data with 2 or more visits and creatinine measures on more than 50% of the visits, and USG measures on more than 25%.

| Mode | Time Split | Sensitivity | Specificity | Accuracy | Total Cats | PPV |
|---|---|---|---|---|---|---|
| CKD6 | 0 | 93.58 | 98.18 | 95.81 | 44906 | 98.2 |
| CKD4 | 0 | 90.85 | 98.89 | 94.75 | 44906 | 98.87 |
| CKD6 | 0.5 | 80.14 | 98.09 | 94.03 | 46643 | 92.43 |
| CKD4 | 0.5 | 76.01 | 98.81 | 93.66 | 46643 | 94.92 |

(continued)

| Mode | Time Split | Sensitivity | Specificity | Accuracy | Total Cats | PPV |
|------|-----------|-------------|-------------|----------|-----------|-----|
| CKD6 | 1 | 69.5 | 98.07 | 91.15 | 40592 | 92 |
| CKD4 | 1 | 64.31 | 98.89 | 90.52 | 40592 | 94.9 |
| CKD6 | 1.5 | 59.47 | 98.04 | 88.87 | 33336 | 90.43 |
| CKD4 | 1.5 | 53.11 | 98.99 | 88.08 | 33336 | 94.25 |
| CKD6 | 2 | 51.96 | 98.01 | 86.24 | 27549 | 89.97 |
| CKD4 | 2 | 45.44 | 98.98 | 85.3 | 27549 | 93.87 |
| CKD6 | 2.5 | 42.12 | 97.94 | 83.8 | 22424 | 87.39 |
| CKD4 | 2.5 | 35.46 | 99.03 | 82.93 | 22424 | 92.56 |
| CKD6 | 3 | 36.34 | 97.99 | 81.01 | 16378 | 87.33 |
| CKD4 | 3 | 28.34 | 98.99 | 79.52 | 16378 | 91.42 |
| CKD6 | 3.5 | 31.35 | 97.87 | 78.75 | 7434 | 85.57 |
| CKD4 | 3.5 | 23.54 | 99.13 | 77.4 | 7434 | 91.62 |

Table 31. Comparison of both models on blind single visit data where creatinine, BUN and USG results are available

| Mode | Time Split | Sensitivity | Specificity | Accuracy | Total Cats | PPV |
|------|-----------|-------------|-------------|----------|-----------|-----|
| CKD6 | 0 | 90.91 | 98.09 | 94.31 | 27052 | 98.15 |
| CKD4 | 0 | 93.39 | 96.7 | 94.96 | 27052 | 96.93 |
| CKD6 | 0.5 | 76.06 | 98.23 | 93.93 | 27984 | 91.16 |
| CKD4 | 0.5 | 83.75 | 97.2 | 94.59 | 27984 | 87.79 |
| CKD6 | 1 | 63.39 | 98.23 | 90.47 | 25468 | 91.1 |
| CKD4 | 1 | 73.09 | 97.49 | 92.06 | 25468 | 89.31 |
| CKD6 | 1.5 | 52.18 | 98.53 | 88.71 | 21564 | 90.51 |
| CKD4 | 1.5 | 63.21 | 98.02 | 90.65 | 21564 | 89.55 |
| CKD6 | 2 | 44.27 | 98.2 | 85.01 | 18857 | 88.82 |
| CKD4 | 2 | 54.92 | 98.38 | 87.76 | 18857 | 91.64 |
| CKD6 | 2.5 | 37.51 | 98.3 | 83.64 | 15570 | 87.52 |
| CKD4 | 2.5 | 48.94 | 98.33 | 86.42 | 15570 | 90.32 |
| CKD6 | 3 | 31.36 | 98.41 | 79.97 | 12714 | 88.17 |
| CKD4 | 3 | 40.06 | 98.72 | 82.59 | 12714 | 92.23 |
| CKD6 | 3.5 | 27.1 | 98.3 | 78.77 | 9944 | 85.73 |
| CKD4 | 3.5 | 35.17 | 98.82 | 81.37 | 9944 | 91.86 |
| CKD6 | 4 | 21.6 | 98.23 | 75.64 | 5858 | 83.63 |
| CKD4 | 4 | 26.29 | 98.81 | 77.43 | 5858 | 90.26 |

## Example 9

Chronic kidney disease (CKD) is defined as evidence of functional impairment or structural damage to the kidney resulting in a reduction in glomerular filtration rate (GFR). CKD has been described as the leading cause of mortality in cats over the age of five (O'Neill et al. 2015), with a prevalence of between 8 and 31% reported in geriatric cats (O'Neill et al. 2014; Lulich et al. 1992; Marino et al. 2014). The aetiology of many feline CKD cases remains unclear, with histological investigations highlighting nephritis and renal fibrosis that may have resulted from a range of underlying causes including toxic insults, hypoxia, chronic glomerulonephritis, chronic pyelonephritis, upper urinary tract obstructions, and viral infections (Brown et al. 2016). The prognosis for cats with CKD depends on the severity of the disease at the time of diagnosis, with cats identified at IRIS stage 4 reported to have a 9- to 25-fold shorter life expectancy than those diagnosed at IRIS stage 2 (Boyd et al. 2008; Geddes et al. 2013; Syme et al. 2006). Early detection of CKD allows the implementation of care pathways that can slow the progression of the disease, improving clinical outlook and quality of life, as well as the avoidance of situations that may cause worsening of kidney function and acute kidney injury (e.g. administration of NSAIDs; Levin and Stevens, 2011).

A single, accurate biomarker to assess renal function in clinical practice does not currently exist (Sparks et al. 2016). While the measurement of GFR provides a direct assessment of renal function, accepted methods are technically challenging to implement in clinical settings. Consequently, serum creatinine remains the standard surrogate for GFR, both as part of the initial diagnosis, as well as when staging the disease using recognised criteria (e.g. IRIS; Finch 2014). Further traditional clinical biomarkers, including urea, proteinuria (an elevated urine protein to creatinine ratio; UP/C), blood pressure and urine specific gravity may also be referenced as part of the diagnosis with UP/C and blood pressure used to substage cats when deciding on the appropriate care pathway. More recently the use of serum symmetric dimethylarginine (SDMA) has become popular in clinical practice, due to early evidence that it is responsive to changes in renal function sooner than serum creatinine, enabling the early detection of CKD in non-azotemic cats (Hall et al. 2014). Additionally fibroblast growth factor-23 (FGF23), an important factor in the regulation of phosphate and vitamin D metabolism, has been shown to increase in the circulation before development of azotemia as GFR declines (Finch et al. 2013). These more recent CKD biomarkers represent progress in the development of diagnostic tests to detect feline CKD with greater sensitivity or at an earlier stage, but due to the complex nature of the disease, further research is needed to fully understand the clinical value of these approaches.

In human healthcare, machine learning models have been used to assess risk and inform practice management (Parikh et al. 2016), predict individual outcomes (Peck et al. 2012; Peck et al. 2013), length-of-stay (Gultepe et al. 2013), recommend treatments (Tsoukalas et al. 2015), and personalized medicine (Callahan et al 2018; Pencina et al. 2016).

In this study a data set of 106,251 individual cat electronic health records (EHRs) from routine veterinary practice were used to train and then validate an algorithm that predicts the risk of cats developing azotemic CKD with high specificity. The clinical use of this algorithm for early diagnosis and the options this brings for new clinical care pathways were discussed.

*Methods*

*1. Data source and initial cleansing*

Data were extracted from electronic health records (EHRs) of cats visiting BANFIELD pet hospitals (Vancouver, WA, USA) between January 1, 1995 and December 31, 2017. At the close of this time period, over 1000 BANFIELD hospitals were operated across 42 US states. Data collected from cats before the age of 1.5 and after the age of 22 years was excluded. With the further inclusion criterion of at least 3 clinic visits per cat this yielded a sample of 910,786 cats. The sample contained domestic short, medium and long haired cats and over 50 pedigree breeds. Extreme outliers for blood and urine tests - more than 6 standard deviations above the maximum of the normal range - were set to missing.

Each individual EHR included patient demographic data (age, breed, body weight and reproductive status), blood and urine test results, and clinical information (formal diagnosis and unstructured medical notes). In total 35 types of information were selected as features for a CKD prediction model. Data points were primarily collected during or around hospital visits, with individual visits timestamped meaning that the data was intrinsically longitudinal.

*2. CKD status and age at evaluation T0*

EHRs in the study dataset were classified in 3 CKD status groups. The first group consists of EHRs with a formally recorded CKD diagnosis ("CKD"). The age of the first CKD diagnosis was used as the age at evaluation (T0). For this group, data collected more than 30 days after the diagnosis was excluded (an additional 30 day window was included to capture serum, blood or urine test data that was returned shortly after the diagnosis visit).

EHRs without a formal CKD diagnosis, but with at least two CKD-suggesting data points from the following list: blood creatinine above normal values, urine-specific gravity below normal values, and "CKD", "azotemic", "ROYAL CANIN Veterinary diet Renal" or "Hill's prescription diet k/d" in the medical notes were classified as "probable CKD". While the exact reason for a lack of a formal diagnosis remains uncertain for these EHRs, it is likely that the veterinarian was either unsure about the diagnosis or did not fill in a formal diagnosis. For this group the age at evaluation (T0) was set to the age at last available visit, and the complete EHR was used.

All EHRs that were not included in the two previous groups, and that have at least 2 years of data (recorded visits) at the end of the EHR to validate absence of CKD were assigned a "no CKD" status. For these EHRs age at evaluation (T0) was set as the age at the last visit minus 2 years, and the last 2 years of data were removed from the EHR.

*3. Data sets for model building and testing*

The truncated EHRs were further filtered based on their information content by imposing that the EHR should include at least 2 visits with accompanying blood creatinine data. This resulted in a data set with 106,251 individual cat EHRs. This data set was randomly split in two parts. In total 70,687 EHRs or approximately 67% of the data was used to build the CKD prediction model. The remaining 35,564 EHRs or approximately 33% were used as a test set to evaluate the model performance. Both data sets were kept separate throughout the analysis to exclude any bias at the testing stage. Prior to use, missing information in the blood and urine test data was imputed without using the CKD status information. This was done separately for model building and test data sets to avoid any flow of information between the two datasets.

*4. Model building*

Prior to use the model building dataset was filtered further ensuring that only the best characterized EHRs were used for learning. EHRs with status "probable CKD" were removed as were 7,549 "CKD" and "no CKD" EHRs with "acute kidney injury" or "urinary tract infection" as comorbidity. This left 53,590 EHRs of which 9,586 were "CKD" and 44,004 "no CKD". To enable the model to work for early detection of CKD, this dataset was then augmented (Perez and Wang, 2017) by adding truncated versions of the original EHRs (last k visits removed with k ranging from 1 to the total number of visits -1). This enriched the dataset with EHRs having a gap of up to 2 years between the last visit seen by the model and the time of diagnosis.

The first step towards a CKD prediction model was to select a limited set of features to be included. Feature selection was conducted by a top-down and bottom-up wrapper method (Tang et al., 2014) using a standard recurrent neural network (RNN, (Goodfellow et al. 2016) Figure 15) with a 3-5-3 hidden layer structure. This RNN model was selected based on exploratory studies (results not shown) where it outperformed alternatives such as k-nearest neighbour with dynamic time warping (KNN-DTW) (Salvador and Stan 2007) and a long short-term memory RNN alternative (LSTM, (Gulli and Pal 2017) , Figure 15). The RNN was implemented with a tanh activation function in the hidden layers and softmax for transforming the output layer into a CKD probability score. Backpropagation through time was used for training with the RMSprop gradient optimization algorithm. Model performance was evaluated based on the F1 cross-entropy in a 3-fold cross-validation setup. The F1 cross-entropy was used as a metric because it balances sensitivity and specificity independent of CKD incidence.

Next a full model architecture screen was performed with the selected features for the above-mentioned RNN structure as well as for a LSTM alternative. For both structures, different configurations of 1 to 5 hidden layers were tested with 3 to 200 nodes per layer. The setup was the same as above except that 20% dropout was added to avoid overfitting (Srivastava et al., 2014). Evaluation was based on the F1 score in a 10-fold cross-validation setup (Powers et al, 2011). Finally the best model configuration was fine-tuned with respect to the training time in the same cross-validation set-up.

*5. Model testing*

Unbiased model performance was assessed by applying the selected prediction model to the test dataset. Predictions were performed for all EHRs in the "CKD", "probable CKD" and "no CKD" groups. Results were interpreted at the level of the crude model output - the probability p of a CKD diagnosis - as well as after categorisation into "no CKD" and "CKD" using p=0.5 as the cut-off point. Categorical results for "CKD" and "no CKD" groups were used to compute sensitivity (proportion of true positives, "CKD" status predicted as CKD) and specificity (proportion of true negatives, "no CKD" predicted as no CKD) estimates, respectively. Confidence intervals for sensitivity and specificity estimates were calculated using the normal approximation. Odds ratio tests for the comorbidity analysis (Table 35) were done with a standard chi-square test.

The ability for the model to predict CKD ahead of the definitive diagnosis was evaluated by truncating the EHRs to various time points before age at diagnosis for the "CKD" group.

*5. Software*

General data management, statistical analyses and plots were performed using R version 3.4.3 (R Core Team, 2017) and imputation was done with the MissForest package version 1.4 (Stekhoven et al., 2012). Machine learning work was done using Tensorflow version 1.3 (github.com) and interfaced from within Python using Keras Deep Learning library version 2.0.8 (faroit.github.io) run on a 500-core, 4 GB memory per core Dell PowerEdge R730xd cluster with dual Intel E5-2690 v3 CPUs.

## Results

### 1. Study dataset and clinical CKD diagnosis

This study was performed on an extract of 106,251 individual cat EHRs of BANFIELD pet hospital visits between 1995 and 2017. Demographics of this sample differentiated by CKD status and summaries of blood and urine test data at the time of diagnosis are shown in Table 33. The CKD prevalence in this sample was 17% when based on the "CKD" status group only, and 42% when including "probable CKD" cats in addition. Cats with "CKD" status were older than "no CKD" cats. The prevalence of missing data was approximately 9% for most of the blood chemistry measures and up to 62% for urine test results, which are not routinely measured on every visit. Results are very similar after breakdown in a model building and test data set (Table 32) showing that these can be used as independent samples of the same population.

**Table 32.** Demographics and summaries for the study data set, split by training and test sets.

|  | No CKD | Probable CKD | CKD |
|---|---|---|---|
| **Training** |  |  |  |
| Mean age (years) at T0 | 6.6 | 10.7 | 13.1 |
| Mean weight (kg) at T0 | 5.55 | 5.24 | 4.47 |
| Mean creatinine (mg/dL) at T0 | 1.7 | 1.9 | 2.8 |
| Mean Urine SG at T0 | 1.05 | 1.035 | 1.02 |
| Percent Missing Creatinine Values | 7% | 10% | 11% |
| Percent Missing Urine SG Values | 68% | 57% | 56% |
|  |  |  |  |
| **Test** |  |  |  |
| Mean age (years) at T0 | 6.5 | 10.6 | 13.1 |
| Mean weight (kg) at T0 | 5.53 | 5.24 | 4.55 |
| Mean creatinine (mg/dL) at T0 | 1.7 | 1.9 | 2.9 |
| Mean Urine SG at T0 | 1.05 | 1.036 | 1.02 |
| Percent Missing Creatinine Values | 7% | 10% | 11% |
| Percent Missing Urine SG Values | 68% | 58% | 57% |

**Table 33.** Demographics and summaries for the study data set.

|  | No CKD | Probable CKD | CKD |
|---|---|---|---|
| **Number of Cats** | 61,239 | 26,604 | 18,408 |
| **Mean visits per Cat** | 5.4 | 10.9 | 8.2 |
| **Male to Female ratio** | 1 : 0.95 | 1 : 1.14 | 1 : 0.92 |
| **Mean age (years) at T0** | 6.6 | 10.7 | 13.1 |
| **Mean weight (kg) at T0** | 5.54 | 5.24 | 4.49 |
| **Mean creatinine (mg/dL) at T0** | 1.70 | 1.90 | 2.81 |
| **Mean Urine SG at T0** | 1.050 | 1.035 | 1.020 |
| **Percent Missing Creatinine Values** | 7% | 10% | 11% |

(continued)

| | No CKD | Probable CKD | CKD |
|---|---|---|---|
| **Percent Missing Urine SG Values** | 68% | 57% | 56% |

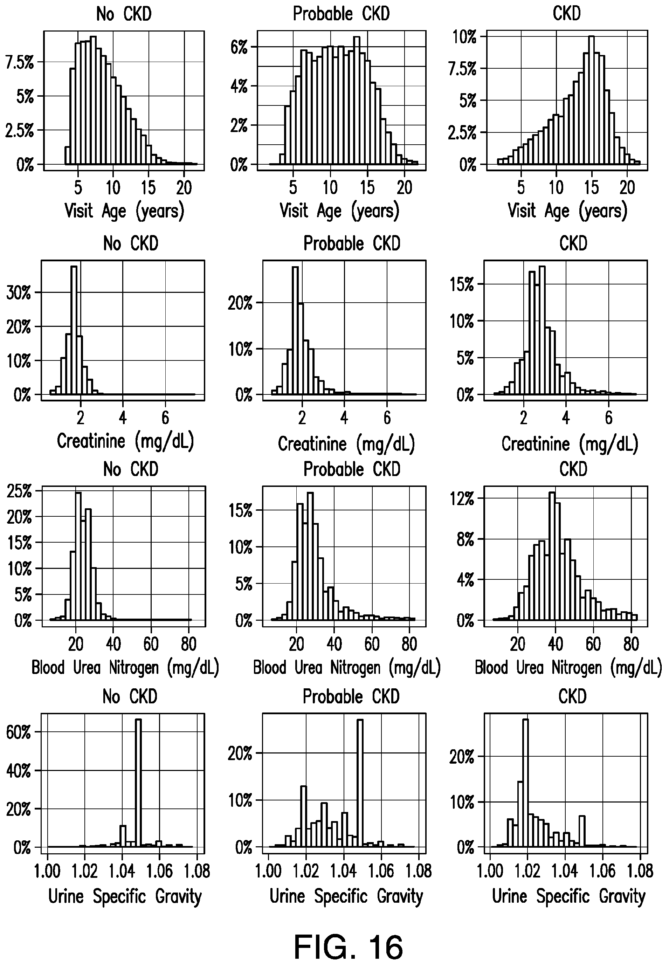

As multiple guidelines for the diagnosis of CKD exist, and these have evolved during the period captured in this study, how the CKD status as used in this study relates to various diagnostic parameters routinely assessed when making CKD diagnoses was explored. Cats with status "CKD" were generally older, have higher creatinine levels and lower USG, compared to cats with "no CKD" status (Figure 16). These results support the quality of the CKD diagnosis within the BANFIELD database versus accepted diagnostic criteria and provides confidence in the background data used to build the model. For all criteria assessed there was a significant overlap in the distributions between CKD status groups, such that any single parameter alone does not have sufficient discriminatory power for diagnosis. This intrinsically multifactorial nature of feline CKD presents an ideal setting for prediction models to add clinical value.

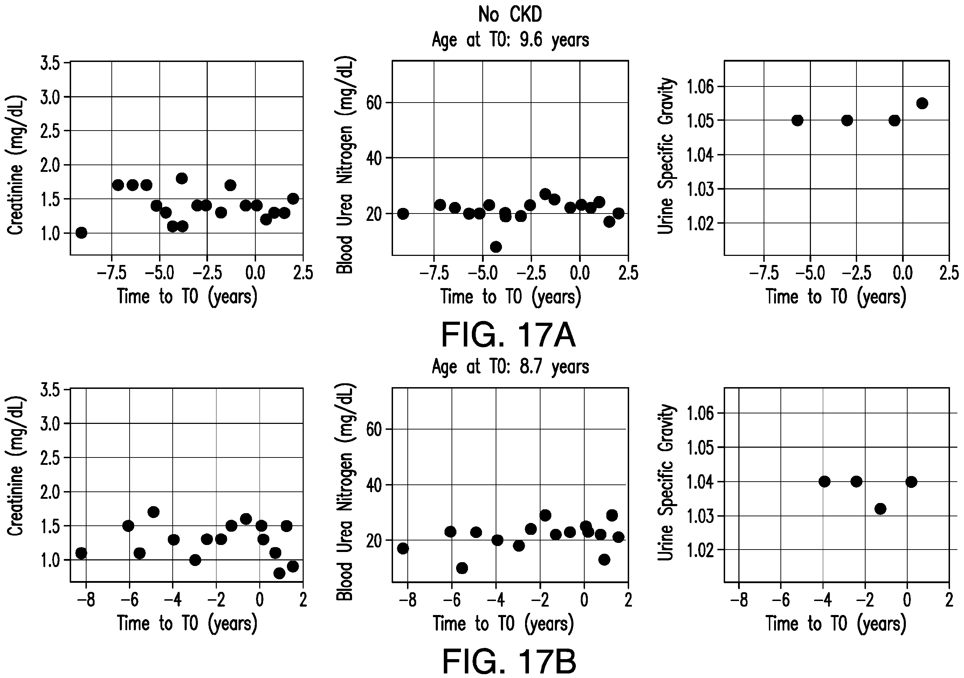

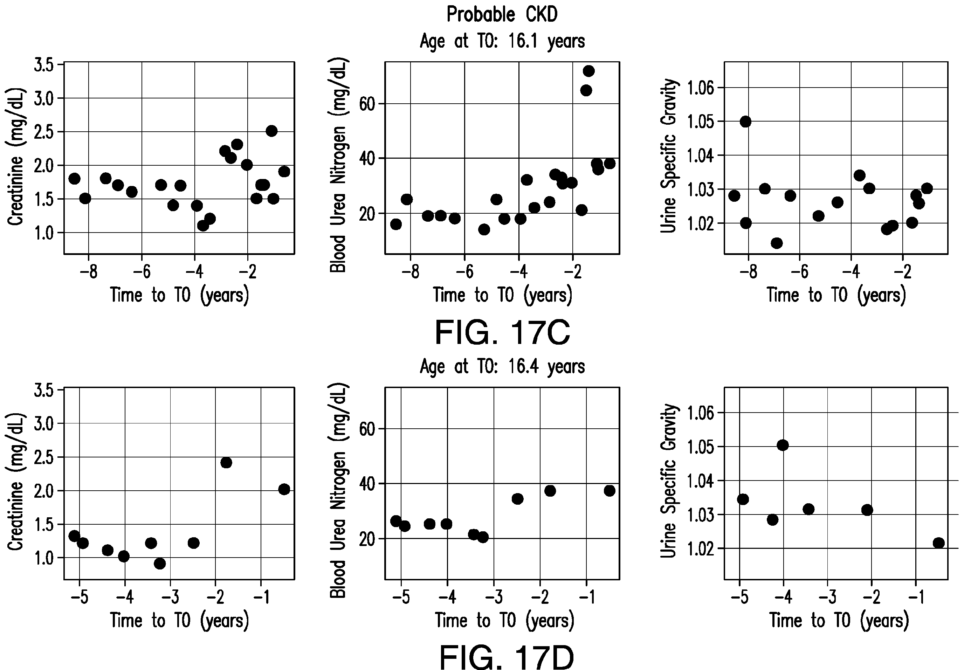

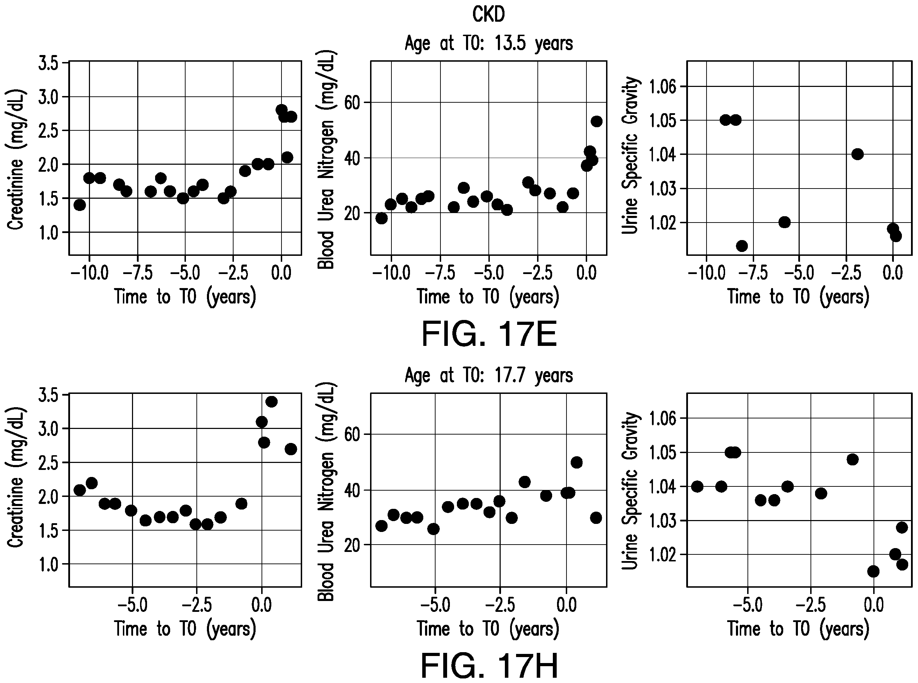

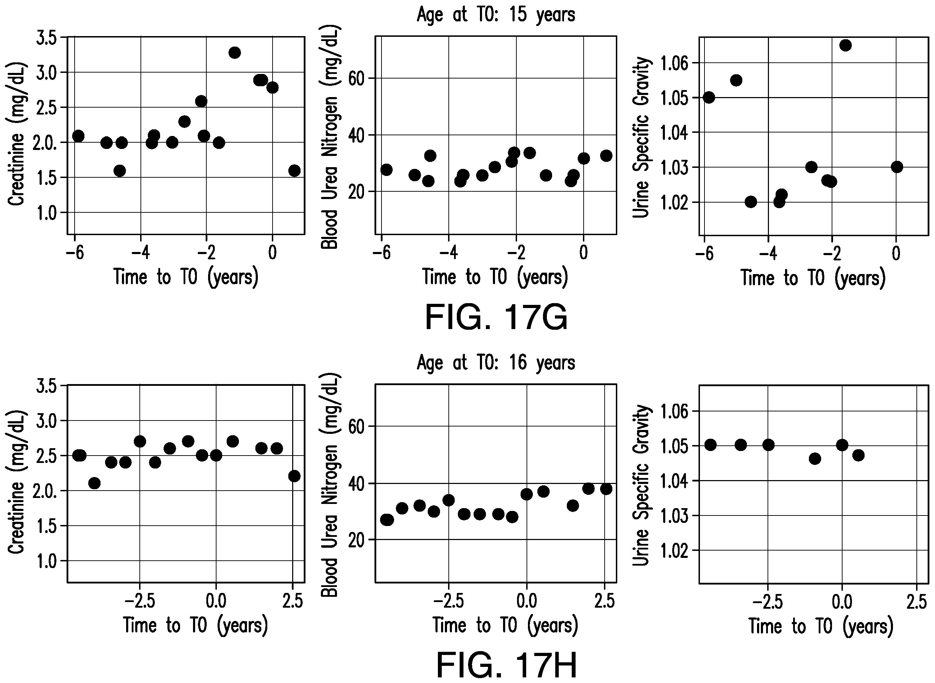

Veterinarians refer to historical (longitudinal) data when making a diagnosis and further analysis of these diagnostic parameters within the EHRs highlighted a range of changes in these parameters, not only based on the status of the cat, but also within the status grouping (Figure 17). This shows that a prediction model should not only consider multiple factors at the time of diagnosis, but also include information on these at different time points before diagnosis as well.

*2. Building a prediction model for CKD*

A standard RNN with a 3-5-3 hidden layer structure was used as a starting point for a prediction model for CKD that acknowledges both the multifactorial and temporal aspects of CKD diagnosis. Using this type of model with 35 candidate factors or features was impractical both for training the model as well as for using it in practice later. Therefore, the most important features were first selected using a top-down and bottom-up feature selection strategy on the training data set. This approach showed that model performance in terms of the cross-entropy score improved by adding features up to 4 and plateaued thereafter (data not shown). As a result, a prediction model with the following features: creatinine, blood urea nitrogen, urine specific gravity and visit age was built.

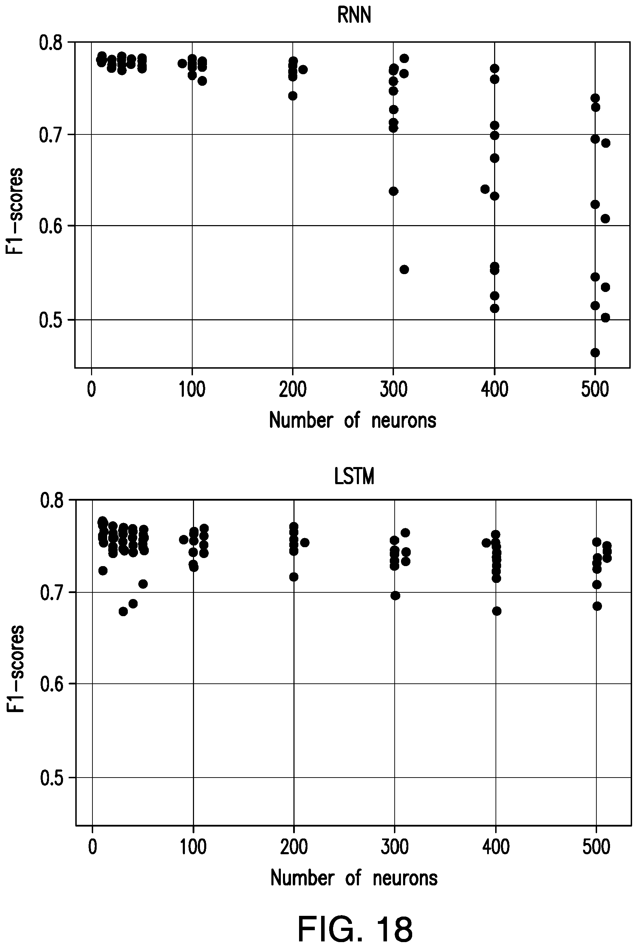

With these 4 features, the best structure for the hidden layers - number of layers and nodes per layer - for a standard RNN and a LSTM variant was determined. Results in terms of cross-entropy score (Figure 18) and the notion that higher cross-entropy scores are better, demonstrated that RNN models were slightly superior to LSTM models. For the RNN, the simpler models with a small number of nodes were better than the complex ones. A two-layer RNN with a 3-7 structure was best. Optimizing this one for training time by testing different numbers of epochs resulted in a final RNN model with a 3-7 structure trained over 16 epochs.

*3. Detecting CKD at the point of diagnosis*

To understand the clinical value of the CKD model, it was applied on the test dataset of 40,205 cat EHRs that were not used for building the model. The model (Table 34) showed a sensitivity of 90.7% (6,418/6,943) based on the status "CKD" and a specificity of 98.9% (22,166/23,432) based on the status "no CKD" (Table 34). Predictions for the "probable CKD" group are split over the "CKD" and "no CKD" predictions.

**Table 34.** A comparison of diagnosed CKD status against predicted status at T0

| | Predicted "no CKD" | Predicted "CKD" | Total |
|---|---|---|---|
| Status "no CKD" | 22166 | 1266 | 23432 |
| Status "probable CKD" | 4223 | 5608 | 9831 |
| Status "CKD" | 524 | 6418 | 6943 |
| Total | 26913 | 13292 | 40205 |

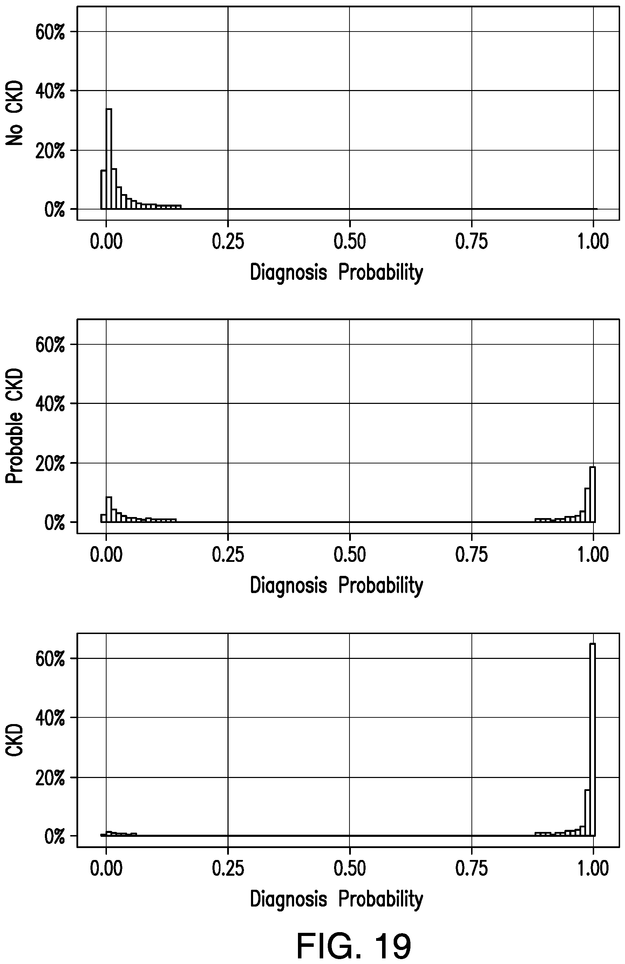

Distributions of the raw CKD prediction model output (Figure 19) show similarly clear pictures for "no CKD" and "CKD" status groups: positioned close to 0 for "no CKD" and close to 1 for "CKD". The "probable CKD" status group is more mixed with about 30% close to 1 and the rest spread out around 0.5 possibly suggesting either diagnosis was ambiguous or early stage cases.

Whether misclassification for "no CKD" cats was linked to specific co-morbidities by comparing co-morbidity incidence between correctly and incorrectly classified "no CKD" cats was also evaluated. It was found that hyperthyroidism and diabetes mellitus are clearly overrepresented in falsely positive classified cats as are hepatopathy and underweight (Table 35).

**Table 35.** Incidence (%) of the 20 most common comorbidities for "No CKD" cats differentiated by their predicted CKD status. The odds ratio for the comorbidity in "predicted as no CKD" versus "predicted as CKD" is given with an uncorrected p-value for a hypothesis test with odds ratio = 1 as null hypothesis.

| Comorbidity | Incidence in predicted no CKD (%) | Incidence in predicted CKD (%) | ODDS_RATIO | P value |
|---|---|---|---|---|
| Hyperthyroidism | 3.18 | 22.03 | 0.116 | $< 10^{-5}$ |
| Diabetes Mellitus | 3.37 | 13.56 | 0.222 | $< 10^{-5}$ |
| Hepatopathy | 4.63 | 11.86 | 0.361 | 0.0004 |
| Underweight | 5.8 | 13.56 | 0.392 | 0.0006 |
| Murmur | 10.32 | 19.49 | 0.475 | 0.0015 |
| Arthritis | 2.23 | 6.78 | 0.313 | 0.0018 |
| Malaise | 11.08 | 18.64 | 0.544 | 0.0106 |
| Constipation, Conservative | 3.29 | 6.78 | 0.468 | 0.0403 |
| Gastroenteritis, Conservative | 5.77 | 10.17 | 0.541 | 0.0455 |
| Vomiting, Conservative | 8.87 | 13.56 | 0.620 | 0.0782 |
| Inflammatory Bowel Disease | 1.4 | 3.39 | 0.406 | 0.0799 |
| Crystalluria | 5.37 | 1.69 | 3.288 | 0.0957 |
| Enteritis, Conservative | 3.29 | 0.85 | 3.984 | 0.1693 |
| Urinary Tract Infection | 8.02 | 5.08 | 1.627 | 0.2472 |
| Respiratory Disease, Upper | 11.51 | 9.32 | 1.265 | 0.4594 |
| Urinary Tract Disease | 4.2 | 3.39 | 1.250 | 0.6627 |
| Obesity | 14.12 | 15.25 | 0.913 | 0.7240 |
| Inappropriate Elimination | 6.4 | 5.93 | 1.085 | 0.8357 |
| Cystitis | 21.94 | 21.19 | 1.045 | 0.8442 |
| Colitis, Conservative | 6.98 | 6.78 | 1.032 | 0.9324 |

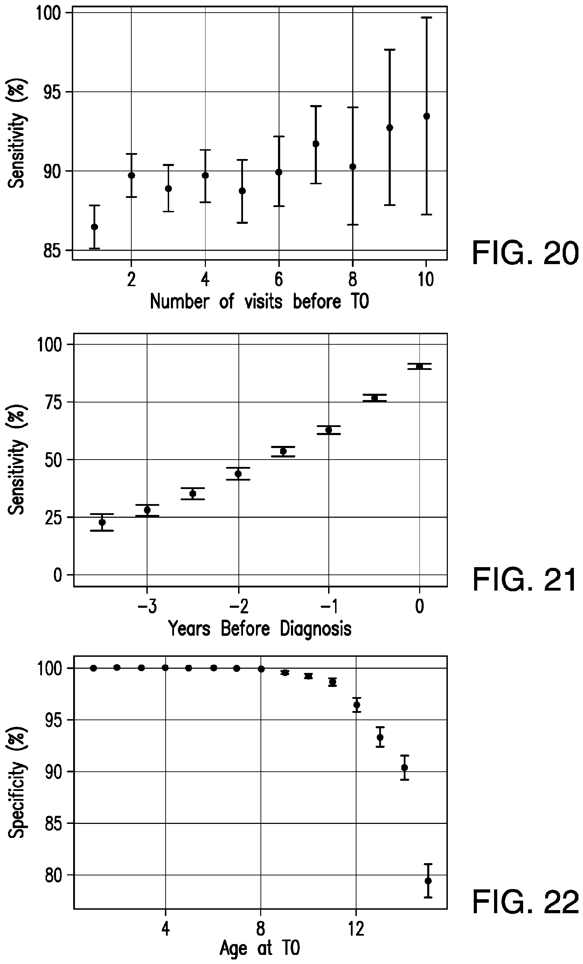

The influence of the amount of prior information (number of visits) on the prediction sensitivity is an important consideration when evaluating the clinical implementation of such an approach. The general model performance data does not address this consideration because it is based on the complete sample of EHRs that includes a range of visits from 1 to 15. Therefore, the model sensitivity was next examined by number of visits in the EHR before the visit where the diagnosis was made. It was found that sensitivity clearly benefits from prior information as it increases up to approximately 90% by using at least 2 visits prior to the diagnosis (Figure 20). This shows that historical information contributes to the diagnosis of CKD up to a horizon of 2 visits which is on average 2 years.

*4. Using the model for early detection*

As the model detects CKD signals around 2 years before the diagnosis, its use for early prediction of future disease risk was evaluated. To achieve this, EHRs were truncated at different points before diagnosis (e.g. for a 1 year early prediction, all information between the diagnosis and 1 year before was removed) and then evaluated the ability of the model to predict future onset of CKD. As expected, sensitivity (Figure 21) decreased when increasing the time between prediction and diagnosis, although of the cats that went on to develop CKD 63% were correctly predicted 1 year before diagnosis and 44.2% 2 years before diagnosis.

To assess specificity in this context, truncation of the EHRs does not make sense as cats remain "no CKD" at all earlier visits to clinic. Therefore, specificity was instead calculated as a function of age at evaluation (Figure 22). Specificity was consistently above 98% until an age of 11 years and declined thereafter reaching 80% for an age of 15 years.

*Discussion*

Computational modelling approaches were applied to a large, rich data set of electronic health records (EHRs) from routine veterinary practice to derive and then validate an algorithm that diagnoses CKD, as well as predicting the risk of cats developing azotemic CKD in the future. From an initial set of 35 candidate features, the model was refined down to 4 (creatinine, blood urea nitrogen, urine specific gravity and visit age). When predicting CKD near the point of diagnosis, the model displayed a sensitivity of 90.7% and a specificity of 98.9%. Interestingly, prediction of CKD risk was possible with 63.0% and 44.2% sensitivity, one and two years before diagnosis, respectively. Specificity was over 99% at both advanced time points.

The selected model features that enable the prediction of the onset of azotemic CKD are routinely referenced by veterinarians when CKD is suspected, and are therefore mechanistically implicated in the disease aetiology. Creatinine and blood urea nitrogen concentrations are filtration markers and their retention in the circulation can indicate reduced functional renal mass. As urea more readily crosses lipid membranes than creatinine and the permeability of the collecting tubule and duct to urea is selectively increased by antidiuretic hormone, urea is retained in the blood not only when functional kidney mass is reduced, but also when the body is responding to water deficits and activating mechanisms that conserve water. Inclusion of both creatinine and urea in this model may help the system avoid falsely identifying acutely volume depleted felines as having CKD; under these circumstances urea would change far more than plasma creatinine. Serial monitoring of creatinine is more sensitive in identifying loss of kidney mass than a single one-off measurement, as creatinine production can be influenced by non-renal factors (e.g. muscle mass; Sparkes et al. 2016). However, the strength of the approach described here is that the algorithm identifies changes over time in a range of diagnostic parameters that together are indicative of progressive deterioration in renal function. These, often subtle changes over time, may be missed by a veterinarian particularly when the laboratory values have not moved outside the normal range.

USG is a measure of the ability of the kidney to excrete solutes (mostly waste products) in excess of water, but as the functional kidney mass declines so does the USG. A single urine sample from a feline with normal healthy kidneys can have varying USG depending on whether the feline needs to conserve or excrete excess water, consequently single assessments are difficult to interpret. Cats often retain some concentrating ability in IRIS stages 2 and 3 CKD with the urine only approaching the isothenuric range as they approach IRIS stage 4 CKD (Elliott et al. 2003). Interpreting serial data on USG in combination with plasma creatinine and blood urea nitrogen likely helps the model to identify patterns predictive of falling kidney functional mass and differentiate these from natural fluctuations around normal or acute episodes of dehydration.

Finally, as CKD is primarily a disease of age it is not surprising that the age of the cat was selected as a feature in the final model. As highlighted in Table 33 the age profiles of the "no CKD" and "CKD" groups were different, but there was sufficient overlap to challenge the model on young as well as old cats. The proportions and age distributions represent the real distribution of cats seen by BANFIELD clinics over the last 20 years. Aging is associated with a range of chronic conditions and CKD is commonly diagnosed before or at the same time as hypertension, hyperthyroidism and diabetes mellitus (Conroy et al. 2018). To understand how the model performed in situations where multiple diagnoses were present in the EHR, whether misclassification for "no CKD" or "CKD" by the model was linked to specific co-morbidities was also evaluated (Table 35). Hyperthyroidism and diabetes mellitus were overrepresented in falsely positive classified cats, most likely due to the non-specific nature of the clinical parameters routinely employed to inform diagnoses across these conditions. It should be noted that the relative performance of the model was mildly influenced by these cases, but this is a challenge that veterinarians also encounter in clinical practice.

The selection of biomarkers presented in this model represent a combination of parameters that gave high predictive accuracy under most clinical situations. Further work (beyond the scope of this paper) has highlighted that other biomarkers can be useful in predicting future CKD when applied using more complex combinations of models. These could, for example, function by reducing the loss of specificity when predicting very old cats (Figure 22) or help to separate other comorbidities (Table 35) more accurately. The other predictive biomarkers identified included urine protein, urine pH and white blood cell count. The volume of missing values related to these parameters in the historic data (due to them not being measured on all visits) has meant that they bring additional noise to the model as well as enhancing signal. Further testing with more complete datasets may show higher predictive power for these and other biomarkers.

Recently serum SDMA concentration has been suggested as an alternative marker of GFR, as it has been shown to correlate closely with plasma creatinine (Jepson et al., 2008) and plasma iohexol clearance in cats (Barff et al., 2014). Retrospective analysis of stored longitudinal samples collected as part of the management of a colony of cats used for nutrition studies showed that serum concentrations of SDMA increased outside of the laboratory reference range in 17 of 21 cats that developed azotemia before an increase in plasma creatinine was detected. On average, elevated SDMA was detected 17 months (range 1.5 to 48 months) prior to elevated creatinine (Hall et al., 2014). The small group of cats and the retrospective nature of this study likely overestimates the sensitivity and specificity of SDMA as a predictor of the development of azotemic CKD. SDMA was not available for much of the time period over which the data used in the present study were collected. It is interesting to note that the algorithms devised from these large longitudinal datasets involving very large numbers of felines presenting to veterinary practices with a range of different diseases were able to predict the development of azotemic CKD even 3 years prior to its onset using data routinely collected in veterinary practice. Whether longitudinal measurement of SDMA would improve the predictive value of the algorithms developed in the present study warrants further research.

Although EHR data is undoubtedly clinically relevant, using it in a scientific setting was a challenge. As such, confirming the accuracy of the CKD diagnosis was an important first step. Data used to build and validate this model came from a very large number of clinics and veterinarians over a period of more than 20 years and cats with a formal CKD diagnosis showed blood and urine patterns that are consistent with currently accepted guidelines (Figure 16); this in itself provides confidence in the use of these data as a reference point to develop the model. Defining the health status of the complementary set of cats without a formal CKD diagnosis was more problematic. A subset of these, those that were classified as "probable CKD", had clear indications for CKD in blood and/or urine test results or references in the medical notes that suggest CKD. This group of cats includes those where the veterinarian was either unsure of the diagnosis (most likely because of conflicting information) or because the cat was in an early stage of the disease, or where for formal reasons they could not be diagnosed. This group was not included when computing sensitivity however, and are aware that this could bias the estimates given that it could contain the more difficult cases to predict. For the other cats without a formal CKD diagnosis a 2-year window with observations and no CKD to be confident of their "no CKD" status was imposed. This also could have biased the specificity estimates as some might have had very early stage CKD.

The prognosis for cats with CKD depends on the severity of the disease at the time of diagnosis, with cats identified at IRIS stage 4 reported to have a significantly shorter life expectancy than those diagnosed at earlier stages (Boyd et al. 2008; Geddes et al. 2013; Syme et al. 2006). Early detection of CKD allows the early implementation of care pathways that can slow the progression of the disease, improving clinical outlook and quality of life, as well as the avoidance of situations that may cause worsening of kidney function and acute kidney injury (Levin and Stevens, 2011). Consequently work continues to develop and validate novel diagnostic tools that support clinicians in the early diagnosis of CKD and represent an improvement in the clinical measures routinely applied in current veterinary practice (e.g. plasma creatinine, USG); the limitations of which are well recognized. Here significant overlap in the distributions of a range of routinely applied diagnostic criteria between cats with and without a CKD diagnosis was demonstrated (Figure 16). This highlights the intrinsically multifactorial nature of CKD, meaning that a single existing clinical parameter alone does not have sufficient discriminatory power to inform a diagnosis.

The CKD prediction model developed in this study brings several advantages for veterinary practice. The first is to support the veterinarian in making the right diagnosis based on blood and urine test data currently available for a particular case. Diagnosis is complicated by the multifactorial nature of CKD, with individual cats often displaying differences in the evolution of these parameters (Figure 17), most likely due to subtle differences in the aetiology and progression of the disease. One might even argue whether humans are able to learn all possible patterns because these can be quite different between individual cats (compare, for example, CKD cats in Figures 17E with Fig 17H). Therefore, having an algorithm highlighting a risk for CKD can be a very helpful addition to a practicing veterinarian's toolkit. A second advantage is the ability of the algorithm to predict CKD risk ahead of conventional diagnostic strategies- with a success (sensitivity) of 44.2% 2 years before diagnosis and of 63% 1 year before diagnosis. To enable this early detection, however, it is important that cats not only regularly (biannual or annual) visit a veterinarian, but also that a blood and a urine sample is taken at each visit. Judging from the database this is currently not a common occurrence (Table 33). Approaches such as this highlight the value in preventative care, with an increased frequency of screening not only supporting the earlier detection of CKD, but in time also presenting opportunities to proactively monitor a broader range of conditions that are diagnosed through routine clinical measures. Finally, it is important to develop and validate care pathways based on the early prediction of CKD, e.g. starting a specifically formulated diet to slow down or halt disease progression.

In conclusion, here evidence for the use of machine learning to build an algorithm that predicts cats at risk of developing CKD up to 2 years prior to diagnosis was presented. The high specificity (>99%) of the algorithm, coupled with a sensitivity of 63%, means that out of 100 cats with a prevalence of 15%, 90 cases will be correctly predicted as either not developing azotemia or developing azotemia in the next 12 months. A particular strength of the current approach lies in the use of health screening data collected as part of routine veterinary practice, meaning that this model can be rapidly implemented into hospital practice and/or diagnostic laboratory software to directly support veterinarians in making clinical decisions.

*References*

Boyd LM, Langston C, Thompson K, et al. Survival in cats with naturally occurring chronic kidney disease (2000-2002). J Vet Intern Med 2008; 22: 1111-1117.

Brown SA. Management of chronic kidney disease. In Elliott J, Grauer GF (editors). British Small Animal Veterinary Association (BSAVA) Manual of Canine and Feline Nephrology and Urology 2007.

Brown CA, Elliott J, Schmiedt CW, Brown SA. Chronic Kidney Disease in Aged Cats: Clinical Features, Morphology, and Proposed Pathogeneses. Vet Pathol. 2016;53(2):309-26.

Callahan A, Shah NH. Machine Learning in Healthcare. Key Advances in Clinical Informatics 2018:279-291

Conroy M, Chang YM, Brodbelt D, Elliott J. Survival after diagnosis of hypertension in cats attending primary care practice in the United Kingdom. J Vet Intern Med. 2018;1-10.

Finch NC. Measurement of glomerular filtration rate in cats; Methods and advantages over routine markers of renal function. J Feline Med Surg. 2014;16(9):736-48.

Finch NC, Geddes RF, Syme HM, et al. Fibroblast growth factor 23 (FGF-23) concentrations in cats with early non azotemic chronic kidney disease (CKD) and in healthy geriatric cats. J Vet Intern Med 2013; 27: 227-233.

Geddes RF, Finch NC, Elliott J, et al. Fibroblast growth factor 23 in feline chronic kidney disease. J Vet Intern Med 2013;27: 234-241.

Gultepe, Eren, et al. From vital signs to clinical outcomes for patients with sepsis: a machine learning basis for a clinical decision support system. Journal of the American Medical Informatics Association 2013; 21.2: 315-325.

Hall JA, Yerramilli M, obare E, et al. Comparison of serum concentrations of symmetric dimethylarginine and creatinine as kidney function biomarkers in cats with chronic kidney disease. J Vet Intern Med 2014;28:1676-1683.

Hochreiter S, Schmidhuber J. Long Short-Term Memory. Neural Computation. 1997;9(8):1735-1780. https://doi.org/10.1162/neco.1997.9.8.1735.

Jepson RE, Brodbelt D, Vallance C, Syme HM, Elliott J. Evaluation of the predictors of azotemia in cats. J Vet Intern Med 2009;23:806-813

Levin A, Stevens PE. Early detection of CKD: the benefits, limitations and effects on prognosis. Nat Rev Nephrol. 2011 28;7(8):446-57.

Lulich et al., Compendium on Continuing Education for the Practising Veterinarian 1992;14:127.

Marino CL, Lascelles BD, Vaden SL, Gruen ME, Marks SL. Prevalence and classification of chronic kidney disease in cats randomly selected from four age groups and in cats recruited for degenerative joint disease studies. J Feline Med Surg. 2014;16(6):465-72.

Morota, Gota, et al. Machine learning and data mining advance predictive big data analysis in precision animal agriculture. J Animal Sci 2018.

O'Neill D, Church D, McGreevy P, Thompson P, Brodbelt D. Prevalence of disorders recorded in cats attending primary-care veterinary practice in England. Vet J 2014;202:286 - 291.

O'Neill DG, Church DB, McGreevy PD, et al. Longevity and mortality of cats attending primary care veterinary practices in England. J Feline Med Surg 2015;17:125-133.

Parikh RB, Kakad M, Bates DW. 2016. Integrating predictive analytics into high-value care: the dawn of precision delivery. JAMA 315, 651652.

Peck JS, Benneyan JC, Nightingale DJ, Gaehde SA. Predicting emergency department inpatient admissions to improve same-day patient flow. Acad Emerg Med 2012;19:E1045E1054.

Peck JS, Gaehde SA, Nightingale DJ, Gelman DY, Huckins DS, Lemons MF, et al., Generalizability of a simple approach for predicting hospital admission from an emergency department. Acad Emerg Med 2013;20:11561163.

Pencina MJ, Peterson ED. Moving from clinical trials to precision medicine: the role for predictive modelling. JAMA 2016;315:17131714.

Perez and Wang (2017). The Effectiveness of Data Augmentation in Image Classification using Deep Learning. arXiv:1712.04621.

Pineda, Arturo Lopez, et al. Deep learning facilitates rapid cohort identification using human and veterinary clinical narratives. BioRxiv 2018: 429720.

R Core Team. R: A language and environment for statistical computing. R Foundation for Statistical Computing, Vienna, Austria. 2017 https://www.R-project.org/

Ross LA, Finco DR, Crowell WA. Effect of dietary phosphorus restriction on the kidneys of cats with reduced renal mass. Am J Vet Res. 1982;43(6):1023-6.

Sparkes AH, Caney S, Chalhoub S, Elliott J, Finch N, Gajanayake I, Langston C, Lefebvre HP, White J, Quimby J. ISFM Consensus Guidelines on the Diagnosis and Management of Feline Chronic Kidney Disease. J Feline Med Surg. 2016;18(3):219-39.

Srivastava et al. (2014). Dropout: a simple way to prevent neural networks from overfitting. Journal of Machine Learning Research 15:1929-1958.

Stekhoven et al. (2012). MissForest - nonparametric missing value imputation for mixed-type data. Oxford Journal Bionformatics 28:112-118.

Syme HM, Markwell PJ, Pfeiffer D, et al. Survival of cats with naturally occurring chronic renal failure is related to severity of proteinuria. J Vet Intern Med 2006; 20: 528-535.

Tang J, Alelyani S and Liu (2014) Feature selection for classification: a review. In: Data Classification: Algorithms and applications. CRC press.

Tsoukalas A, Albertson T, Tagkopoulos I. From data to optimal decision making: a data-driven, probabilistic machine learning approach to decision support for patients with sepsis. JMIR medical informatics 201; 53.1.

Although the presently disclosed subject matter and its advantages have been described in detail, it should be understood that various changes, substitutions and alterations can be made herein without departing from the scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the presently disclosed subject matter, processes, machines, manufacture, compositions of matter, means, methods, or steps, presently existing or later to be developed that perform substantially the same function or achieve substantially the same result as the corresponding embodiments described herein can be utilized according to the presently disclosed subject matter. Accordingly, the appended claims are intended to include within their scope such processes, machines, manufacture, compositions of matter, means, methods, or steps.

## Claims

1. A system for identifying susceptibility to developing chronic kidney disease (CKD) for a feline, the system comprising:

   a processor; and
   a memory that stores code that, when executed by the processor, causes the computer system to:

      receive at least one input level of one or more biomarkers from the feline and optionally an input level of an age of the feline, wherein at least one of the one or more biomarkers comprises information relating to a urine specific gravity level, a creatinine level, a urine protein level, a blood urea nitrogen (BUN) or urea level, a white blood cell count (WBC), urine pH, or any combination thereof;
      analyze and transform the at least one input level of the one or more biomarkers and optionally the input level of the age by organizing and/or modifying each input level to derive a probability score or a classification label via a classification algorithm, wherein the classification algorithm comprises code developed from a training dataset, the training dataset comprising medical information relating to both a first plurality of biomarkers and optionally ages from a first set of sample felines and a second plurality of biomarkers and optionally ages from a second set of sample felines, wherein the classification algorithm is developed using a training algorithm;
      wherein the classification algorithm is one of a hard classifier, which determines the classification label of whether the feline is at risk of developing CKD, or a soft classifier, which determines the probability score of the feline developing CKD;
      generate an output, wherein the output is the classification label or the probability score;
      determine or categorize, based on the output, whether the feline is at risk of developing CKD; and
      determine a customized recommendation based on the determining or categorizing.

2. The system according to claim 1, wherein:

   (i) the code, when executed by the processor, further causes the system to display the determination or categorization and customized recommendation on a graphical user interface, or
   (ii) wherein the system further comprises:
   a communication device for transmitting and receiving information; wherein:

      the at least one input level is received from a remote second system, via the communication device; and

the code, when executed by the processor, further causes the system to transmit the determination or categorization and customized recommendation to the remote second system, via the communication device.

3. A computer-implemented method of identifying susceptibility to developing chronic kidney disease (CKD) for a feline, comprising the steps of:

receiving at least one input level of one or more biomarkers from the feline and optionally an input level of an age of the feline, wherein at least one of the one or more biomarkers comprises information relating to a urine specific gravity level, a creatinine level, a urine protein level, a blood urea nitrogen (BUN) or urea level, a white blood cell count (WBC), urine pH, or any combination thereof;

analyzing and transforming the at least one input level of the one or more biomarkers and optionally the input level of the age by organizing and/or modifying each input level to derive a probability score or a classification label via a classification algorithm, wherein the classification algorithm comprises code developed from a training dataset, the training dataset comprising medical information relating to both a first plurality of biomarkers and optionally age from a first set of sample felines and a second plurality of biomarkers and optionally age from a second set of sample felines, wherein the classification algorithm is developed using a training algorithm;

wherein the classification algorithm is one of a hard classifier, which determines the classification label of whether the feline is at risk of developing CKD, or a soft classifier, which determines the probability score of the feline developing CKD;

generating an output, wherein the output is the classification label or the probability score;

determining or categorizing, based on the output, whether the feline is at risk of developing CKD; and

determining a customized recommendation based on the determining or categorizing.

4. The computer-implemented method according to claim 3, wherein:

(i) the method further comprises the step of displaying the determination or categorization and customized recommendation on a graphical user interface, or

(ii) the at least one input level is received from a remote second system, via a communication device; and further comprising the step of:

transmitting the determination or categorization and customized recommendation to the remote second system, via the communication device.

5. A non-transitory computer readable medium, storing instructions that, when executed by a processor, cause a computer system to execute the steps of the method of any one of claims 3 to 5.

6. The system, the non-transitory computer-readable medium or the computer-implemented method according to any one of the claims above, wherein the classification algorithm is developed using one of: a supervised training algorithm under supervision of the one or more biomarkers, and optionally the ages; or an unsupervised training algorithm.

7. The system, the non-transitory computer-readable medium or the computer-implemented method according to any one of the claims above, wherein the at least one input level comprises sequential measurements of the one or more biomarkers measured at different time points.

8. The system, the non-transitory computer-readable medium or the computer-implemented method according to any one of the claims above, wherein the first set of sample felines have been diagnosed with CKD and the second set of sample felines have not been diagnosed with CKD.

9. The system, the non-transitory computer-readable medium or the computer-implemented method according to any one of the claims above, wherein the training dataset is stratified into 2 or more folds for cross validation and/or is filtered by a set of inclusion and/or exclusion criteria.

10. The system, the non-transitory computer-readable medium or the computer-implemented method according to any one of the claims above, wherein the training algorithm comprises an algorithm selected from the group consisting of logistic regression, artificial neural network (ANN), recurrent neural network (RNN), K-nearest neighbor (KNN), Naive Bayes, support vector machine (SVM), random forest, AdaBoost, KNN with dynamic time warping (DTW), RNN with long short-term memory (LSTM), a regularization algorithm comprising 5% or more dropout to prevent overfitting, and any combination thereof.

11. The system, the non-transitory computer-readable medium or the computer-implemented method according to any one of the claims above, wherein custom recommendation is a dietary regimen, and the dietary regimen is selected from the group consisting of a low phosphorous diet, a low protein diet, a low sodium diet, a potassium supplement diet, a polyunsaturated fatty acids (PUPA) supplement diet, an anti-oxidant supplement diet, a vitamin B supplement diet, a liquid diet and any combination thereof.

12. The system, the non-transitory computer-readable medium or the computer-implemented method according to any one of the claims above, wherein the one or more biomarkers comprises:

   (i) information relating to a urine specific gravity level, a creatinine level and a blood urea nitrogen (BUN) or urea level, or
   (ii) information relating to a urine specific gravity level, a creatinine level, a urine protein level, a blood urea nitrogen (BUN) or urea level, a white blood cell count (WBC) and urine pH.

13. The system, the non-transitory computer-readable medium or the computer-implemented method according to any one of the claims above, comprising receiving at least one input level of one or more biomarkers from the feline and an input level of an age of the feline, preferably comprising receiving

   input levels of biomarkers comprising information relating to a urine specific gravity level, a creatinine level and a blood urea nitrogen (BUN) or urea level; and
   an input level of an age of the feline.

14. The system, the non-transitory computer-readable medium or the computer-implemented method according to any one of the claims above, wherein the input levels of the biomarkers and the age of the feline relate to medical records of one or more visit of the feline or at least 2 visits of the feline.

15. The system, the non-transitory computer-readable medium or the computer-implemented method according to any one of the claims above, wherein the classification label or the probability score relates to:

   (i) the feline's status of contracting chronic kidney disease (CKD) at the time of the determination of the classification label or the probability score,
   (ii) the feline's risk of developing chronic kidney disease (CKD) after the determination of the classification label or the probability score,
   (iii) the feline's risk of developing chronic kidney disease (CKD) about 1 year after the determination of the classification label or the probability score, or
   (iv) the feline's risk of developing chronic kidney disease (CKD) about 2 years after the determination of the classification label or the probability score.

**Patentansprüche**

1. System zur Identifizierung der Anfälligkeit für die Entwicklung einer chronischen Nierenerkrankung (CKD) bei einer Katze, wobei das System umfasst:

   einen Prozessor und
   einen Speicher, in dem ein Code gespeichert ist, der bei Ausführung durch den Prozessor das Computersystem zu Folgendem veranlasst:

      Empfangen mindestens eines Eingangswertes eines oder mehrerer Biomarker von der Katze und optional eines Eingangswertes eines Alters der Katze, wobei mindestens einer der einen oder mehreren Biomarker Informationen in Bezug auf einen Wert für das spezifische Gewicht des Urins, einen Kreatininwert, einen Urinproteinwert, einen Blut-Harnstoff-Stickstoff- (BUN) oder Harnstoffwert, eine Leukozytenzahl (WBC), einen Urin-pH-Wert oder irgendeine Kombination davon umfasst;
      Analysieren und Transformieren des mindestens einen Eingangswertes des einen oder der mehreren Biomarker und optional des Eingangswertes des Alters durch Organisieren und/oder Modifizieren jedes Eingangswertes, um einen Wahrscheinlichkeitswert oder ein Klassifikationskennzeichen über einen Klassifikationsalgorithmus abzuleiten, wobei der Klassifikationsalgorithmus einen Code umfasst, der aus einem Trainingsdatensatz entwickelt wurde, wobei der Trainingsdatensatz medizinische Informationen umfasst, die sich sowohl auf eine erste Vielzahl von Biomarkern und optional auf das Alter aus einem ersten Satz von Probenkatzen als auch auf eine zweite Vielzahl von Biomarkern und optional auf das Alter aus einem zweiten Satz von Probenkatzen beziehen, wobei der Klassifikationsalgorithmus unter Verwendung eines Trainingsalgorithmus entwickelt wird;

wobei der Klassifikationsalgorithmus einer von einem harten Klassifikator, der das Klassifikationskennzeichen ermittelt, ob die Katze ein Risiko zur Entwicklung von CKD aufweist, oder einem weichen Klassifikator ist, der den Wahrscheinlichkeitswert für die Entwicklung von CKD bei der Katze ermittelt;

Erzeugen einer Ausgabe, wobei es sich bei der Ausgabe um das Klassifikationskennzeichen oder den Wahrscheinlichkeitswert handelt;

Bestimmen oder Kategorisieren, basierend auf der Ausgabe, ob die Katze ein Risiko für die Entwicklung von CKD aufweist, und

Bestimmen einer individualisierten Empfehlung auf der Grundlage der Bestimmung oder Kategorisierung.

2. System nach Anspruch 1, wobei:

(i) der Code, wenn er von dem Prozessor ausgeführt wird, ferner das System veranlasst, die Bestimmung oder Kategorisierung und die individualisierte Empfehlung auf einer grafischen Benutzeroberfläche anzuzeigen, oder
(ii) wobei das System ferner umfasst:
eine Kommunikationsvorrichtung zum Übertragen und Empfangen von Informationen; wobei:

der mindestens eine Eingangswert von einem entfernten zweiten System über die Kommunikationsvorrichtung empfangen wird und

der Code, wenn er von dem Prozessor ausgeführt wird, das System außerdem veranlasst, die Bestimmung oder Kategorisierung und die individualisierte Empfehlung über die Kommunikationsvorrichtung an das entfernte zweite System zu übertragen.

3. Computer-implementiertes Verfahren zur Identifizierung der Anfälligkeit für die Entwicklung einer chronischen Nierenerkrankung (CKD) bei einer Katze, umfassend die folgenden Schritte:

Empfangen mindestens eines Eingangswertes eines oder mehrerer Biomarker von der Katze und optional eines Eingangswertes eines Alters der Katze, wobei mindestens einer der einen oder mehreren Biomarker Informationen in Bezug auf einen Wert für das spezifische Gewicht des Urins, einen Kreatininwert, einen Urinproteinwert, einen Blut-Harnstoff-Stickstoff- (BUN) oder Harnstoffwert, eine Leukozytenzahl (WBC), einen Urin-pH-Wert oder irgendeine Kombination davon umfasst;

Analysieren und Transformieren des mindestens einen Eingangswertes des einen oder der mehreren Biomarker und optional des Eingangswertes des Alters durch Organisieren und/oder Modifizieren jedes Eingangswertes, um einen Wahrscheinlichkeitswert oder ein Klassifikationskennzeichen über einen Klassifikationsalgorithmus abzuleiten, wobei der Klassifikationsalgorithmus einen Code umfasst, der aus einem Trainingsdatensatz entwickelt wurde, wobei der Trainingsdatensatz medizinische Informationen umfasst, die sich sowohl auf eine erste Vielzahl von Biomarkern und optional auf das Alter aus einem ersten Satz von Probenkatzen als auch auf eine zweite Vielzahl von Biomarkern und optional auf das Alter aus einem zweiten Satz von Probenkatzen beziehen, wobei der Klassifikationsalgorithmus unter Verwendung eines Trainingsalgorithmus entwickelt wird;

wobei der Klassifikationsalgorithmus einer von einem harten Klassifikator, der das Klassifikationskennzeichen ermittelt, ob die Katze ein Risiko zur Entwicklung von CKD aufweist, oder einem weichen Klassifikator ist, der den Wahrscheinlichkeitswert für die Entwicklung von CKD bei der Katze ermittelt;

Erzeugen einer Ausgabe, wobei es sich bei der Ausgabe um das Klassifikationskennzeichen oder den Wahrscheinlichkeitswert handelt;

Bestimmen oder Kategorisieren, basierend auf der Ausgabe, ob die Katze ein Risiko für die Entwicklung von CKD aufweist, und

Bestimmen einer individualisierten Empfehlung auf der Grundlage der Bestimmung oder Kategorisierung.

4. Computerimplementiertes Verfahren nach Anspruch 3, wobei:

(i) das Verfahren ferner den Schritt des Anzeigens der Bestimmung oder Kategorisierung und der individualisierten Empfehlung auf einer grafischen Benutzeroberfläche umfasst, oder
(ii) der mindestens eine Eingangswert von einem entfernten zweiten System über eine Kommunikationsvorrichtung empfangen wird und ferner den folgenden Schritt umfasst:
Übermitteln der Bestimmung oder Kategorisierung und der individualisierten Empfehlung an das entfernte zweite System über die Kommunikationsvorrichtung.

5. Nicht-transitorisches computerlesbares Medium, das Anweisungen speichert, die, wenn sie von einem Prozessor ausgeführt werden, ein Computersystem veranlassen, die Schritte des Verfahrens nach einem der Ansprüche 3 bis 5 auszuführen.

6. System, nicht-transitorisches computerlesbares Medium oder computerimplementiertes Verfahren nach einem der vorstehenden Ansprüche, wobei der Klassifikationsalgorithmus unter Verwendung eines der folgenden Verfahren entwickelt wird: eines überwachten Trainingsalgorithmus unter Überwachung des einen oder der mehreren Biomarker und gegebenenfalls des Alters oder eines nicht überwachten Trainingsalgorithmus.

7. System, nicht-transitorisches computerlesbares Medium oder computerimplementiertes Verfahren nach einem der vorstehenden Ansprüche, wobei der mindestens eine Eingangswert sequentielle Messungen des einen oder der mehreren Biomarker umfasst, die zu unterschiedlichen Zeitpunkten gemessen wurden.

8. System, nicht-transitorisches computerlesbares Medium oder computerimplementiertes Verfahren nach einem der vorstehenden Ansprüche, wobei der erste Stichprobensatz von Katzen mit CKD diagnostiziert worden ist und der zweite Stichprobensatz von Katzen nicht mit CKD diagnostiziert worden ist.

9. System, nicht-transitorisches computerlesbares Medium oder computerimplementiertes Verfahren nach einem der vorstehenden Ansprüche, wobei der Trainingsdatensatz zur Kreuzvalidierung in zwei oder mehr Schichten stratifiziert und/oder durch einen Satz von Einschluss-und/oder Ausschlusskriterien gefiltert wird.

10. System, nicht-transitorisches computerlesbares Medium oder computerimplementiertes Verfahren nach einem der vorstehenden Ansprüche, wobei der Trainingsalgorithmus einen Algorithmus umfasst, der ausgewählt ist aus der Gruppe bestehend aus logistischer Regression, künstlichem neuronalen Netzwerk (ANN), rekurrentem neuronalen Netzwerk (RNN), K-Nearest Neighbor (KNN), Naive Bayes, Support Vector Machine (SVM), Random Forest, Ada-Boost, KNN mit Dynamic Time Warping (DTW), RNN mit Long Short Memory (LSTM), einem Regularisierungsalgorithmus, der 5% oder mehr Dropout umfasst, um Overfitting zu verhindern, und einer beliebigen Kombination davon.

11. System, nicht-transitorisches computerlesbares Medium oder computerimplementiertes Verfahren nach einem der vorstehenden Ansprüche, wobei es sich bei der individualisierten Empfehlung um eine Diät handelt und die Diät ausgewählt ist aus der Gruppe bestehend aus einer Diät mit niedrigem Phosphorgehalt, einer Diät mit niedrigem Proteingehalt, einer Diät mit niedrigem Natriumgehalt, einer Kalium-Ergänzungsdiät, einer Diät zur Ergänzung mehrfach ungesättigter Fettsäuren (PUFA), einer Diät zur Ergänzung von Antioxidantien, einer Vitamin-B-Ergänzungsdiät, einer Flüssigdiät und einer beliebigen Kombination davon.

12. System, nicht-transitorisches computerlesbares Medium oder computerimplementiertes Verfahren nach einem der vorstehenden Ansprüche, wobei der eine oder die mehreren Biomarker Folgendes umfassen:

(i) Informationen über das spezifische Gewicht des Urins, den Kreatininwert und den Blut-Harnstoff-Stickstoff-(BUN) oder Harnstoffwert, oder
(ii) Informationen über das spezifische Gewicht des Urins, den Kreatininwert, den Proteinwert im Urin, den Blut-Harnstoff-Stickstoff- (BUN) oder Harnstoffwert, die Leukozytenzahl (WBC) und den Urin-pH-Wert.

13. System, nicht-transitorisches computerlesbares Medium oder computerimplementiertes Verfahren nach einem der vorstehenden Ansprüche, umfassend das Empfangen mindestens eines Eingangswertes eines oder mehrerer Biomarker von der Katze und eines Eingangswertes des Alters der Katze, vorzugsweise umfassend das Empfangen von

Eingangswerten von Biomarkern, die Informationen in Bezug auf ein spezifisches Gewicht des Urins, einen Kreatininwert und einen Blut-Harnstoff-Stickstoff-(BUN) oder Harnstoffwert umfassen und einem Eingangswert des Alters der Katze.

14. System, nicht-transitorisches computerlesbares Medium oder computerimplementiertes Verfahren nach einem der vorstehenden Ansprüche, wobei sich die Eingangswerte der Biomarker und das Alter der Katze auf medizinische Aufzeichnungen von einem oder mehreren Kontrollterminen der Katze oder von mindestens zwei Kontrollterminen der Katze beziehen.

15. System, nicht-transitorisches computerlesbares Medium oder computerimplementiertes Verfahren nach einem der vorstehenden Ansprüche, wobei sich das Klassifikationskennzeichen oder der Wahrscheinlichkeitswert auf Folgendes bezieht:

(i) den Risikostatus der Katze für eine chronische Nierenerkrankung (CKD) zum Zeitpunkt der Bestimmung des Klassifikationskennzeichens oder des Wahrscheinlichkeitswertes,
(ii) das Risiko der Katze, eine chronische Nierenerkrankung (CKD) zu entwickeln, nachdem das Klassifikationskennzeichen oder der Wahrscheinlichkeitswert bestimmt wurde,
(iii) das Risiko der Katze, etwa ein Jahr nach der Bestimmung des Klassifikationskennzeichens oder des Wahrscheinlichkeitswertes eine chronische Nierenerkrankung (CKD) zu entwickeln, oder
(iv) das Risiko der Katze, eine chronische Nierenerkrankung (CKD) zu entwickeln, etwa zwei Jahre nach der Bestimmung des Klassifikationskennzeichens oder des Wahrscheinlichkeitswertes.

**Revendications**

1. Système pour identifier la susceptibilité au développement d'une maladie rénale chronique (MRC) pour un félin, le système comprenant :

un processeur ; et
une mémoire qui stocke un code qui, lorsqu'il est exécuté par le processeur, amène le système informatique à :

recevoir au moins un niveau d'entrée d'un ou plusieurs biomarqueurs du félin et facultativement un niveau d'entrée d'un âge du félin, dans lequel au moins un des un ou plusieurs biomarqueurs comprend des informations concernant un niveau de densité de l'urine, un taux de créatinine, un taux de protéine dans l'urine, un taux d'azote uréique (BUN) ou d'urée dans le sang, une numération de globules blancs (WBC), un pH de l'urine, ou une combinaison quelconque de ceux-ci ;
analyser et transformer l'au moins un niveau d'entrée des un ou plusieurs biomarqueurs et facultativement le niveau d'entrée de l'âge par organisation et/ou modification de chaque niveau d'entrée pour déduire un score de probabilité ou une étiquette de classification par l'intermédiaire d'un algorithme de classification, dans lequel l'algorithme de classification comprend un code développé à partir d'un ensemble de données d'apprentissage, l'ensemble de données d'apprentissage comprenant des informations médicales concernant à la fois une première pluralité de biomarqueurs et facultativement les âges d'un premier ensemble d'échantillon de félins et une deuxième pluralité de biomarqueurs et facultativement les âges d'un deuxième ensemble d'échantillon de félins, dans lequel l'algorithme de classification est développé au moyen d'un algorithme d'apprentissage ;
dans lequel l'algorithme de classification est l'un parmi un classificateur dur, qui détermine l'étiquette de classification du fait que le félin présente ou non un risque de développer une MRC, ou un classificateur souple, qui détermine le score de probabilité que le félin développe une MRC ;
générer une sortie, dans lequel la sortie est l'étiquette de classification ou le score de probabilité ;
déterminer ou catégoriser, sur la base de la sortie, le fait que le félin présente ou non un risque de développer une MRC ; et
déterminer une recommandation personnalisée sur la base de la détermination ou de la catégorisation.

2. Système selon la revendication 1, dans lequel :

(i) le code, lorsqu'il est exécuté par le processeur, amène en outre le système à afficher la détermination ou catégorisation et la recommandation personnalisée sur une interface utilisateur graphique, ou
(ii) le système comprenant en outre :

un dispositif de communication pour transmettre et recevoir des informations ; dans lequel : l'au moins un niveau d'entrée est reçu depuis un deuxième système distant, par l'intermédiaire du dispositif de communication ; et
le code, lorsqu'il est exécuté par le processeur, amène en outre le système à transmettre la détermination ou catégorisation et la recommandation personnalisée au deuxième système distant, par l'intermédiaire du dispositif de communication.

3. Procédé mis en oeuvre par ordinateur d'identification de la susceptibilité au développement d'une maladie rénale chronique (MRC) pour un félin, comprenant les étapes de :

réception d'au moins un niveau d'entrée d'un ou plusieurs biomarqueurs du félin et facultativement d'un niveau d'entrée d'un âge du félin, dans lequel au moins un des un ou plusieurs biomarqueurs comprend des informations concernant un niveau de densité de l'urine, un taux de créatinine, un taux de protéine dans l'urine, un taux d'azote uréique (BUN) ou d'urée dans le sang, une numération de globules blancs (WBC), un pH de l'urine, ou une combinaison quelconque de ceux-ci ;
analyse et transformation de l'au moins un niveau d'entrée des un ou plusieurs biomarqueurs et facultativement du niveau d'entrée de l'âge par organisation et/ou modification de chaque niveau d'entrée pour déduire un score de probabilité ou une étiquette de classification par l'intermédiaire d'un algorithme de classification, dans lequel l'algorithme de classification comprend un code développé à partir d'un ensemble de données d'apprentissage, l'ensemble de données d'apprentissage comprenant des informations médicales concernant à la fois une première pluralité de biomarqueurs et facultativement l'âge d'un premier ensemble d'échantillon de félins et une deuxième pluralité de biomarqueurs et facultativement l'âge d'un deuxième ensemble d'échantillon de félins, dans lequel l'algorithme de classification est développé au moyen d'un algorithme d'apprentissage ;
dans lequel l'algorithme de classification est l'un parmi un classificateur dur, qui détermine l'étiquette de classification du fait que le félin présente ou non un risque de développer une MRC, ou un classificateur souple, qui détermine le score de probabilité que le félin développe une MRC ;
génération d'une sortie, dans lequel la sortie est l'étiquette de classification ou le score de probabilité ;
détermination ou catégorisation, sur la base de la sortie, du fait que le félin présente ou non un risque de développer une MRC ; et
détermination d'une recommandation personnalisée sur la base de la détermination ou de la catégorisation.

4. Procédé mis en oeuvre par ordinateur selon la revendication 3, dans lequel :

(i) le procédé comprend en outre l'étape d'affichage de la détermination ou catégorisation et de la recommandation personnalisée sur une interface utilisateur graphique, ou
(ii) l'au moins un niveau d'entrée est reçu depuis un deuxième système distant, par l'intermédiaire d'un dispositif de communication ; et comprenant en outre l'étape de :
transmission de la détermination ou catégorisation et de la recommandation personnalisée au deuxième système distant, par l'intermédiaire du dispositif de communication.

5. Support lisible par ordinateur non transitoire, stockant des instructions qui, lorsqu'elles sont exécutées par un processeur, amènent un système informatique à exécuter les étapes du procédé selon l'une quelconque des revendications 3 à 5.

6. Système, support lisible par ordinateur non transitoire ou procédé mis en oeuvre par ordinateur selon l'une quelconque des revendications précédentes, dans lesquels l'algorithme de classification est développé en utilisant l'un parmi : un algorithme d'apprentissage supervisé sous la supervision des un ou plusieurs biomarqueurs, et facultativement les âges ; ou un algorithme d'apprentissage non supervisé.

7. Système, support lisible par ordinateur non transitoire ou procédé mis en oeuvre par ordinateur selon l'une quelconque des revendications précédentes, dans lesquels l'au moins un niveau d'entrée comprend des mesures séquentielles des un ou plusieurs biomarqueurs mesurés à différents temps.

8. Système, support lisible par ordinateur non transitoire ou procédé mis en oeuvre par ordinateur selon l'une quelconque des revendications précédentes, dans lesquels le premier ensemble d'échantillon de félins a été diagnostiqué avec MRC et le deuxième ensemble d'échantillon de félins n'a pas été diagnostiqué avec MRC.

9. Système, support lisible par ordinateur non transitoire ou procédé mis en oeuvre par ordinateur selon l'une quelconque des revendications précédentes, dans lesquels l'ensemble de données d'apprentissage est stratifié en 2 fois ou plus pour validation croisée et/ou est filtré par un ensemble de critères d'inclusion et/ou d'exclusion.

10. Système, support lisible par ordinateur non transitoire ou procédé mis en oeuvre par ordinateur selon l'une quelconque des revendications précédentes, dans lesquels l'algorithme d'apprentissage comprend un algorithme choisi dans le groupe constitué d'une régression logistique, un réseau neuronal artificiel (ANN), un réseau neuronal récurrent (RNN), le K plus proche voisin (KNN), un bayésien naïf, une machine à vecteurs de support (SVM), une forêt aléatoire, AdaBoost, un KNN à déformation temporelle dynamique (DTW), un RNN à mémoire court et long terme (LSTM), un algorithme de régularisation comprenant 5 % ou plus de perte afin d'éviter un surapprentissage, et une combinaison quelconque de ceux-ci.

11. Système, support lisible par ordinateur non transitoire ou procédé mis en oeuvre par ordinateur selon l'une quelconque des revendications précédentes, dans lesquels la recommandation personnalisée est un régime alimentaire, et le régime alimentaire est choisi dans le groupe constitué d'un régime pauvre en phosphore, un régime pauvre en protéine, un régime pauvre en sodium, une alimentation complémentée en potassium, une alimentation complémentée en acides gras polyinsaturés (PUFA), une alimentation complémentée en antioxydant, une alimentation complémentée en vitamine B, un régime liquide et une combinaison quelconque de ceux-ci.

12. Système, support lisible par ordinateur non transitoire ou procédé mis en oeuvre par ordinateur selon l'une quelconque des revendications précédentes, dans lesquels les un ou plusieurs biomarqueurs comprennent :

    (i) des informations concernant un niveau de densité de l'urine, un taux de créatinine et un taux d'azote uréique (BUN) ou d'urée dans le sang, ou
    (ii) des informations concernant un niveau de densité de l'urine, un taux de créatinine, un taux de protéine dans l'urine, un taux d'azote uréique (BUN) ou d'urée dans le sang, une numération de globules blancs (WBC) et un pH de l'urine.

13. Système, support lisible par ordinateur non transitoire ou procédé mis en oeuvre par ordinateur selon l'une quelconque des revendications ci-dessus, comprenant la réception d'au moins un niveau d'entrée d'un ou plusieurs biomarqueurs du félin et un niveau d'entrée d'un âge du félin, de préférence comprenant la réception de niveaux d'entrée de biomarqueurs comprenant des informations concernant un niveau de densité de l'urine, un taux de créatinine et un taux d'azote uréique (BUN) ou d'urée dans le sang ; et
un niveau d'entrée d'un âge du félin.

14. Système, support lisible par ordinateur non transitoire ou procédé mis en oeuvre par ordinateur selon l'une quelconque des revendications précédentes, dans lesquels le niveau d'entrées des biomarqueurs et l'âge du félin sont associés à des dossiers médicaux d'une ou plusieurs visites du félin ou au moins 2 visites du félin.

15. Système, support lisible par ordinateur non transitoire ou procédé mis en oeuvre par ordinateur selon l'une quelconque des revendications précédentes, dans lesquels l'étiquette de classification ou le score de probabilité concerne :

    (i) le statut du félin suivant qu'il a contracté ou non la maladie rénale chronique (MRC) au moment de la détermination de l'étiquette de classification ou du score de probabilité,
    (ii) le risque que le félin développe une maladie rénale chronique (MRC) après la détermination de l'étiquette de classification ou du score de probabilité,
    (iii) le risque que le félin développe une maladie rénale chronique (MRC) environ 1 an après la détermination de l'étiquette de classification ou du score de probabilité, ou
    (iv) le risque que le félin développe une maladie rénale chronique (MRC) environ 2 ans après la détermination de l'étiquette de classification ou du score de probabilité.

Distribution of visits based on cat age

FIG. 1

EP 3 740 760 B1

Continued on FIG. 2A continued

FIG. 2A

FIG. 2A continued

FIG. 2B

Continued on FIG. 2B continued

From FIG. 2B

BUN

WBC

URINE_PROTEIN

1.0
0.8
0.6
0.4
0.2
0.0

FIG. 2B continued

Continued on FIG. 2C continued

FIG. 2C

FIG. 2C continued

FIG. 3

FIG. 3 continued

FIG. 4A

FIG. 4B

FIG. 4C

FIG. 4D

Feature Selection: Wrapper top-down method with F1-measure

FIG. 5

FIG. 6

PRECISION-RECALL CURVE

ROC

K=3 AUC 0.84
K=5 AUC 0.85
K=7 AUC 0.86
K=9 AUC 0.87
K=11 AUC 0.87
K=13 AUC 0.87
K=15 AUC 0.87
K=17 AUC 0.87

K=3 AUC 0.87
K=5 AUC 0.89
K=7 AUC 0.90
K=9 AUC 0.90
K=11 AUC 0.91
K=13 AUC 0.91
K=15 AUC 0.91
K=17 AUC 0.91

FIG. 7A

FIG. 7B

EP 3 740 760 B1

FIG. 8A

FIG. 8B

RNN

(CKD Probability)

Hidden Layer L4 (6-40 nodes)

Hidden Layer L3 (6-250 nodes)

Hidden Layer L2 (6-250 nodes)

Hidden Layer L1 (6-250 nodes)

INPUT
(8K cats, 60K visits, 6 selected features)

FIG. 9

DATASET

Visit 1
Visit 2
...
Visit 31

URINE SG
URINE PROTEIN
URINE PH
BUN
WBC
CREATININE

Cat 1

Cat 8806

**FIG. 10A**

RNN Training

Visit N-1 → RNN Hidden Layers visit N-1

Visit N → RNN Hidden Layers visit N-1

Visit N+1 → RNN Hidden Layers visit N-1

Predicted Outcome (CKD/Healthy)

Parameter refinement

Known Outcome

**FIG. 10B**

| 1 layer | 2 layers | | | 3 layers | | | | | | |
|---|---|---|---|---|---|---|---|---|---|---|
| **LSTM** 10-0-0 | 5-5-0 | 7-3-0 | 3-7-0 | 3-3-3 | 3-3-5 | 3-5-3 | 2-4-4 | 5-3-3 | 4-2-4 | 4-4-2 | |
| 78.3% | 80.3% | 82.4% | 82.6% | 81.9% | 82.1% | 83.1% | 82.6% | 82.2% | 79.8% | 82.6% | F1 Score |
| 91.9% | 92.7% | 93.2% | 92.8% | 93.6% | 92.9% | 93.7% | 93.4% | 93.8% | 92.6% | 93.3% | AUC ROC |
| 86.9% | 88.5% | 89.8% | 89.6% | 90.3% | 89.1% | 90.4% | 90.2% | 90.7% | 87.0% | 90.4% | AUC PR |
| 20-0-0 | 10-10-0 | 13-7-0 | 7-13-0 | 7-7-7 | 5-5-10 | 5-10-5 | 4-8-8 | 10-5-5 | 8-4-8 | 8-8-4 | |
| 82.4% | 82.4% | 82.8% | 83.3% | 84.2% | 83.4% | 81.8% | 82.2% | 80.8% | 83.1% | 81.5% | F1 Score |
| 93.5% | 93.6% | 93.7% | 93.6% | 93.9% | 93.7% | 93.7% | 93.5% | 92.1% | 93.7% | 92.7% | AUC ROC |
| 90.6% | 90.5% | 90.5% | 90.7% | 91.1% | 90.6% | 89.5% | 90.0% | 87.9% | 90.8% | 89.6% | AUC PR |
| 30-0-0 | 15-15-0 | 20-10-0 | 10-20-0 | 10-10-10 | 8-8-15 | 8-15-8 | 6-12-12 | 15-8-8 | 12-6-12 | 12-12-6 | |
| 80.0% | 83.5% | 81.6% | 83.0% | 81.3% | 82.8% | 83.5% | 82.3% | 78.1% | 80.6% | 81.8% | F1 Score |
| 92.2% | 93.9% | 93.5% | 93.5% | 93.0% | 93.2% | 93.8% | 93.3% | 91.8% | 92.3% | 92.9% | AUC ROC |
| 88.3% | 90.9% | 89.7% | 90.5% | 89.6% | 90.0% | 90.7% | 89.8% | 86.4% | 88.7% | 88.9% | AUC PR |
| 40-0-0 | 20-20-0 | 27-13-0 | 13-27-0 | 13-13-13 | 10-10-20 | 10-20-10 | 8-16-16 | 20-10-10 | 16-8-16 | 16-16-8 | |
| 81.9% | 82.5% | 82.8% | 84.2% | 83.0% | 82.5% | 82.9% | 83.5% | 82.6% | 83.4% | 82.1% | F1 Score |
| 93.0% | 93.4% | 92.8% | 93.8% | 93.2% | 92.9% | 93.6% | 94.0% | 92.9% | 93.1% | 93.4% | AUC ROC |
| 89.4% | 90.3% | 89.8% | 90.9% | 90.2% | 89.7% | 90.6% | 90.9% | 89.7% | 90.2% | 89.9% | AUC PR |
| 50-0-0 | 25-25-0 | 33-17-0 | 17-33-0 | 17-17-17 | 13-13-25 | 13-25-13 | 10-20-20 | 25-13-13 | 20-10-20 | 20-20-10 | |
| 81.5% | 82.5% | 81.7% | 81.3% | 80.9% | 82.6% | 81.7% | 83.0% | 81.3% | 83.2% | 82.7% | F1 Score |
| 93.2% | 92.5% | 92.5% | 92.6% | 92.1% | 93.5% | 91.4% | 93.0% | 91.9% | 93.7% | 93.1% | AUC ROC |
| 89.7% | 89.7% | 89.2% | 89.3% | 88.9% | 90.5% | 88.5% | 90.0% | 89.2% | 90.5% | 90.0% | AUC PR |

FIG. 11

EP 3 740 760 B1

| RNN | 1 layer | 2 layers | | | 3 layers | | | | | | | |
|---|---|---|---|---|---|---|---|---|---|---|---|---|
| | 10-0-0 | 5-5-0 | 7-3-0 | 3-7-0 | 3-3-3 | 3-3-5 | 3-5-3 | 2-4-4 | 5-3-3 | 4-2-4 | 4-4-2 | |
| | 83.3% | 81.1% | 83.1% | 82.8% | 81.2% | 83.9% | 83.9% | 82.1% | 82.7% | 83.0% | 81.4% | F1 Score |
| | 93.4% | 92.4% | 93.1% | 93.2% | 93.7% | 93.9% | 94.1% | 93.0% | 93.7% | 93.6% | 93.3% | AUC ROC |
| | 90.4% | 89.2% | 90.2% | 90.4% | 90.5% | 91.0% | 91.3% | 89.5% | 90.4% | 90.5% | 90.3% | AUC PR |
| | 20-0-0 | 10-10-0 | 13-7-0 | 7-13-0 | 7-7-7 | 5-5-10 | 5-10-5 | 4-8-8 | 10-5-5 | 8-4-8 | 8-8-4 | |
| | 78.0% | 79.0% | 83.4% | 83.4% | 82.4% | 82.3% | 83.4% | 83.3% | 80.4% | 83.1% | 82.8% | F1 Score |
| | 91.8% | 91.9% | 93.4% | 93.2% | 93.2% | 93.2% | 93.7% | 94.1% | 92.5% | 93.3% | 93.5% | AUC ROC |
| | 87.4% | 87.3% | 90.6% | 90.3% | 89.7% | 90.0% | 90.9% | 91.2% | 89.1% | 90.4% | 90.7% | AUC PR |
| | 30-0-0 | 15-15-0 | 20-10-0 | 10-20-0 | 10-10-10 | 8-8-15 | 8-15-8 | 6-12-12 | 15-8-8 | 12-6-12 | 12-12-6 | |
| | 83.0% | 82.5% | 83.0% | 82.4% | 83.5% | 82.9% | 80.9% | 82.8% | 83.8% | 81.9% | 83.3% | F1 Score |
| | 93.7% | 92.8% | 93.8% | 92.3% | 93.3% | 92.6% | 93.7% | 93.0% | 93.4% | 93.6% | 93.6% | AUC ROC |
| | 90.5% | 89.9% | 90.8% | 89.5% | 90.5% | 89.6% | 90.7% | 90.0% | 90.5% | 90.6% | 90.5% | AUC PR |
| | 40-0-0 | 20-20-0 | 27-13-0 | 13-27-0 | 13-13-13 | 10-10-20 | 10-20-10 | 8-16-16 | 20-10-10 | 16-8-16 | 16-16-8 | |
| | 82.1% | 82.4% | 82.1% | 82.1% | 82.1% | 83.0% | 82.1% | 81.7% | 81.2% | 82.5% | 81.5% | F1 Score |
| | 93.0% | 93.5% | 93.1% | 93.2% | 92.4% | 93.7% | 93.4% | 93.1% | 92.8% | 93.2% | 92.8% | AUC ROC |
| | 89.8% | 90.6% | 90.0% | 90.2% | 89.3% | 90.6% | 90.2% | 89.9% | 89.2% | 90.3% | 89.4% | AUC PR |
| | 50-0-0 | 20-25-0 | 33-17-0 | 17-33-0 | 17-17-17 | 13-13-25 | 13-25-13 | 10-20-20 | 25-13-13 | 20-10-20 | 20-20-10 | |
| | 79.9% | 82.5% | 81.8% | 81.4% | 83.2% | 83.1% | 81.8% | 83.2% | 82.3% | 83.1% | 82.5% | F1 Score |
| | 91.1% | 93.9% | 92.6% | 93.3% | 93.7% | 93.6% | 92.8% | 93.8% | 93.0% | 93.9% | 93.7% | AUC ROC |
| | 87.5% | 90.7% | 89.4% | 90.0% | 90.8% | 90.8% | 89.4% | 90.7% | 90.1% | 90.8% | 90.7% | AUC PR |

## FIG. 11 continued

EP 3 740 760 B1

FIG. 12

FIG. 13A

ROC CURVE

FIG. 13B

FIG. 13C

PRECISION–RECALL CURVE

FIG. 13D

FIG. 14A

ROC

FIG. 14B

FIG. 14C

PRECISION-RECALL CURVE

* Prediction based on Prior
(Randomly predict CKD
with 26% probability)

fold 1 AUC: 0.90
fold 2 AUC: 0.93
fold 3 AUC: 0.92
fold 4 AUC: 0.90
fold 5 AUC: 0.93
overall AUC: 0.91

FIG. 14D

FIG. 15

FIG. 16

No CKD
Age at T0: 9.6 years

FIG. 17A

Age at T0: 8.7 years

FIG. 17B

Probable CKD

Age at T0: 16.1 years

FIG. 17C

Age at T0: 16.4 years

FIG. 17D

Age at T0: 13.5 years

FIG. 17E

Age at T0: 17.7 years

FIG. 17H

EP 3 740 760 B1

FIG. 17G

FIG. 17H

RNN

LSTM

FIG. 18

FIG. 19

FIG. 20

FIG. 21

FIG. 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62619681
- US 62698046
- US 20120077690 A1
- US 20130323751 A1

- EP 3112871 A1
- EP 2462445 A1
- EP 3054301 A1

### Non-patent literature cited in the description

- **ELLIOTT et al.** Dietary therapy for feline chronic kidney disease. *Encyclopedia of feline clinical nutrition,* 2015
- **JONATHAN D. FOSTER.** Update on Mineral and Bone Disorders in Chronic Kidney Disease. *Vet Clin North Am Small Anim Pract.,* November 2016, vol. 46 (6), 1131-49
- Dietary therapy for feline chronic kidney disease. **ELLIOTT et al.** Encyclopedia of feline clinical nutrition. 2015
- Chronic renal disease: the importance of nutrition. **ELLIOTT et al.** Encyclopedia of feline clinical nutrition. 2015
- **STEKHOVEN, DANIEL J.** MissForest - nonparametric missing value imputation for mixed- type data. *Oxford Journal's Bioinformatics,* 2012, vol. 28 (1), 112-118
- **GRANITTO, PABLO M. et al.** Recursive feature elimination with random forest for PTR- MS analysis of agroindustrial products. *Chemometrics and Intelligent Laboratory Systems,* 2006, vol. 83 (2), 83-90
- **GIORGINO, TONI.** Computing and visualizing dynamic time warping alignments in R: the dtw package. *Journal of statistical Software,* 2009, vol. 31 (7), 1-24
- **TAN, SONGBO.** Neighbor-weighted k-nearest neighbor for unbalanced text corpus. *Expert Systems with Applications,* 2005, vol. 28 (4), 667-671
- **SRIVASTAVA, NITISH et al.** Dropout: a simple way to prevent neural networks from overfitting. *Journal of Machine Learning Research,* 2014, vol. 15 (1), 1929-1958
- **BOYD LM ; LANGSTON C ; THOMPSON K et al.** Survival in cats with naturally occurring chronic kidney disease (2000-2002). *J Vet Intern Med,* 2008, vol. 22, 1111-1117
- British Small Animal Veterinary Association (BSAVA) Manual of Canine and Feline Nephrology and Urology. 2007

- **BROWN CA ; ELLIOTT J ; SCHMIEDT CW ; BROWN SA.** Chronic Kidney Disease in Aged Cats: Clinical Features, Morphology, and Proposed Pathogeneses. *Vet Pathol.,* 2016, vol. 53 (2), 309-26
- **CALLAHAN A ; SHAH NH.** Machine Learning in Healthcare. *Key Advances in Clinical Informatics,* 2018, 279-291
- **CONROY M ; CHANG YM ; BRODBELT D ; ELLIOTT J.** Survival after diagnosis of hypertension in cats attending primary care practice in the United Kingdom. *J Vet Intern Med.,* 2018, 1-10
- **FINCH NC.** Measurement of glomerular filtration rate in cats; Methods and advantages over routine markers of renal function. *J Feline Med Surg.,* 2014, vol. 16 (9), 736-48
- **FINCH NC ; GEDDES RF ; SYME HM et al.** Fibroblast growth factor 23 (FGF-23) concentrations in cats with early non azotemic chronic kidney disease (CKD) and in healthy geriatric cats. *J Vet Intern Med,* 2013, vol. 27, 227-233
- **GEDDES RF ; FINCH NC ; ELLIOTT J et al.** Fibroblast growth factor 23 in feline chronic kidney disease. *J Vet Intern Med,* 2013, vol. 27, 234-241
- **GULTEPE, EREN et al.** From vital signs to clinical outcomes for patients with sepsis: a machine learning basis for a clinical decision support system. *Journal of the American Medical Informatics Association,* 2013, vol. 21 (2), 315-325
- **HALL JA ; YERRAMILLI M ; OBARE E et al.** Comparison of serum concentrations of symmetric dimethylarginine and creatinine as kidney function biomarkers in cats with chronic kidney disease. *J Vet Intern Med,* 2014, vol. 28, 1676-1683
- **HOCHREITER S ; SCHMIDHUBER J.** Long Short-Term Memory. *Neural Computation.,* 1997, vol. 9 (8), 1735-1780, https://doi.org/10.1162/neco.1997.9.8.1735
- **JEPSON RE ; BRODBELT D ; VALLANCE C ; SYME HM ; ELLIOTT J.** Evaluation of the predictors of azotemia in cats. *J Vet Intern Med,* 2009, vol. 23, 806-813

- **LEVIN A ; STEVENS PE.** Early detection of CKD: the benefits, limitations and effects on prognosis. *Nat Rev Nephrol.,* 2011, vol. 28 (7(8)), 446-57
- **LULICH et al.** *Compendium on Continuing Education for the Practising Veterinarian,* 1992, vol. 14, 127
- **MARINO CL ; LASCELLES BD ; VADEN SL ; GRUEN ME ; MARKS SL.** Prevalence and classification of chronic kidney disease in cats randomly selected from four age groups and in cats recruited for degenerative joint disease studies. *J Feline Med Surg.,* 2014, vol. 16 (6), 465-72
- **MOROTA, GOTA et al.** Machine learning and data mining advance predictive big data analysis in precision animal agriculture. *J Animal Sci,* 2018
- **O'NEILL D ; CHURCH D ; MCGREEVY P ; THOMPSON P ; BRODBELT D.** Prevalence of disorders recorded in cats attending primary-care veterinary practice in England. *Vet J,* 2014, vol. 202, 286-291
- **O'NEILL DG ; CHURCH DB ; MCGREEVY PD et al.** Longevity and mortality of cats attending primary care veterinary practices in England. *J Feline Med Surg,* 2015, vol. 17, 125-133
- **PARIKH RB ; KAKAD M ; BATES DW.** Integrating predictive analytics into high-value care: the dawn of precision delivery. *JAMA,* 2016, vol. 315, 651652
- **PECK JS ; BENNEYAN JC ; NIGHTINGALE DJ ; GAEHDE SA.** Predicting emergency department inpatient admissions to improve same-day patient flow. *Acad Emerg Med,* 2012, vol. 19 (E1045E1054
- **PECK JS ; GAEHDE SA ; NIGHTINGALE DJ ; GELMAN DY ; HUCKINS DS ; LEMONS MF et al.** Generalizability of a simple approach for predicting hospital admission from an emergency department. *Acad Emerg Med,* 2013, vol. 20, 11561163
- **PENCINA MJ ; PETERSON ED.** Moving from clinical trials to precision medicine: the role for predictive modelling. *JAMA,* 2016, vol. 315, 17131714
- **PEREZ ; WANG.** *The Effectiveness of Data Augmentation in Image Classification using Deep Learning,* 2017
- **PINEDA, ARTURO LOPEZ et al.** Deep learning facilitates rapid cohort identification using human and veterinary clinical narratives. *BioRxiv,* 2018, 429720
- **R CORE TEAM. R.** A language and environment for statistical computing. *R Foundation for Statistical Computing,* 2017, https://www.R-project.org
- **ROSS LA ; FINCO DR ; CROWELL WA.** Effect of dietary phosphorus restriction on the kidneys of cats with reduced renal mass. *Am J Vet Res.,* 1982, vol. 43 (6), 1023-6
- **SPARKES AH ; CANEY S ; CHALHOUB S ; ELLIOTT J ; FINCH N ; GAJANAYAKE I ; LANGSTON C ; LEFEBVRE HP ; WHITE J ; QUIMBY J.** ISFM Consensus Guidelines on the Diagnosis and Management of Feline Chronic Kidney Disease. *J Feline Med Surg.,* 2016, vol. 18 (3), 219-39
- **SRIVASTAVA et al.** Dropout: a simple way to prevent neural networks from overfitting. *Journal of Machine Learning Research,* 2014, vol. 15, 1929-1958
- **STEKHOVEN et al.** MissForest - nonparametric missing value imputation for mixed-type data. *Oxford Journal Bionformatics,* 2012, vol. 28, 112-118
- **SYME HM ; MARKWELL PJ ; PFEIFFER D et al.** Survival of cats with naturally occurring chronic renal failure is related to severity of proteinuria. *J Vet Intern Med,* 2006, vol. 20, 528-535
- Feature selection for classification: a review. **TANG J ; ALELYANI S ; LIU.** Data Classification: Algorithms and applications. CRC press, 2014
- **TSOUKALAS A ; ALBERTSON T ; TAGKOPOULOS I.** From data to optimal decision making: a data-driven, probabilistic machine learning approach to decision support for patients with sepsis. *JMIR medical informatics,* vol. 201 (53), 1